(19) Europäisches Patentamt / European Patent Office / Office européen des brevets

(11) **EP 2 748 312 B1**

(12) **EUROPEAN PATENT SPECIFICATION**

(45) Date of publication and mention
of the grant of the patent:
**14.09.2016 Bulletin 2016/37**

(21) Application number: **12756097.7**

(22) Date of filing: **24.08.2012**

(51) Int Cl.:
*C12N 9/04* (2006.01)     *C12Q 1/26* (2006.01)
*C12Q 1/60* (2006.01)     *G01N 33/92* (2006.01)

(86) International application number:
**PCT/EP2012/003574**

(87) International publication number:
**WO 2013/026576 (28.02.2013 Gazette 2013/09)**

(54) **CHOLESTEROL OXIDASE**

CHOLESTERINOXIDASE

OXYDASE DE CHOLESTÉROL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.08.2011 EP 11006940**

(43) Date of publication of application:
**02.07.2014 Bulletin 2014/27**

(73) Proprietors:
• **Roche Diagnostics GmbH
68305 Mannheim (DE)**
Designated Contracting States:
**DE**
• **F.Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Ultizyme International Ltd.
Meguro
Tokyo 152-0013 (JP)**

(72) Inventors:
• **KOJIMA, Katsuhiro
Tokyo 152-0013 (JP)**
• **MORI, Kazushige
Tokyo 152-0013 (JP)**
• **SODE, Koji
Tokyo 183-8538 (JP)**

(74) Representative: **Jung, Michael et al
Roche Diagnostics GmbH
Patent Department (LMP.....6164)
Sandhofer Strasse 116
68305 Mannheim (DE)**

(56) References cited:
• **VRIELINK ALICE ET AL: "Cholesterol oxidase: biochemistry and structural features", FEBS JOURNAL, vol. 276, no. 23, December 2009 (2009-12), pages 6826-6843, XP002669677, ISSN: 1742-464X, DOI: 10.1111/J.1742-4658.2009.07377.X**
• **POLLEGIONI LOREDANO ET AL: "Cholesterol oxidase: biotechnological applications", FEBS JOURNAL, vol. 276, no. 23, December 2009 (2009-12), pages 6857-6870, XP002669678, ISSN: 1742-464X, DOI: 10.1111/J.1742-4658.2009.07379.X**
• **YUE Q KIMBERLEY ET AL: "Crystal structure determination of cholesterol oxidase from Streptomyces and structural characterization of key active site mutants", BIOCHEMISTRY, vol. 38, no. 14, 6 April 1999 (1999-04-06) , pages 4277-4286, XP002669679, ISSN: 0006-2960**
• **TOYAMA MITSUTOSHI ET AL: "Alteration of substrate specificity of cholesterol oxidase from Streptomyces sp. by site-directed mutagenesis", PROTEIN ENGINEERING, vol. 15, no. 6, June 2002 (2002-06), pages 477-483, XP002669680, ISSN: 0269-2139**

EP 2 748 312 B1

**(Cont. next page)**

- YAN SUN ET AL: "Improvement of the thermostability and enzymatic activity of cholesterol oxidase by site-directed mutagenesis", BIOTECHNOLOGY LETTERS, vol. 33, no. 10, 24 June 2011 (2011-06-24) , pages 2049-2055, XP019952703, SPRINGER NETHERLANDS, DORDRECHT ISSN: 1573-6776, DOI: 10.1007/S10529-011-0669-6
- DATABASE UniProt [Online] 23 November 2004 (2004-11-23), "SubName: Full=Putative cholesterol oxidase;", XP002683266, retrieved from EBI accession no. UNIPROT:Q5Z338 Database accession no. Q5Z338 & ISHIKAWA J ET AL: "The complete genomic sequence of Nocardia farcinica IFM 10152", PROCEEDINGS OF THE NATIONAL ACADEMY OF SCIENCES, NATIONAL ACADEMY OF SCIENCES, US, vol. 101, no. 41, 12 October 2004 (2004-10-12), pages 14925-14930, XP002995067, ISSN: 0027-8424, DOI: 10.1073/PNAS.0406410101
- DATABASE UniProt [Online] 29 May 2007 (2007-05-29), "SubName: Full=Cholesterol oxidase; EC=1.1.3.6; Flags: Precursor;", XP002683267, retrieved from EBI accession no. UNIPROT:A4X855 Database accession no. A4X855

- DATABASE UniProt [Online] 2 November 2010 (2010-11-02), "SubName: Full=Putative cholesterol oxidase;", XP002683268, retrieved from EBI accession no. UNIPROT:E0TBM8 Database accession no. E0TBM8
- DOUKYU NORIYUKI: "Characteristics and biotechnological applications of microbial cholesterol oxidases", APPLIED MICROBIOLOGY AND BIOTECHNOLOGY, vol. 83, no. 5, July 2009 (2009-07), pages 825-837, XP002669681, ISSN: 0175-7598, DOI: 10.1007/S00253-009-2059-8
- PARRA A ET AL: "Bioanalytical device based on cholesterol oxidase-bonded SAM-modified electrodes", ANALYTICAL AND BIOANALYTICAL CHEMISTRY, vol. 388, no. 5-6, 6 March 2007 (2007-03-06), pages 1059-1067, XP019537456, SPRINGER, BERLIN, DE ISSN: 1618-2650, DOI: 10.1007/S00216-007-1187-1

SEQUENCE LISTING

<110> Roche Diagnostics GmbH F. Hoffmann-La Roche AG ultizyme International Ltd.
<120> cholesterol oxidase
<130> 27498 wo
<160> 162
<170> PatentIn version 3.1
<210> 1
<211> 546
<212> PRT
<213> Streptomyces Sp.
<220>
<221> MISC_FEATURE
<222> (159)..(159)
<223> Xaa is Met, Phe, Leu, val, Cys, Ile, Ala, Gln, Tyr, Lys, or Ser
<220>
<221> MISC_FEATURE
<222> (228)..(228)
<223> Xaa is Val, Met, Ile, Ala, Thr, Lys, Cys, Ser, Gly, Glu, Tyr, Pro, Asn,
Gln, Trp or His
<221> MISC_FEATURE
<222> (396)..(396)
<223> Xaa is Phe, Trp, Ser, Thr, Lys, Ala, Asn, His or Asp, provided that when
Xaa159 is Met, then xaa396 is not Phe
<400> 1

```
Met Thr Ala Gln Gln His Leu Ser Arg Arg Arg Met Leu Gly Met Ala
1               5                   10                  15
Ala Phe Gly Ala Ala Ala Leu Ala Gly Gly Thr Thr Ile Ala Ala Pro
            20                  25                  30
Arg Ala Ala Ala Ala Ala Lys Ser Ala Ala Asp Asn Gly Gly Tyr Val
        35                  40                  45
Pro Ala Val Val Ile Gly Thr Gly Tyr Gly Ala Ala Val Ser Ala Leu
    50                  55                  60
Arg Leu Gly Glu Ala Gly Val Gln Thr Leu Met Leu Glu Met Gly Gln
65                  70                  75                  80
Leu Trp Asn Gln Pro Gly Pro Asp Gly Asn Ile Phe Cys Gly Met Leu
                85                  90                  95
Asn Pro Asp Lys Arg Ser Ser Trp Phe Lys Asn Arg Thr Glu Ala Pro
            100                 105                 110
Leu Gly Ser Phe Leu Trp Leu Asp Val Val Asn Arg Asn Ile Asp Pro
        115                 120                 125
Tyr Ala Gly Val Leu Asp Arg Val Asn Tyr Asp Gln Met Ser Val Tyr
    130                 135                 140
Val Gly Arg Gly Val Gly Gly Gly Ser Leu Val Asn Gly Gly Xaa Ala
145                 150                 155                 160
Val Glu Pro Lys Arg Ser Tyr Phe Glu Glu Ile Leu Pro Arg Val Asp
            165                 170                 175
Ser Ser Glu Met Tyr Asp Arg Tyr Phe Pro Arg Ala Asn Ser Met Leu
        180                 185                 190
Arg Val Asn His Ile Asp Thr Lys Trp Phe Glu Asp Thr Glu Trp Tyr
        195                 200                 205
Lys Phe Ala Arg Val Ser Arg Glu Gln Ala Gly Lys Ala Gly Leu Gly
    210                 215                 220
Thr Val Phe Xaa Pro Asn Val Tyr Asp Phe Gly Tyr Met Gln Arg Glu
225                 230                 235                 240
Ala Ala Gly Glu Val Pro Lys Ser Ala Leu Ala Thr Glu Val Ile Tyr
            245                 250                 255
Gly Asn Asn His Gly Lys Gln Ser Leu Asp Lys Thr Tyr Leu Ala Ala
        260                 265                 270
Ala Leu Gly Thr Gly Lys Val Thr Ile Gln Thr Leu His Gln Val Lys
        275                 280                 285
Thr Ile Arg Gln Thr Lys Asp Gly Gly Tyr Ala Leu Thr Val Glu Gln
    290                 295                 300
Lys Asp Thr Asp Gly Lys Leu Leu Ala Thr Lys Glu Ile Ser Cys Arg
305                 310                 315                 320
Tyr Leu Phe Leu Gly Ala Gly Ser Leu Gly Ser Thr Glu Leu Leu Val
            325                 330                 335
```

```
Arg Ala Arg Asp Thr Gly Thr Leu Pro Asn Leu Asn Ser Glu Val Gly
            340                 345                 350
Ala Gly Trp Gly Pro Asn Gly Asn Ile Met Thr Ala Arg Ala Asn His
            355                 360                 365
Met Trp Asn Pro Thr Gly Ala His Gln Ser Ser Ile Pro Ala Leu Gly
    370                 375                 380
Ile Asp Ala Trp Asp Asn Ser Asp Ser Ser Val Xaa Ala Glu Ile Ala
385                 390                 395                 400
Pro Met Pro Ala Gly Leu Glu Thr Trp Val Ser Leu Tyr Leu Ala Ile
                405                 410                 415
Thr Lys Asn Pro Gln Arg Gly Thr Phe Val Tyr Asp Ala Ala Thr Asp
                420                 425                 430
Arg Ala Lys Leu Asn Trp Thr Arg Asp Gln Asn Ala Pro Ala Val Asn
            435                 440                 445
Ala Ala Lys Ala Leu Phe Asp Arg Ile Asn Lys Ala Asn Gly Thr Ile
            450                 455                 460
Tyr Arg Tyr Asp Leu Phe Gly Thr Gln Leu Lys Ala Phe Ala Asp Asp
465                 470                 475                 480
Phe Cys Tyr His Pro Leu Gly Gly Cys Val Leu Gly Lys Ala Thr Asp
                485                 490                 495
Asp Tyr Gly Arg Val Ala Gly Tyr Lys Asn Leu Tyr Val Thr Asp Gly
                500                 505                 510
Ser Leu Ile Pro Gly Ser Val Gly Val Asn Pro Phe Val Thr Ile Thr
        515                 520                 525
Ala Leu Ala Glu Arg Asn Val Glu Arg Ile Ile Lys Gln Asp Val Thr
        530                 535                 540
Ala Ser
545
```

<210> 2
<211> 542
<212> PRT
<213> Streptomyces virginiae
<220>
<221> MISC_FEATURE
<222> (155)..(155)
<223> Xaa is Met, Phe, Leu, Val, Cys, Ile, Ala, Gln, Tyr, Lys, or Ser
<220>
<221> MISC_FEATURE
<222> (224)..(224)
<223> Xaa is Val, Met, Ile, Ala, Thr, Lys, Cys, Ser, Gly, Glu, Tyr, Pro, Asn,
Gln, Trp or His
<221> MISC_FEATURE
<222> (392)..(392)
<223> Xaa is Phe, Trp, Ser, Thr, Lys, Ala, Asn, His or Asp, provided that when
xaa155 is Met, then xaa392 is not Phe
<400> 2

```
Met Glu Gln His Leu Ser Arg Arg Arg Leu Leu Gly Met Thr Ala Leu
1               5                   10              15
Gly Ala Ala Ala Leu Ala Gly Ser Thr Thr Ile Gly Ala Pro Arg Ala
            20                  25              30
Leu Ala Ala Asp Arg Ala Asp Gly Val Ala Phe Phe Pro Ala Val Val
        35              40              45
Ile Gly Thr Gly Tyr Gly Ala Ala Val Ser Ala Leu Arg Leu Gly Glu
    50              55              60
Ala Gly Val Arg Thr Val Met Leu Glu Met Gly Gln Leu Trp Asn Gln
65              70              75              80
Pro Gly Pro Asp Gly Asn Val Phe Ala Gly Met Leu Lys Pro Asp Lys
            85              90              95
Arg Ser Ser Trp Phe Lys Asn Arg Thr Glu Ala Pro Leu Gly Ser Phe
        100             105             110
Leu Trp Leu Asp Leu Ala Asn Arg Asp Ile Asp Pro Tyr Ala Gly Val
        115             120             125
Leu Asp Arg Val Asn Phe Asp Gln Met Ser Val Tyr Val Gly Arg Gly
    130             135             140
Val Gly Gly Gly Ser Leu Val Asn Gly Gly Xaa Ala Val Ala Pro Lys
145             150             155             160
Arg Ser Tyr Phe Glu Glu Val Leu Pro Arg Val Asp Ser Ala Glu Met
            165             170             175
```

```
Tyr Ser Arg Tyr Phe Pro Arg Ala Asn Ser Met Leu Arg Val Asn His
        180                 185                 190
Ile Asp Asp Gly Trp Phe Glu Ser Thr Glu Trp Tyr Lys Phe Ala Arg
        195                 200                 205
Val Ser Arg Asp Gln Ala Gln Lys Ala Gly Leu Gly Thr Val His Xaa
    210                 215                 220
Pro Asn Val Tyr Asp Phe Asp His Met Arg Arg Glu Ala Ala Gly Glu
225                 230                 235                 240
Ala Pro Lys Ser Ala Leu Ala Gly Glu Val Ile Tyr Gly Asn Asn His
            245                 250                 255
Gly Lys Gln Ser Leu Asp Lys Thr Tyr Leu Ala Ala Ala Leu Gly Thr
            260                 265                 270
Gly Lys Val Thr Ile Glu Thr Leu His Gln Ala Arg Thr Ile Arg Gln
        275                 280                 285
Gln Lys Asp Gly Thr Tyr Leu Leu Thr Val Glu Gln Arg Asp Ala Asp
    290                 295                 300
Gly Arg Leu Leu Ala Thr Lys Glu Ile Ser Cys Arg His Leu Phe Leu
305                 310                 315                 320
Gly Ala Gly Ser Leu Gly Ser Thr Glu Leu Leu Leu Arg Ala Arg Glu
            325                 330                 335
Thr Gly Thr Leu Pro Asp Leu Ser Ser Glu Ile Gly Ala Gly Trp Gly
            340                 345                 350
Pro Asn Gly Asn Ile Met Thr Ala Arg Ala Asn His Val Trp Asn Pro
        355                 360                 365
Thr Gly Ala Asn Gln Ser Ser Ile Pro Ala Leu Gly Ile Asp Asp Trp
    370                 375                 380
Asp Asn Pro Gln Asn Pro Val Xaa Ala Glu Ile Ala Pro Met Pro Ala
385                 390                 395                 400
Gly Leu Glu Thr Trp Val Ser Leu Tyr Leu Ala Ile Thr Lys Asn Pro
            405                 410                 415
Glu Arg Gly Thr Phe Ala Tyr Asp Ala Ala Thr Asp Arg Ala Ala Leu
            420                 425                 430
Arg Trp Thr Arg Asp Gln Asn Thr Pro Ala Val Ser Ala Ala Lys Ser
        435                 440                 445
Leu Phe Asp Arg Ile Asn Lys Ala Asn Thr Thr Met Tyr Arg Tyr Asp
    450                 455                 460
Leu Phe Gly Lys Gln Leu Lys Ala Phe Ser Asp Phe Thr Tyr His
465                 470                 475                 480
Pro Leu Gly Gly Cys Val Leu Gly Arg Ala Thr Asp Asp Tyr Gly Arg
            485                 490                 495
Val Lys Gly Tyr Lys Asn Leu Tyr Val Thr Asp Gly Ser Leu Ile Pro
            500                 505                 510
Gly Ser Ile Gly Val Asn Pro Phe Val Thr Ile Thr Ala Leu Ala Glu
            515                 520                 525
Arg Asn Ile Glu Arg Val Ile Arg Gln Asp Val Thr Ala Ala
        530                 535                 540
```

<210> 3
<211> 547
<212> PRT
<213> Streptomyces avermitilis

<220>
<221> MISC_FEATURE
<222> (160)..(160)
<223> Xaa is Met, Phe, Leu, val, Cys, Ile, Ala, Gln, Tyr, Lys, or Ser
<220>
<221> MISC_FEATURE
<222> (229)..(229)
<223> Xaa is Val, Met, Ile, Ala, Thr, Lys, Cys, Ser, Gly, Glu, Tyr, Pro, Asn,
Gln, Trp or His
<221> MISC_FEATURE
<222> (397)..(397)
<223> Xaa is Phe, Trp, Ser, Thr, Lys, Ala, Asn, His or Asp, provided that when
Xaa160 is Met, then Xaa397 is not Phe
<400> 3

22

```
Met Ile Ala His Gln Pro Leu Ser Arg Arg Arg Met Leu Gly Val Ala
1               5                   10                  15
Ala Leu Gly Ala Ala Ala Leu Ala Gly Gln Thr Thr Ile Thr Ala Ala
            20              25                  30
```

```
Pro Arg Ala Ala Ala Ala Thr Ala Thr Ser Gly Ser Gly Gly Thr Phe
        35                  40                  45
Val Pro Ala Val Val Val Gly Thr Gly Tyr Gly Ala Ala Val Ser Ala
        50                  55                  60
Leu Arg Leu Gly Glu Ala Gly Val Pro Thr Leu Met Leu Glu Met Gly
65                  70                  75                  80
Arg Leu Trp Asn Gln Pro Gly Pro Asp Gly Asn Val Phe Ser Gly Met
                85                  90                  95
Leu Lys Pro Asp Lys Arg Ser Ser Trp Phe Lys Thr Arg Thr Glu Ala
            100                 105                 110
Pro Leu Gly Ser Phe Leu Trp Leu Asp Leu Ala Asn Arg Asp Ile Glu
            115                 120                 125
Pro Tyr Ala Gly Val Leu Asp Arg Val Asn Phe Asp Gln Met Ser Val
    130                 135                 140
Tyr Leu Gly Arg Gly Val Gly Gly Gly Ser Leu Val Asn Gly Gly Xaa
145                 150                 155                 160
Ala Val Thr Pro Arg Arg Ser Tyr Phe Glu Glu Val Leu Pro Gln Val
                165                 170                 175
Asp Ala Glu Glu Met Tyr Thr Lys Tyr Phe Pro Arg Ala Asn Ser Thr
            180                 185                 190
Leu Arg Val Asn Asn Ile Asp Lys Ser Trp Phe Glu Gln Thr Asp Trp
        195                 200                 205
Tyr Ser Phe Ala Arg Val Ser Arg Arg Gln Ala Ser Asn Ala Gly Leu
    210                 215                 220
Ser Thr Thr Phe Xaa Pro Asn Val Tyr Asp Trp Asp Tyr Met Arg Arg
225                 230                 235                 240
Glu Ala Asp Gly Ala Val Pro Lys Ser Ala Leu Ala Ala Glu Val Ile
            245                 250                 255
Tyr Gly Asn Asn His Gly Lys Val Ser Leu Asp Lys Ser Tyr Leu Ala
            260                 265                 270
Ala Ala Leu Gly Thr Gly Lys Val Thr Ile Glu Thr Leu His Gln Val
    275                 280                 285
Lys Thr Ile Arg Gln Gln Asn Asp Gly Thr Tyr Leu Leu Thr Val Glu
    290                 295                 300
Gln Arg Asp Thr Gly Gly Lys Leu Leu Gly Thr Lys Glu Val Ser Cys
305                 310                 315                 320
Arg His Leu Phe Leu Gly Ala Gly Ser Leu Gly Ser Thr Glu Leu Leu
            325                 330                 335
Leu Arg Ala Arg Glu Thr Gly Thr Leu Pro Gly Leu Ser Pro Glu Val
        340                 345                 350
Gly Gly Gly Trp Gly Pro Asn Gly Asn Ile Met Thr Ala Arg Ala Asn
        355                 360                 365
His Met Trp Asn Pro Thr Gly Thr Lys Gln Ser Ser Ile Pro Ala Leu
    370                 375                 380
Gly Ile Asp Asp Trp Asp Asn Pro Asp Thr Pro Val Xaa Ala Glu Ile
385                 390                 395                 400
Ala Pro Leu Pro Ala Gly Val Glu Thr Trp Val Ser Leu Tyr Leu Ala
            405                 410                 415
Ile Thr Lys Asn Pro Glu Arg Gly Thr Phe Val Tyr Asp Ala Ala Lys
        420                 425                 430
Asp Arg Ala Asp Leu Arg Trp Thr Arg Asp Gln Asn Ala Pro Ala Ile
        435                 440                 445
Ala Ala Ala Lys Ser Leu Phe Asp Arg Ile Asn Lys Ala Asn Ala Thr
    450                 455                 460
Ile Tyr Arg Tyr Asp Leu Phe Gly Lys Gln Ile Lys Ala Phe Ala Asp
465                 470                 475                 480
Asp Phe Cys Tyr His Pro Leu Gly Gly Cys Val Leu Gly Lys Ala Thr
            485                 490                 495
Asp Asp Tyr Gly Arg Val Thr Gly Tyr Lys Asn Leu Tyr Val Thr Asp
        500                 505                 510
Gly Ser Leu Ile Pro Gly Ser Ile Gly Val Asn Pro Phe Val Thr Ile
        515                 520                 525
Ala Ala Leu Ala Glu Arg Asn Ile Glu Arg Val Ile Lys Gln Asp Ile
    530                 535                 540
```

Ala Asp ser
545

<210> 4

<211> 549

<212> PRT

<213> Streptomyces natalensis

<220>

<221> MISC_FEATURE

<222> (162)..(162)

<223> Xaa is Met, Phe, Leu, Val, Cys, Ile, Ala, Gln, Tyr, Lys, or Ser

<220>

<221> MISC_FEATURE

<222> (231)..(231)

<223> Xaa is Val, Met, Ile, Ala, Thr, Lys, Cys, Ser, Gly, Glu, Tyr, Pro, Asn, Gln, Trp or His

<221> MISC_FEATURE

<222> (399)..(399)

<223> Xaa is Phe, Trp, Ser, Thr, Lys, Ala, Asn, His or Asp, provided that when Xaa162 is Met, then Xaa399 is not Phe

<400> 4

```
Met Phe Glu Asn Gln His Leu Ser Arg Arg Arg Leu Leu Gly Leu Ala
1               5                   10                  15
Ala Leu Gly Gly Ala Ala Ala Ala Gly Met Thr Thr Ile Thr Ser Ala
            20                  25                  30
Pro His Ala Ala Ala Ala Asp Arg Arg Ser Pro Gln Ala Arg Ser Gly
        35                  40                  45
Ser Phe Val Pro Ala Val Val Ile Gly Thr Gly Tyr Gly Ala Ala Val
    50                  55                  60
Ser Ala Leu Arg Leu Gly Glu Ala Gly Ile Pro Thr Leu Met Leu Glu
65                  70                  75                  80
Met Gly Gln Leu Trp Asn Lys Pro Ala Asp Asp Gly Asn Val Phe Cys
                85                  90                  95
Gly Met Leu Ser Pro Asp Arg Arg Ser Ser Trp Phe Lys Ser Arg Thr
            100                 105                 110
Glu Ala Pro Leu Gly Ser Phe Leu Trp Leu Asp Val Ile Asn Arg Asp
        115                 120                 125
Ile Asp Pro Tyr Ala Gly Val Leu Asp Lys Val His Phe Asp Gln Met
    130                 135                 140
Ser Val Tyr Val Gly Arg Gly Val Gly Gly Gly Ser Leu Val Asn Gly
145                 150                 155                 160
Gly Xaa Ala Val Val Pro Lys Arg Ser Tyr Phe Glu Glu Val Leu Pro
                165                 170                 175
Arg Val Asp Ala Ala Glu Met Tyr Asp Arg Tyr Phe Pro Arg Ala Asn
            180                 185                 190
Ser Met Leu Lys Val Asn His Ile Asp Lys Gly Trp Phe Glu Glu Thr
        195                 200                 205
Glu Trp Tyr Lys Phe Ala Arg Val Ser Arg Glu Gln Ala Gly Lys Ala
    210                 215                 220
Gly Leu Gly Thr Thr Phe Xaa Pro Asn Val Tyr Asp Phe Asp Tyr Met
225                 230                 235                 240
Arg Arg Glu Ala Asn Gly Glu Ser Pro Lys Ser Ala Leu Ala Thr Glu
            245                 250                 255
Val Ile Tyr Gly Asn Asn His Gly Lys Gln Ser Leu Asp Lys Thr Tyr
        260                 265                 270
Leu Ala Ala Ala Leu Gly Thr Gly Lys Val Thr Ile Glu Thr Leu His
    275                 280                 285
Gln Val Arg Ala Ile His Gln Gln Pro Asp Gly Ser Tyr Val Leu Ser
    290                 295                 300
Val Asp Gln Ile Asp Thr Ala Gly Gln Thr Val Ala His Lys Glu Ile
305                 310                 315                 320
Ser Cys Arg His Leu Phe Leu Gly Ala Gly Ser Leu Gly Ser Thr Glu
            325                 330                 335
Leu Leu Val Arg Ala Arg Asp Thr Gly Ala Leu Pro Asp Leu Asn Ala
            340                 345                 350
Glu Val Gly Ala Gly Trp Gly Pro Asn Gly Asn Ile Met Thr Gly Arg
        355                 360                 365
Ala Asn His Val Trp Asn Pro Thr Gly Ala His Gln Ser Ser Ile Pro
    370                 375                 380
Ala Leu Gly Ile Asp Asp Trp Asn Asn Pro Thr Ala Pro Val Xaa Ala
385                 390                 395                 400
Glu Ile Ala Pro Met Pro Ala Gly Leu Glu Thr Trp Val Ser Leu Tyr
                405                 410                 415
```

```
Leu Ala Ile Thr Lys Asn Pro Glu Arg Gly Thr Phe Val Tyr Asp Lys
            420                 425                 430
Ala Thr Asp Arg Ala Ala Leu Arg Trp Thr Arg Asp Gln Asn Thr Pro
            435                 440                 445
Ala Val Asn Ala Ala Arg Ser Leu Phe Asp Arg Ile Asn Lys Ala Asn
        450                 455                 460
Gly Thr Met Tyr Arg Tyr Asp Leu Phe Gly Pro Gln Leu Lys Asn Phe
465                 470                 475                 480
Ser Asp Asp Phe Cys Tyr His Pro Leu Gly Gly Cys Val Leu Gly Lys
                485                 490                 495
Ala Thr Asp Gly Tyr Gly Arg Val Ala Gly Tyr His Asn Leu Tyr Val
            500                 505                 510
Thr Asp Gly Ala Leu Ile Pro Gly Ser Ile Gly Val Asn Pro Phe Val
            515                 520                 525
Thr Ile Thr Ala Leu Ala Glu Arg Asn Ile Glu Arg Ile Ile Ala Glu
    530                 535                 540
Asp Val Lys Ala Ala
545
```

<210> 5

<211> 552

<212> PRT

<213> Streptomyces chattanoogensis

<220>

<221> MISC_FEATURE

<222> (165)..(165)

<223> Xaa is Met, Phe, Leu, val, Cys, Ile, Ala, Gln, Tyr, Lys, or Ser

<220>

<221> MISC_FEATURE

<222> (234)..(234)

<223> Xaa is Val, Met, Ile, Ala, Thr, Lys, Cys, Ser, Gly, Glu, Tyr, Pro, Asn, Gln, Trp or His

<221> MISC_FEATURE

<222> (402)..(402)

<223> Xaa is Phe, Trp, Ser, Thr, Lys, Ala, Asn, His or Asp, provided that when Xaa165 is Met, then Xaa402 is not Phe

<400> 5

```
Met Phe Glu Asn Gln Gln Asn Gln His Leu Ser Arg Arg Arg Leu Leu
1               5               10              15
Gly Leu Ala Ala Leu Ser Gly Ala Ala Val Ala Gly Met Thr Thr Ile
            20              25              30
Ser Ala Ala Pro Arg Ala Ala Ala Ala Asp Lys Arg Ser Pro Lys Ala
        35              40              45
Gly Ser Gly Ser Phe Val Pro Ala Val Val Ile Gly Thr Gly Tyr Gly
    50              55              60
Ala Ala Val Ser Ala Leu Arg Leu Gly Glu Ala Gly Ile Pro Thr Leu
65              70              75              80
Met Leu Glu Met Gly Gln Leu Trp Asn Lys Pro Ala Asp Asp Gly Asn
            85              90              95
Val Phe Cys Gly Met Leu Lys Pro Asp Arg Arg Ser Ser Trp Phe Lys
        100             105             110
Ser Arg Thr Glu Ala Pro Leu Gly Ser Phe Leu Trp Leu Asp Val Ile
    115             120             125
Asn Arg Asp Ile Asp Pro Tyr Ala Gly Val Leu Asp Lys Val His Phe
    130             135             140
Asp Gln Met Ser Val Tyr Val Gly Arg Gly Val Gly Gly Gly Ser Leu
145             150             155             160
Val Asn Gly Gly Xaa Ala Val Val Pro Lys Arg Ser Tyr Phe Glu Glu
            165             170             175
Val Leu Pro Arg Val Asp Ala Ala Glu Met Tyr Asp Arg Tyr Phe Pro
        180             185             190
Arg Ala Asn Ser Met Leu Lys Val Asn His Ile Asp Lys Gly Trp Phe
    195             200             205
Glu Glu Thr Glu Trp Tyr Lys Phe Ala Arg Val Ser Arg Glu Gln Ala
210             215             220
Gly Lys Ala Gly Leu Ser Thr Thr Phe Xaa Pro Asn Val Tyr Asp Phe
225             230             235             240
Asp Tyr Met Arg Arg Glu Ala Asn Gly Glu Ser Pro Lys Ser Ala Leu
            245             250             255
```

```
Ala Thr Glu Val Ile Tyr Gly Asn Asn His Gly Lys Gln Ser Leu Asp
            260                 265             270
Lys Thr Tyr Leu Ala Ala Ala Leu Gly Thr Gly Lys Val Thr Ile Glu
        275             280             285
Thr Leu His Gln Val Lys Ala Ile His Gln Gln Pro Asp Gly Ser Tyr
    290             295             300
Val Leu Ser Val Asp Gln Ile Asp Thr Ala Gly Gln Thr Val Ala His
305             310             315             320
Lys Glu Ile Ala Cys Arg His Leu Phe Leu Gly Ala Gly Ser Leu Gly
                325             330             335
Ser Thr Glu Leu Leu Val Arg Ala Arg Asp Thr Gly Ala Leu Pro Asp
            340             345             350
Leu Asn Ala Glu Val Gly Ala Gly Trp Gly Pro Asn Gly Asn Ile Met
        355             360             365
Thr Gly Arg Ala Asn His Val Trp Asn Thr Thr Gly Ala His Gln Ser
    370             375             380
Ser Ile Pro Ala Leu Gly Ile Asp Asp Trp Asp Asn Pro Ala Ala Pro
385             390             395             400
Val Xaa Ala Glu Ile Ala Pro Met Pro Ala Gly Leu Glu Thr Trp Val
            405             410             415
Ser Leu Tyr Leu Ala Ile Thr Lys Asn Pro Glu Arg Gly Thr Phe Val
            420             425             430
Tyr Asp Lys Ala Thr Asp Arg Ala Ala Leu Arg Trp Thr Arg Asp Gln
            435             440             445
Asn Thr Pro Ala Val Asn Ala Ala Lys Ser Leu Phe Asp Arg Ile Asn
    450             455             460
Lys Ala Asn Thr Thr Met Tyr Arg Tyr Asp Leu Phe Gly Pro Gln Leu
465             470             475             480
Lys Asn Phe Ser Asp Asp Phe Cys Tyr His Pro Leu Gly Gly Cys Val
            485             490             495
Leu Gly Lys Ala Thr Asp Gly Tyr Gly Arg Val Ala Gly Tyr Arg Asn
        500             505             510
Leu Tyr Val Thr Asp Gly Ala Leu Ile Pro Gly Ser Ile Gly Val Asn
    515             520             525
Pro Phe Val Thr Ile Thr Ala Leu Ala Glu Arg Asn Ile Glu Arg Ile
    530             535             540
Ile Ala Glu Asp Val Lys Ala Ala
545             550
```

<210> 6

<211> 549

<212> PRT

<213> Streptomyces diastaticus

<220>

<221> MISC_FEATURE

<222> (162)..(162)

<223> Xaa is Met, Phe, Leu, Val, Cys, Ile, Ala, Gln, Tyr, Lys, or Ser

<220>

<221> MISC_FEATURE

<222> (231)..(231)

<223> Xaa is Val, Met, Ile, Ala, Thr, Lys, Cys, Ser, Gly, Glu, Tyr, Pro, Asn, Gln, Trp or His

<221> MISC_FEATURE

<222> (399)..(399)

<223> Xaa is Phe, Trp, Ser, Thr, Lys, Ala, Asn, His or Asp, provided that when xaa162 is Met, then xaa399 is not Phe

<400> 6

```
Met Ile Glu Asn Gln His Leu Ser Arg Arg Arg Leu Leu Gly Leu Ala
1               5               10              15
Ala Leu Gly Gly Ala Ala Val Ala Gly Met Thr Thr Ile Ser Val Ala
        20              25                      30
Pro Arg Ala Ala Ala Ala Gly Gln Gly Ser Pro Arg Ala Gly Asp Gly
        35              40              45
Ala Phe Val Pro Ala Val Val Ile Gly Thr Gly Tyr Gly Ala Ala Val
    50              55              60
Ser Ala Leu Arg Leu Gly Glu Ala Gly Ile Pro Thr Leu Met Leu Glu
65              70              75              80
Met Gly Gln Leu Trp Asn Lys Pro Ala Asp Asp Gly Asn Ile Phe Cys
                85              90                      95
```

```
Gly Met Leu Lys Pro Asp Arg Arg Ser Ser Trp Phe Lys Ser Arg Thr
            100                 105                 110
Glu Ala Pro Leu Gly Ser Phe Leu Trp Leu Asp Val Ile Asn Arg Asn
            115                 120                 125
Ile Asp Pro Tyr Ala Gly Val Leu Asp Lys Val His Phe Asp Glu Met
            130                 135                 140
Ser Val Tyr Val Gly Arg Gly Val Gly Gly Gly Ser Leu Val Asn Gly
145                 150                 155                 160
Gly Xaa Ala Val Val Pro Lys Arg Ser Tyr Phe Glu Glu Val Leu Pro
                165                 170                 175
Arg Val Asp Ala Ala Gln Met Tyr Asp Arg Tyr Phe Pro Arg Ala Asn
            180                 185                 190
Ser Met Leu Lys Val Asn His Ile Asp Lys Gly Trp Phe Glu Asp Thr
            195                 200                 205
Glu Trp Tyr Lys Tyr Ala Arg Val Ser Arg Glu Gln Ala Gly Lys Ala
    210                 215                 220
Gly Leu Ser Thr Thr Phe Xaa Pro Asn Val Tyr Asp Phe Asp His Met
225                 230                 235                 240
Arg Arg Glu Ala Asp Gly Thr Ala Pro Lys Ser Ala Leu Ala Gly Glu
                245                 250                 255
Val Ile Tyr Gly Asn Asn His Gly Lys Gln Ser Leu Asp Lys Thr Tyr
                260                 265                 270
Leu Ala Ala Ala Leu Gly Thr Gly Lys Val Thr Ile Glu Thr Leu His
            275                 280                 285
Gln Val Lys Ala Ile Arg Arg Gln Pro Asp Gly Ser Tyr Val Leu Ser
    290                 295                 300
Val Val Gln Ser Asp Ala Asp Gly Lys Thr Ile Ala Gln Lys Glu Ile
305                 310                 315                 320
Gly Cys Arg His Leu Phe Leu Gly Ala Gly Ser Leu Gly Ser Thr Glu
                325                 330                 335
Leu Leu Val Arg Ala Arg Asp Thr Gly Thr Leu Pro Glu Leu Asn Ala
            340                 345                 350
Glu Val Gly Ala Gly Trp Gly Pro Asn Gly Asn Ile Met Thr Gly Arg
            355                 360                 365
Ala Asn His Val Trp Asn Pro Thr Gly Ala His Gln Ser Ser Ile Pro
    370                 375                 380
Ala Leu Gly Ile Asp Asp Trp Asp Asn Pro Ala Ala Pro Val Xaa Ala
385                 390                 395                 400
Glu Ile Ala Pro Met Pro Ala Gly Leu Glu Thr Trp Val Ser Leu Tyr
                405                 410                 415
Leu Ala Ile Thr Lys Asn Pro Glu Arg Gly Thr Phe Val Tyr Asp Lys
            420                 425                 430
Ala Thr Asp Arg Ala Lys Leu Arg Trp Thr Arg Asp Gln Asn Thr Pro
            435                 440                 445
Ala Val Asn Ala Ala Lys Ser Leu Phe Asp Arg Ile Asn Lys Ala Asn
    450                 455                 460
Thr Thr Met Tyr Arg Tyr Asp Leu Phe Gly Ser Gln Leu Lys Asn Phe
465                 470                 475                 480
Ser Asp Asp Phe Ser Tyr His Pro Leu Gly Gly Cys Val Leu Gly Lys
                485                 490                 495
Ala Thr Asp Leu Tyr Gly Arg Val Ala Gly Tyr Gln Asn Leu Tyr Val
            500                 505                 510
Met Asp Gly Ala Leu Val Pro Gly Ser Ile Gly Val Asn Pro Phe Val
            515                 520                 525
Thr Ile Thr Ala Leu Ala Glu Arg Asn Ile Glu Arg Ile Ile Ala Glu
    530                 535                 540
Asp Val Lys Ala Ala
545
```

<210> 7
<211> 552
<212> PRT
<213> Streptomyces griseus
<220>
<221> MISC_FEATURE
<222> (165)..(165)
<223> Xaa is Met, Phe, Leu, Val, Cys, Ile, Ala, Gln, Tyr, Lys, or ser

<220>
<221> MISC_FEATURE
<222> (234)..(234)
<223> Xaa is Val, Met, Ile, Ala, Thr, Lys, Cys, Ser, Gly, Glu, Tyr, Pro, Asn, Gln, Trp or His
<221> MISC_FEATURE
<222> (402)..(402)
<223> Xaa is Phe, Trp, Ser, Thr, Lys, Ala, Asn, His or Asp, provided that when Xaa165 is Met, then xaa402 is not Phe
<400> 7

```
Met Phe Glu Asn Gln Gln Asn Gln His Leu Ser Arg Arg Arg Leu Leu
1               5                   10                  15
Gly Leu Ala Ala Leu Ser Gly Ala Ala Val Ala Gly Leu Thr Thr Ile
            20                  25                  30
Ser Ala Ala Pro Gln Ala Ala Ala Ala Gly Arg Arg Ala Pro Arg Ala
        35                  40                  45
Gly Asp Gly Ser Phe Val Glu Ala Val Val Ile Gly Thr Gly Tyr Gly
    50                  55                  60
Ala Ala Val Ser Ala Leu Arg Leu Gly Glu Ala Gly Val Pro Thr Leu
65                  70                  75                  80
Met Leu Glu Met Gly Arg Leu Trp Asn Lys Pro Ala Glu Asp Gly Asn
                85                  90                  95
Ile Phe Cys Gly Met Leu Lys Pro Asp Arg Arg Ser Thr Trp Phe Lys
            100                 105                 110
Ser Arg Thr Glu Ala Pro Leu Gly Ser Phe Leu Trp Leu Asp Val Val
        115                 120                 125
Asn Arg Asp Ile Asp Pro Tyr Ala Gly Val Leu Asp Arg Val His Phe
    130                 135                 140
Asp Glu Met Ser Val Tyr Val Gly Arg Gly Val Gly Gly Gly Ser Leu
145                 150                 155                 160
Val Asn Gly Gly Xaa Ala Val Val Pro Lys Arg Ala Tyr Phe Glu Glu
                165                 170                 175
Val Leu Pro Lys Val Asp Ala Ala Glu Met Tyr Asp Arg Tyr Phe Pro
            180                 185                 190
Arg Ala Asn Ser Met Leu Lys Val Asn His Ile Asp Lys Thr Trp Phe
        195                 200                 205
Glu Asp Thr Glu Trp Tyr Lys Phe Ala Arg Val Ser Arg Glu Gln Ala
    210                 215                 220
Ser Lys Ala Gly Leu Gly Thr Thr Phe Xaa Pro Asn Val Tyr Asp Phe
225                 230                 235                 240
Gly His Met Arg Arg Glu Ala Thr Gly Glu Ala Pro Lys Ser Ala Leu
                245                 250                 255
Ala Gly Glu Val Ile Tyr Gly Asn Asn His Gly Lys Gln Ser Leu Asp
            260                 265                 270
Lys Thr Tyr Leu Ala Ala Ala Leu Gly Thr Gly Lys Val Thr Ile Glu
        275                 280                 285
Thr Leu His Gln Val Lys Ala Ile Arg Gln Gln Lys Asp Gly Gly Tyr
    290                 295                 300
Val Leu Ser Val Asp Gln Thr Asp Ala Asp Gly Lys Thr Val Gly His
305                 310                 315                 320
Lys Glu Ile Gly Cys Arg His Leu Phe Leu Gly Ala Gly Ser Leu Gly
                325                 330                 335
Ser Thr Glu Leu Leu Val Arg Ala Arg Asp Thr Gly Ala Leu Pro Asp
            340                 345                 350
Leu Asn Ala Glu Val Gly Gly Gly Trp Gly Pro Asn Gly Asn Ile Met
        355                 360                 365
Thr Gly Arg Ala Asn His Val Trp Asn Pro Thr Gly Ala His Gln Ser
    370                 375                 380
Ser Ile Pro Ala Leu Gly Ile Asp Asp Trp Asn Asn Ala Ala Ala Pro
385                 390                 395                 400
Val Xaa Ala Glu Ile Ala Pro Met Pro Ala Gly Ala Glu Thr Trp Val
                405                 410                 415
Ser Leu Tyr Leu Ala Ile Thr Lys Asn Pro Glu Arg Gly Thr Phe Val
            420                 425                 430
Tyr Asp Lys Ala Thr Asp Arg Val Ala Leu Arg Trp Thr Arg Asp Gln
        435                 440                 445
Asn Thr Pro Ala Val Asn Ala Ala Lys Ser Leu Phe Asp Arg Ile Asn
    450                 455                 460
Gln Ala Asn Thr Thr Val Tyr Arg Tyr Asp Leu Phe Gly Lys Gln Val
465                 470                 475                 480
```

```
Lys Ala Phe Ser Asp Asp Phe Ser Tyr His Pro Leu Gly Gly Cys Val
                485                     490                     495
Leu Gly Lys Ala Thr Asp Leu Tyr Gly Arg Val Ala Gly His Arg Asn
            500                     505                     510
Leu Tyr Val Met Asp Gly Ser Leu Ile Pro Gly Ser Ile Gly Val Asn
        515                     520                     525
Pro Phe Val Thr Ile Thr Ala Leu Ala Glu Arg Asn Ile Glu Arg Ile
    530                     535                     540
Ile Ala Glu Asp Val Lys Ala Ala
545                     550
```

<210> 8
<211> 547
<212> PRT
<213> Streptomyces sp.
<220>
<221> MISC_FEATURE
<222> (160)..(160)
<223> Xaa is Met, Phe, Leu, Val, Cys, Ile, Ala, Gln, Tyr, Lys, or Ser
<220>
<221> MISC_FEATURE
<222> (229)..(229)
<223> Xaa is Val, Met, Ile, Ala, Thr, Lys, Cys, Ser, Gly, Glu, Tyr, Pro, Asn, Gln, Trp or His
<221> MISC_FEATURE
<222> (397)..(397)
<223> Xaa is Phe, Trp, Ser, Thr, Lys, Ala, Asn, His or Asp, provided that when xaa160 is Met, then xaa397 is not Phe
<400> 8

```
Met Asn Ala His Gln Pro Leu Ser Arg Arg Arg Met Leu Gly Leu Ala
1               5                   10                  15
Ala Leu Gly Ala Ala Ala Leu Thr Gly Gln Thr Thr Ile Thr Ala Ala
            20                  25                  30
Pro Arg Ala Ala Ala Thr Ala Pro Gly Gly Ser Gly Gly Thr Phe
        35                  40                  45
Val Pro Ala Val Val Ile Gly Thr Gly Tyr Gly Ala Ala Val Ser Ala
    50                  55                  60
Leu Arg Leu Gly Glu Ala Gly Val Ser Thr Leu Met Leu Glu Met Gly
65                  70                  75                  80
Gln Leu Trp Asn Gln Pro Gly Pro Asp Gly Asn Val Phe Cys Gly Met
                85                  90                  95
Leu Lys Pro Asp Lys Arg Ser Ser Trp Phe Lys Thr Arg Thr Glu Ala
            100                 105                 110
Pro Leu Gly Ser Phe Leu Trp Leu Asp Leu Ala Asn Arg Asp Ile Asp
        115                 120                 125
Pro Tyr Ala Gly Val Leu Asp Arg Val Asn Phe Asp Gln Met Ser Val
    130                 135                 140
Tyr Val Gly Arg Gly Val Gly Gly Gly Ser Leu Val Asn Gly Gly Xaa
145                 150                 155                 160
Ala Val Thr Pro Arg Arg Ser Tyr Phe Gln Glu Val Leu Pro Gln Val
            165                 170                 175
Asp Ala Asp Glu Met Tyr Gly Thr Tyr Phe Pro Arg Ala Asn Ser Gly
        180                 185                 190
Leu Arg Val Asn Asn Ile Asp Lys Asp Trp Phe Glu Gln Thr Glu Trp
        195                 200                 205
Tyr Thr Phe Ala Arg Val Ala Arg Leu Gln Ala Glu Asn Ala Gly Leu
    210                 215                 220
Lys Thr Thr Phe Xaa Pro Asn Val Tyr Asp Trp Asp Tyr Met Arg Gly
225                 230                 235                 240
Glu Ala Asp Gly Thr Asn Pro Lys Ser Ala Leu Ala Ala Glu Val Ile
            245                 250                 255
Tyr Gly Asn Asn His Gly Lys Val Ser Leu Asp Lys Ser Tyr Leu Ala
        260                 265                 270
Ala Ala Leu Gly Thr Gly Lys Val Thr Val Glu Thr Leu His Gln Val
    275                 280                 285
Lys Thr Ile Arg Gln Gln Asn Asp Gly Thr Tyr Leu Leu Thr Val Glu
290                 295                 300
Gln Lys Asp Pro Asp Gly Lys Leu Leu Gly Thr Lys Glu Ile Ser Cys
305                 310                 315                 320
```

35

```
Arg His Leu Phe Leu Gly Ala Gly Ser Leu Gly Ser Ile Glu Leu Leu
            325             330             335
Leu Arg Ala Arg Glu Thr Gly Thr Leu Pro Gly Leu Ser Ser Glu Ile
            340             345             350
Gly Gly Gly Trp Gly Pro Asn Gly Asn Ile Met Thr Ala Arg Ala Asn
            355             360             365
His Val Trp Asn Pro Thr Gly Ser Lys Gln Ser Ser Ile Pro Ala Leu
370             375             380
Gly Ile Asp Asp Trp Asp Asn Pro Asp Asn Pro Val Xaa Ala Glu Ile
385             390             395             400
Ala Pro Met Pro Ala Gly Leu Glu Thr Trp Val Ser Leu Tyr Leu Ala
            405             410             415
Ile Thr Lys Asn Pro Glu Arg Gly Thr Phe Val Tyr Asp Ala Ala Lys
            420             425             430
Asp Arg Ala Asp Leu Arg Trp Thr Arg Asp Gln Asn Ala Pro Ala Val
            435             440             445
Ala Ala Ala Lys Ser Leu Phe Asp Arg Val Asn Lys Ala Asn Thr Thr
    450             455             460
Ile Tyr Arg Tyr Asp Leu Phe Gly Lys Gln Ile Lys Ala Phe Ala Asp
465             470             475             480
Asp Phe Cys Tyr His Pro Leu Gly Gly Cys Val Leu Gly Lys Ala Thr
            485             490             495
Asp Asn Tyr Gly Arg Val Ser Gly Tyr Lys Asn Leu Tyr Val Thr Asp
            500             505             510
Gly Ser Leu Ile Pro Gly Ser Ile Gly Val Asn Pro Phe Val Thr Ile
    515             520             525
Thr Ala Leu Ala Glu Arg Asn Val Glu Arg Val Ile Lys Glu Asp Ile
    530             535             540
Ala Gly Ser
545
```

<210> 9

<211> 543

<212> PRT

<213> Streptomyces sp.

<220>

<221> MISC_FEATURE

<222> (157)..(157)

<223> Xaa is Met, Phe, Leu, Val, Cys, Ile, Ala, Gln, Tyr, Lys, or ser

<220>

<221> MISC_FEATURE

<222> (226)..(226)

<223> Xaa is Val, Met, Ile, Ala, Thr, Lys, Cys, Ser, Gly, Glu, Tyr, Pro, Asn,
Gln, Trp or His

<221> MISC_FEATURE

<222> (394)..(394)

<223> Xaa is Phe, Trp, Ser, Thr, Lys, Ala, Asn, His or Asp, provided that when
xaa157 is Met, then xaa394 is not Phe

<400> 9

```
Met Pro Gly Met Ala Ser Leu Asn Arg Arg Arg Phe Leu Gly Leu Ala
1               5                   10                  15
Ala Leu Asn Ser Ala Ala Ala Leu Gly Leu Thr Ser Ile Ser Ala Thr
            20                  25                  30
Thr Ala Arg Ala Ala Thr Val Ala Pro Leu Pro Asp Tyr Ser Pro Ala
        35                  40                  45
Val Val Ile Gly Thr Gly Tyr Gly Ala Ala Val Thr Ala Leu Arg Leu
    50                  55                  60
Gly Glu Ala Gly Val Pro Thr Val Met Leu Glu Met Gly Gln Leu Trp
65                  70                  75                  80
Asn Glu Ala Gly Pro Asp Gly Lys Val Phe Cys Asp Met Leu Lys Pro
            85                  90                  95
Asp Arg Arg Ser Met Trp Phe Lys Lys Arg Thr Glu Ala Pro Leu Ala
            100                 105                 110
Ser Phe Leu Trp Leu Asp Val Ala Asn His Asp Ile Asp Pro Tyr Ala
        115                 120                 125
Gly Val Leu Asp Arg Val Asn Tyr Gly Gly Met Ser Val Tyr Val Gly
    130                 135                 140
Arg Gly Val Gly Gly Gly Ser Leu Val Asn Gly Ala Xaa Ala Val Gln
145                 150                 155                 160
```

```
Pro Lys Arg Ser Tyr Phe Glu Glu Ile Leu Pro Arg Val Asp Ala Asp
            165                 170                     175
Glu Met Tyr Gly Lys Tyr Tyr Pro Arg Ala Asn Ala Gly Leu Gly Val
            180                 185                 190
Asn His Ile Asp Pro Asp Trp Phe Glu Thr Cys Lys Ser Tyr Gln Phe
            195                 200                 205
Ala Arg Val Ser Arg Lys Ala Ala Gln Lys Thr Gly Leu Lys Thr Thr
            210                 215                 220
Phe Xaa Pro Ser Val Tyr Asp Phe Glu Tyr Met Lys Lys Glu Glu Ala
225                     230                 235                 240
Gly Thr Val Glu Arg Ser Ala Leu Ala Ser Glu Val Ile Tyr Gly Asn
            245                 250                     255
Asn His Gly Lys Arg Ser Leu Asp Lys Thr Tyr Leu Ala Ala Ala Leu
            260                 265                 270
Gly Thr Gly His Val Thr Ile Gln Thr Leu His Glu Val Arg Glu Ile
            275                 280                 285
Ile Gln Gln Asp Gly Thr Tyr Thr Leu Val Val Arg Glu Ser Asp
    290                 295                 300
Ala Leu Gly Asn Val Leu Ala Thr Lys His Leu Ser Thr Lys Tyr Leu
305                     310                 315                 320
Phe Leu Gly Ala Gly Ser Leu Gly Ser Thr Glu Leu Leu Val Arg Ala
                325                 330                 335
Arg Asp Thr Gly Arg Leu Pro Arg Leu Ser Glu Ala Val Gly Gln Gly
            340                 345                 350
Trp Gly Thr Asn Gly Asn Val Met Leu Gly Arg Ala Asn His Val Trp
            355                 360                 365
Asp Thr Thr Gly Ser Leu Glu Ser Gly Met Pro Ala Leu Gly Ile Asp
    370                 375                 380
Asp Trp Asp Asn Pro Ala His Pro Val Xaa Ala Glu Ile Ala Pro Val
385                 390                 395                 400
Pro Ala Gly Leu Glu Thr Trp Ala Ser Leu Tyr Leu Ala Ile Thr Lys
                405                 410                 415
Asn Pro Glu Arg Gly His Phe Thr Tyr Asp Ala Ala Ser Asp Ser Ala
            420                 425                 430
Lys Leu Gln Trp Ser Pro Asp Gln Gly Gln Pro Ser Ile Asp Ala Ala
            435                 440                 445
Lys Ser Leu Phe Asp Arg Ile Asn Lys Ala Asn Ser Thr Ile Tyr Arg
    450                 455                 460
Tyr Asp Leu Phe Gly Asp Thr Arg Ala Phe Glu Asn Arg Phe Thr Tyr
465                 470                 475                 480
His Pro Leu Gly Gly Leu Val Leu Gly Glu Ala Thr Asp Asp Tyr Gly
                485                 490                 495
Arg Val Lys Gly Tyr Arg Asn Leu Tyr Val Thr Asp Gly Ser Leu Ile
            500                 505                 510
Pro Gly Ser Thr Gly Val Asn Pro Phe Val Thr Ile Thr Ala Leu Ala
            515                 520                 525
Glu Arg Asn Ile Glu Arg Val Leu Ala Glu Asp Gly Val Arg Ala
    530                 535                 540
```

<210> 10

<211> 547

<212> PRT

<213> Streptomyces sp.

<220>

<221> MISC_FEATURE

<222> (165)..(165)

<223> Xaa is Met, Phe, Leu, Val, Cys, Ile, Ala, Gln, Tyr, Lys, or Ser

<220>

<221> MISC_FEATURE

<222> (234)..(234)

<223> Xaa is Val, Met, Ile, Ala, Thr, Lys, Cys, Ser, Gly, Glu, Tyr, Pro, Asn, Gln, Trp or His

<221> MISC_FEATURE

<222> (402)..(402)

<223> Xaa is Phe, Trp, Ser, Thr, Lys, Ala, Asn, His or Asp, provided that when xaa165 is Met, then xaa402 is not Phe

<400> 10

```
Met Ala Gly Trp Ala Glu Ser Ala His Asp Ala Ala Met Thr His Asn
1               5                   10                  15
```

Leu Thr Arg Arg Gln Leu Gly Leu Asn Ala Leu Arg Ala Ala Ala Ala
20 25 30
Leu Gly Ile Thr Arg Ile Gly Leu Gly Ala Ala Ala Ala Glu Pro
35 40 45
Pro Ala Pro Tyr Ala Pro Ala Val Val Val Gly Ser Gly Tyr Gly
50 55 60
Ser Ala Val Ala Ala Leu Arg Leu Gly Gln Ala Gly Val Arg Thr Val
65 70 75 80
Val Leu Glu Met Gly Arg Leu Trp Asp Thr Pro Gly Pro Asp Gly Lys
85 90 95
Val Phe Pro Ser Thr Ser Ala Pro Asp Gln Arg Ser Met Trp Phe Arg
100 105 110
Thr Arg Thr Glu Ala Pro Leu Ala Gln Phe Leu Trp Leu Asp Val Val
115 120 125
Asn Arg Asp Ile Ser Pro Tyr Pro Gly Val Leu Asp Arg Val Asn His
130 135 140
Gly Gly Met Ser Val Tyr Val Gly Arg Gly Val Gly Gly Gly Ser Leu
145 150 155 160
Val Asn Gly Gly Xaa Ala Pro Thr Pro Arg Arg Ser Tyr Phe Ala Glu
165 170 175
Val Phe Pro Arg Val Asp Ala Glu Glu Met Tyr Gly Thr Tyr Phe Pro
180 185 190
Arg Ala Arg Ala Met Leu Gly Val Asn Gly Ile Asp Pro Ala Trp Phe
195 200 205
Glu Ser Thr Glu Trp Tyr Arg Phe Ala Arg Ile Ser Arg Lys His Ala
210 215 220
Gln Asn Thr Gly Leu Lys Thr Thr Phe Xaa Pro Asn Val Tyr Asp Phe
225 230 235 240
Gly Tyr Met Lys Arg Glu Ala Ala Gly Thr Ala Thr Arg Ser Ala Leu
245 250 255
Ala Gly Glu Val Ile Tyr Gly Asn Asn His Gly Lys Lys Ser Val Asp
260 265 270
Lys Thr Tyr Leu Ala Ala Ala Leu Gly Thr Gly Asn Val Thr Ile Glu
275 280 285
Thr Met Gln Arg Val Val Ala Val Arg Gln Asp Leu Ala Gly Gly Tyr
290 295 300
Val Leu Thr Val His Thr Ser Asp Val Thr Gly Arg Val Thr Gln Val
305 310 315 320
Arg Glu Leu Gly Cys Arg Gln Leu Phe Leu Gly Ala Gly Ser Leu Gly
325 330 335
Thr Thr Glu Ile Leu Leu Arg Ala Arg Glu Thr Gly Ala Leu Pro Ala
340 345 350
Leu Ser Glu Lys Val Gly Leu Gly Trp Gly Pro Asn Gly Asn Val Met
355 360 365
Thr Ala Arg Ala Asn His Leu Trp Asp Thr Val Gly Cys Asn Gln Ala
370 375 380
Thr Met Pro Ala Leu Gly Ile Asp Asp Trp Asp Asn Ala Ala Asn Pro
385 390 395 400
Val Xaa Ala Glu Ile Ala Pro Leu Pro Met Gly Ile Glu His Trp Ile
405 410 415
Ser Met Tyr Leu Ala Ile Thr Lys Asn Pro Glu Arg Gly Arg Phe Val
420 425 430
Tyr Asp Ala Ala Thr Asp Ser Ala Arg Leu Asn Trp Thr Arg Asp Gln
435 440 445
Asn Ala Pro Ala Val Ala Ala Lys Asn Leu Phe Asp Arg Ile Asn
450 455 460
Arg Arg Asn Val Thr Ile Tyr Arg Tyr Asp Leu Phe Gly Asp Asn Lys
465 470 475 480
Ala Phe Ala Asp Asp Phe Thr Tyr His Pro Leu Gly Gly Cys Val Leu
485 490 495
Gly Glu Ala Thr Asp Ala Tyr Gly Arg Val Lys Gly Tyr Gln Gly Leu
500 505 510
Tyr Val Leu Asp Gly Ser Leu Val Pro Gly Ser Leu Gly Val Asn Pro
515 520 525
Phe Val Thr Ile Thr Ala Leu Ala Glu Arg Asn Met Glu Arg Ile Leu
530 535 540
Ala Gln Pro
545

<210> 11
<211> 550
<212> PRT
<213> Streptomyces sp.
<220>
<221> MISC_FEATURE
<222> (166)..(166)
<223> Xaa is Met, Phe, Leu, Val, Cys, Ile, Ala, Gln, Tyr, Lys, or Ser
<220>
<221> MISC_FEATURE
<222> (235)..(235)
<223> Xaa is Val, Met, Ile, Ala, Thr, Lys, Cys, Ser, Gly, Glu, Tyr, Pro, Asn, Gln, Trp or His
<221> MISC_FEATURE
<222> (403)..(403)
<223> Xaa is Phe, Trp, Ser, Thr, Lys, Ala, Asn, His or Asp, provided that when xaa166 is Met, then xaa403 is not Phe
<400> 11

```
Met Gly Gly Arg Arg Glu Ser Ala His Asp Ala Ala Met Thr Ser Asn
1               5               10              15
Leu Thr Arg Arg Gln Met Leu Gly Leu Gly Ala Leu Ser Thr Ala Ala
        20              25              30
Ala Leu Gly Phe Thr Arg Ile Gly Ala Ala Ser Ala Ala Ala Leu Glu
        35              40              45
Pro Pro Ala Ala Ser Tyr Ala Pro Ala Ile Val Val Gly Ser Gly Tyr
    50              55              60
Gly Ser Ala Val Ala Ala Leu Arg Leu Gly Gln Ala Gly Val Arg Thr
65              70              75              80
Val Val Leu Glu Met Gly Arg Leu Trp Asp Thr Pro Gly Ala Asp Gly
            85              90              95
Lys Val Phe Pro Ser Thr Ser Ala Pro Asp Gln Arg Ser Met Trp Phe
            100             105             110
Arg Asn Arg Thr Glu Ala Pro Leu Ala Gln Phe Leu Trp Leu Asp Val
        115             120             125
Val Asn Arg Asp Ile Ser Pro Tyr Pro Gly Val Leu Asp Arg Val Asn
    130             135             140
Tyr Gly Asp Met Ser Val Tyr Val Gly Arg Gly Val Gly Gly Gly Ser
145             150             155             160
Leu Val Asn Gly Gly Xaa Ser Pro Thr Pro Arg Arg Ser Tyr Phe Ser
            165             170             175
Glu Val Leu Pro Arg Val Asp Ala Asp Glu Met Tyr Gly Thr Tyr Tyr
        180             185             190
Pro Arg Ala Arg Ala Met Leu Gly Val Gly Asp Ile Asp Pro Ala Trp
        195             200             205
Phe Glu Ser Thr Glu Trp Tyr Arg Phe Ala Arg Ile Ser Arg Lys His
    210             215             220
Ala Gln Asn Thr Gly Leu Lys Thr Val Phe Xaa Pro Asn Val Tyr Asp
225             230             235             240
Phe Glu Tyr Met Lys Arg Glu Ala Ala Gly Thr Ala Thr Arg Ser Ala
            245             250             255
Leu Ala Gly Glu Val Ile Tyr Gly Asn Asn His Gly Lys Lys Ser Val
            260             265             270
Asp Lys Thr Tyr Leu Ala Ala Ala Ile Gly Thr Gly Asn Val Thr Ile
        275             280             285
Glu Thr Met Gln Arg Val Val Ala Val Arg Gln Asp Pro Ala Gly Gly
    290             295             300
Tyr Val Leu Thr Val Arg Thr Ser Asp Val Thr Gly Arg Val Thr Gln
305             310             315             320
Val Arg Glu Leu Gly Cys Arg Arg Leu Phe Leu Gly Ala Gly Ser Leu
            325             330             335
Gly Thr Thr Glu Ile Leu Leu Arg Ala Arg Glu Thr Gly Thr Leu Pro
            340             345             350
Ala Leu Ser Glu Lys Val Gly Leu Gly Trp Gly Pro Asn Gly Asn Val
        355             360             365
Met Thr Ala Arg Ala Asn His Leu Trp Asp Thr Val Gly Ser Asn Gln
370             375             380
Ala Thr Met Pro Ala Leu Gly Ile Asp Asp Trp Asp Asn Ala Ala Asn
385             390             395             400
```

```
Pro Val Xaa Ala Glu Ile Ala Pro Leu Pro Met Gly Phe Glu His Trp
            405                     410                 415
Ile Ser Met Tyr Leu Ala Ile Thr Lys Asn Pro Glu Arg Gly His Phe
            420                     425                 430
Thr Tyr Asp Ala Ala Ser Asp Ser Ala Arg Leu Gln Trp Arg Arg Asp
            435                     440                 445
Gln Asn Thr Pro Ala Val Arg Ala Ala Lys Asn Leu Phe Asp Arg Ile
    450                     455                 460
Asn Arg Ala Asn Phe Thr Ile Tyr Arg Tyr Asp Leu Phe Gly Gly Asn
465                     470                 475                 480
Lys Asn Phe Ala Asp Asp Phe Thr Tyr His Pro Leu Gly Gly Cys Val
            485                     490                 495
Leu Gly Glu Ala Thr Asp Asp Phe Gly Arg Ala Lys Gly Tyr Gln Gly
            500                     505                 510
Leu Tyr Val Val Asp Gly Ser Leu Val Pro Gly Ser Leu Gly Val Asn
            515                     520                 525
Pro Phe Val Thr Ile Thr Ala Leu Ala Glu Arg Asn Met Ala Arg Ile
    530                     535                 540
Leu Ala Gln Asp Pro His
545                 550
```

<210> 12

<211> 540

<212> PRT

<213> Streptomyces scabiei

<220>

<221> MISC_FEATURE

<222> (154)..(154)

<223> Xaa is Met, Phe, Leu, Val, Cys, Ile, Ala, Gln, Tyr, Lys, or Ser

<220>

<221> MISC_FEATURE

<222> (223)..(223)

<223> Xaa is Val, Met, Ile, Ala, Thr, Lys, Cys, Ser, Gly, Glu, Tyr, Pro, Asn, Gln, Trp or His

<221> MISC_FEATURE

<222> (391)..(391)

<223> Xaa is Phe, Trp, Ser, Thr, Lys, Ala, Asn, His or Asp, provided that when Xaa154 is Met, then Xaa391 is not Phe

<400> 12

```
Met Gln Arg Gln Leu Thr Arg Arg His Ile Leu Gly Met Ala Ala Leu
1               5                   10                  15
Gln Thr Ala Ala Gly Leu Gly Leu Thr Arg Ile Gly Leu Gln Ser Ala
            20                  25                  30
Arg Ala Ala Glu Pro Asp Ala Val Asp Asn Ala Pro Ala Leu Val Ile
        35                  40                  45
Gly Ser Gly Tyr Gly Ala Ala Val Ala Ala Leu Arg Leu Gly Gln Ala
    50                  55                  60
Gly Ile Arg Thr Leu Val Leu Glu Met Gly Arg Ala Trp Thr Thr Pro
65                  70                  75                  80
Gly Ala Asp Gly Lys Ile Phe Cys Ser Thr Lys Glu Pro Asp Glu Arg
                85                  90                  95
Ser Met Trp Phe Lys Thr Arg Thr Glu Ala Pro Leu Ala Thr Phe Leu
            100                 105                 110
Trp Leu Asp Val Val Asn Gln Asp Ile Ser Arg Tyr Pro Gly Val Leu
        115                 120                 125
Asp Arg Val Arg His Ala Asn Met Ser Val Phe Leu Gly Arg Gly Val
    130                 135                 140
Gly Gly Gly Ser Leu Val Asn Gly Ser Xaa Ala Val Thr Pro Leu Arg
145                 150                 155                 160
Ser Tyr Phe Ala Glu Gln Phe Pro Thr Val Asp Thr Ala Glu Met Tyr
            165                 170                 175
Ser Thr Tyr Phe Pro Arg Ala Arg Ser Met Leu Gly Val Asn Thr Val
            180                 185                 190
Asp Pro Ala Trp Phe Glu Ser Thr Glu Trp Tyr Arg Phe Ser Arg Val
        195                 200                 205
Ser Arg Ala His Ala Ala Lys Ala Gly Leu Arg Thr Thr Phe Xaa Pro
    210                 215                 220
Ser Val Tyr Asp Phe Asp His Met Gln Arg Glu Ala Ala Gly Thr Ala
225                 230                 235                 240
```

```
Thr Lys Ser Ala Leu Ala Gly Glu Val Ile Tyr Gly Asn Asn His Gly
                245                     250             255
Lys Lys Ser Leu Asp Lys Thr Tyr Leu Ala Ala Ala Leu Gly Thr Gly
            260             265             270
Asn Val Thr Ile His Thr Met Glu Arg Ala Arg Gly Ile Arg Arg Leu
        275             280             285
Gly Asp Gly Thr Tyr Val Val Thr Ala Asp Arg Ile Asp Gly Thr Gly
    290             295             300
Ala Val Val Glu Thr Lys Glu Tyr Gly Cys Thr Tyr Leu Phe Leu Gly
305             310             315                 320
Ala Gly Ser Val Gly Thr Thr Glu Leu Leu Val Arg Ala Arg Ala Lys
            325             330             335
Gly Thr Leu Pro Ala Leu Asn Ala Ser Val Gly Ala Gly Trp Gly Pro
            340             345             350
Asn Gly Asn Val Met Leu Gly Arg Ala Asn His Leu Trp Asp Thr Val
            355             360             365
Gly Ala Asn Gln Ser Thr Met Pro Val Met Gly Ile Asp Asp Trp Ala
    370             375             380
Asn Thr Ala Asn Pro Val Xaa Ala Glu Ile Ala Pro Leu Pro Thr Gly
385             390             395                 400
Leu Glu His Trp Val Ser Leu Tyr Leu Ala Ile Thr Lys Asn Thr Glu
            405             410             415
Arg Ala Ser Phe Thr Tyr Asp Ala Ala Thr Asp Ser Ala Lys Leu Gly
            420             425             430
Trp Ser Ala Ala Gln Ser Ala Val Ser Ser Ser Met Ala Lys Lys Leu
            435             440             445
Phe Asp Arg Ile Asn Ser Ala Asn Ser Thr Met Tyr Arg Tyr Asp Leu
    450             455             460
Phe Gly Ser Ser Asn Lys Val Phe Ala Asp Asp Phe Thr Tyr His Pro
465             470             475                 480
Leu Gly Gly Cys Val Leu Gly Arg Ala Thr Asp Asp Tyr Gly Arg Val
            485             490             495
Lys Gly Tyr Glu Asn Leu Tyr Val Thr Asp Gly Ser Leu Val Pro Gly
            500             505             510
Ser Ile Gly Val Asn Pro Phe Val Thr Ile Thr Ala Leu Ala Glu Arg
            515             520             525
Asn Val Ala Arg Val Leu Val Glu Asp Thr Ala Pro
530             535             540
```

<210> 13

<211> 543

<212> PRT

<213> Streptomyces bingchenggensis

<220>

<221> MISC_FEATURE

<222> (158)..(158)

<223> Xaa is Met, Phe, Leu, Val, Cys, Ile, Ala, Gln, Tyr, Lys, or Ser

<220>

<221> MISC_FEATURE

<222> (227)..(227)

<223> Xaa is Val, Met, Ile, Ala, Thr, Lys, Cys, Ser, Gly, Glu, Tyr, Pro, Asn, Gln, Trp or His

<221> MISC_FEATURE

<222> (395)..(395)

<223> Xaa is Phe, Trp, Ser, Thr, Lys, Ala, Asn, His or Asp, provided that when xaa158 is Met, then Xaa395 is not Phe

<400> 13

```
Met Thr Ala Asn Leu Thr Arg Arg His Ile Leu Gly Leu Ala Ala Leu
1                5                10                    15
Arg Gly Ala Ala Ala Leu Gly Leu Thr Arg Ile Ala Leu Ser Pro Ala
        20                    25                    30
Ala Ala Ala Asp Arg Pro Ala Pro Ala Ala Ser Ala Glu Tyr Ala Pro
        35                    40                    45
Ala Val Val Val Gly Ser Gly Tyr Gly Ala Ala Val Ala Ala Leu Arg
    50                    55                    60
Leu Gly Glu Ala Gly Val Lys Thr Leu Val Leu Glu Met Gly Arg Leu
65                    70                    75                    80
Trp Asn Ala Pro Ala Ser Asp Gly Lys Val Tyr Cys Ser Met Thr Ala
                85                    90                    95
```

```
Pro Asp Arg Arg Ser Met Trp Phe Lys Thr Arg Thr Glu Ala Pro Leu
        100                 105                 110
Ala Thr Phe Leu Trp Leu Asp Val Ile Asn Lys Asp Ile Thr Pro Tyr
        115                 120                 125
Pro Gly Val Leu Asp Arg Val Arg Phe Pro Asn Met Ser Val Tyr Val
        130                 135                 140
Gly Arg Gly Val Gly Gly Gly Ser Leu Val Asn Gly Gly Xaa Ala Val
145                 150                 155                 160
Thr Pro Ser Arg Ala Tyr Phe Gln Glu Val Leu Pro Gln Val Asp Ala
                165                 170                 175
Asp Ala Met Tyr Ala Thr Tyr Phe Pro Leu Ala Asn Arg Met Leu Gly
            180                 185                 190
Ala Ala Thr Val Pro Ser Ala Trp Phe Glu Ser Thr Glu Trp Tyr Lys
        195                 200                 205
Tyr Ala Arg Val Ser Arg Asp Gln Ala Lys Thr Ala Gly Leu Lys Thr
210                 215                 220
Val Phe Xaa Pro Asn Val Tyr Asp Phe Asp Tyr Met Gln Arg Glu Ala
225                 230                 235                 240
Ala Gly Thr Ala Thr Lys Ser Ala Leu Ala Gln Glu Val Ile Tyr Gly
                245                 250                 255
Asn Asn Phe Gly Lys Arg Ser Leu Asp Lys Thr Tyr Leu Ala Ala Ala
            260                 265                 270
Leu Gly Thr Gly Asn Val Thr Ile Arg Thr Leu Ser Arg Ala Arg Ala
        275                 280                 285
Ile Arg Arg Ala Ala Asp Gly Thr Tyr Val Leu Thr Val Asp Arg Leu
    290                 295                 300
Asp Asp Thr Gly Ala Val Val Gly Thr Asp Glu Ile Ser Cys Arg Ser
305                 310                 315                 320
Leu Phe Leu Gly Ala Gly Ser Leu Gly Thr Thr Glu Leu Leu Leu Arg
                325                 330                 335
Ala Arg Glu Thr Gly Thr Leu Pro Ala Leu Ser Pro Gln Ile Gly Arg
            340                 345                 350
Gly Trp Gly Gly Asn Gly Asn Val Met Leu Gly Arg Ala Asn His Ile
        355                 360                 365
Trp Asn Thr Thr Gly Ala Asn Gln Ser Thr Ile Pro Val Met Gly Ile
    370                 375                 380
Asp Asp Trp Ser Asn Ala Thr Asn Pro Val Xaa Ala Glu Ile Ala Pro
385                 390                 395                 400
Leu Pro Ala Gly Thr Glu Thr Trp Ala Ser Leu Tyr Leu Ala Ile Thr
                405                 410                 415
Lys Asn Pro Glu Arg Gly Thr Phe Thr Tyr Asp Ala Ala Lys Asp Ala
            420                 425                 430
Ala Val Leu Asn Trp Thr Gly Thr Gln Ser Ala Pro Ser Val Thr Ala
        435                 440                 445
Ala Lys Ser Leu Phe Asp Arg Leu Asn Ser Ala Asn Gly Thr Ile Tyr
    450                 455                 460
Arg Tyr Asp Leu Phe Gly Asp Thr Arg Ala Phe Ala Ala Asp Phe Cys
465                 470                 475                 480
Tyr His Pro Leu Gly Gly Cys Val Leu Gly Arg Ala Thr Asp Ala Tyr
                485                 490                 495
Gly Arg Ala Val Gly Tyr Asp Arg Leu Tyr Val Thr Asp Gly Ser Leu
            500                 505                 510
Ile Pro Gly Ser Ile Gly Val Asn Pro Phe Val Thr Ile Thr Ala Leu
        515                 520                 525
Ala Glu Arg Thr Met Ala Arg Val Leu Ala Glu Asp Gly Val Gly
    530                 535                 540
```

<210> 14

<211> 551

<212> PRT

<213> Streptomyces violaceusniger

<220>

<221> MISC_FEATURE

<222> (164)..(164)

<223> Xaa is Met, Phe, Leu, Val, Cys, Ile, Ala, Gln, Tyr, Lys, or Ser

<220>

<221> MISC_FEATURE

<222> (233)..(233)
<223> Xaa is Val, Met, Ile, Ala, Thr, Lys, Cys, Ser, Gly, Glu, Tyr, Pro, Asn, Gln, Trp or His
<221> MISC_FEATURE
<222> (401)..(401)
<223> Xaa is Phe, Trp, Ser, Thr, Lys, Ala, Asn, His or Asp, provided that when Xaa164 is Ile, then xaa401 is not Phe
<400> 14

```
Met Ala Arg Ser Gly Asp Ser Ala His His Ala Ala Met Thr Ala Asp
1               5                   10                  15
Leu Thr Arg Arg His Ile Leu Gly Leu Ala Ala Leu Gln Ser Ala Ala
            20                  25                  30
Ala Leu Gly Leu Thr Arg Ile Gly Leu Thr Pro Ala Ala Ala Ala Gln
        35                  40                  45
Arg Ala Asp His Phe Pro Ala Val Val Val Gly Ser Gly Tyr Gly Ala
    50                  55                  60
Ala Val Ala Ala Leu Arg Leu Gly Glu Ala Gly Ile Arg Thr Leu Val
65                  70                  75                  80
Val Glu Met Gly Arg Leu Trp Asp Thr Pro Gly Pro Asp Gly Arg Val
                85                  90                  95
His Cys Ser Met Thr Ala Pro Asp Gln Arg Ser Met Trp Phe Lys Thr
            100                 105                 110
Arg Thr Glu Ala Pro Leu Ser Thr Phe Leu Trp Leu Asp Val Ile Asn
        115                 120                 125
Lys Asp Ile Thr Pro Tyr Pro Gly Val Leu Asp Arg Val Arg Phe Pro
    130                 135                 140
Gly Met Ser Val Tyr Val Gly Arg Gly Val Gly Gly Gly Ser Leu Val
145                 150                 155                 160
Asn Gly Gly Xaa Ala Val Thr Pro Ser Arg Ser Tyr Phe Gln Gln Met
            165                 170                 175
Leu Pro Gln Val Ala Ala Asp Pro Met Tyr Asp Thr Tyr Phe Pro Leu
        180                 185                 190
Ala Asn Arg Met Leu Gly Ala Asn Thr Val Pro Ser Ala Trp Phe Glu
        195                 200                 205
Ala Thr Glu Trp Tyr Thr Tyr Ala Arg Val Ala Arg Asp Gln Ala Ala
    210                 215                 220
Arg Ala Gly Leu Lys Thr Val Phe Xaa Pro Asn Val Tyr Asp Phe Asp
225                 230                 235                 240
Tyr Met Arg Arg Glu Ala Asp Gly Thr Ala Thr Lys Ser Ala Leu Ala
                245                 250                 255
Gln Glu Val Ile Tyr Gly Asn Asn Phe Gly Lys Arg Ser Leu Asp Lys
            260                 265                 270
Thr Tyr Leu Ala Ala Ala Leu Gly Thr Gly Gln Val Thr Leu His Thr
        275                 280                 285
Leu Ser Arg Ala Arg Ala Leu Arg Arg Ala Pro Asp Gly Ser Tyr Val
    290                 295                 300
Leu Thr Val Glu Arg Val Asp Thr Thr Gly Thr Val Val Ser Thr Asp
305                 310                 315                 320
Glu Ile Thr Cys Gly Ser Leu Phe Leu Gly Ala Gly Ser Leu Gly Thr
                325                 330                 335
Thr Glu Leu Leu Leu Arg Ala Arg Glu Thr Gly Ala Leu Pro Glu Leu
            340                 345                 350
Ser Glu Glu Val Gly Arg Gly Trp Gly Gly Asn Gly Asn Val Met Leu
        355                 360                 365
Gly Arg Ala Asn His Val Trp His Pro Thr Gly Ala Arg Gln Ser Thr
    370                 375                 380
Ile Pro Val Met Ala Ile Asp Asp Trp Ser Asn Thr Ala Asn Pro Val
385                 390                 395                 400
Xaa Ala Glu Ile Ala Pro Leu Pro Ala Gly Leu Glu Thr Trp Val Ser
                405                 410                 415
Leu Tyr Leu Ala Ile Thr Arg Asn Pro Glu Arg Ala Thr Phe Thr Tyr
        420                 425                 430
Asp Ala Ala Lys Asp Ala Ala Val Leu Asp Trp Thr Arg Ala Gln Ser
        435                 440                 445
Ala Pro Ser Val Ala Ala Ala Lys Ala Leu Phe Asp Arg Val Asn Tyr
    450                 455                 460
Ala Asn Ala Thr Ile Tyr Arg Tyr Asp Leu Phe Gly Asp Thr Arg Ala
465                 470                 475                 480
Ile Ala Asp Asp Phe Cys Tyr His Pro Leu Gly Gly Cys Val Leu Gly
                485                 490                 495
```

49

```
Arg Ala Thr Asp Pro Tyr Gly Arg Val Thr Gly Tyr Asp Gly Leu Tyr
            500                     505                 510
Val Thr Asp Gly Ser Leu Ile Pro Gly Ser Ile Gly Val Asn Pro Phe
            515                     520                 525
Val Thr Ile Thr Ala Leu Ala Glu Arg Thr Met Ala Arg Val Leu Ala
            530                     535                 540
Glu Asp Arg Val Gly Ala Arg
545                     550
```

<210> 15

<211> 533

<212> PRT

<213> Stackebrandtia nassauensis

<220>

<221> MISC_FEATURE

<222> (148)..(148)

<223> Xaa is Met, Phe, Leu, Val, Cys, Ile, Ala, Gln, Tyr, Lys, or Ser

<220>

<221> MISC_FEATURE

<222> (217)..(217)

<223> Xaa is Val, Met, Ile, Ala, Thr, Lys, Cys, Ser, Gly, Glu, Tyr, Pro, Asn, Gln, Trp or His

<221> MISC_FEATURE

<222> (385)..(385)

<223> Xaa is Phe, Trp, Ser, Thr, Lys, Ala, Asn, His or Asp, provided that when xaa148 is Met, then xaa385 is not Phe

<400> 15

```
Met Gly Ile Ser Arg Arg Lys Leu Leu Gly Leu Gly Ala Leu Ala Ala
1               5               10              15
Gly Ala Ser Ala Gly Val Thr Thr Ile Gly Pro Ala Ala Ala Ala Thr
            20              25              30
Ser Gly Asp Phe Val Pro Ala Leu Val Ile Gly Ser Gly Tyr Gly Ala
            35              40              45
Ala Val Ala Ala Leu Arg Leu Gly Glu Ala Gly Val Arg Thr Thr Ile
    50              55              60
Leu Glu Met Gly Arg Leu Trp His Asp Pro Gly His Asp Gly Lys Ile
65              70              75              80
Phe Cys Ala Thr Thr Asn Pro Asp His Arg Ser Met Trp Phe Arg His
            85              90              95
Arg Thr Glu Ala Pro Leu Asp Thr Phe Leu Trp Leu Asp Val Val Asn
        100             105             110
Arg Pro Ile Lys Pro Tyr Pro Gly Val Leu Asp Arg Val His Phe Asp
        115             120             125
Glu Met Ser Val Tyr Val Gly Arg Gly Val Gly Gly Gly Ser Leu Val
    130             135             140
Asn Gly Gly Xaa Ala Val Val Pro Arg Arg Lys Tyr Phe Gln Gln Val
145             150             155             160
Leu Pro Glu Val Asp Ala Asp Asp Met Tyr Arg Lys Phe Phe Pro Leu
            165             170             175
Ala Thr Glu Cys Leu Gly Val Asn Asp Ile Asp Thr Asp Tyr Phe Thr
        180             185             190
Asp Ser Asp Tyr Tyr Glu Phe Ala Arg Val Ala Gly Arg Gln Ala Glu
        195             200             205
Glu Thr Gly Leu Ala Thr Thr Phe Xaa Pro Asn Val Tyr Asp Phe Asp
    210             215             220
His Met Ala Arg Glu Glu Ala Gly Thr Ala Thr Lys Ser Ala Leu Ala
225             230             235             240
Ala Glu Val Ile Tyr Gly Asn Asn His Gly Lys Arg Ser Leu Asp His
            245             250             255
Ser Tyr Leu Ala Ser Ala Leu Gly Thr Gly Asn Val Thr Ile Glu Thr
            260             265             270
Leu His Gln Val Arg Asp Ile Arg Gln Asn Arg Asp Gly Ser Tyr Val
    275             280             285
Val Thr Val Asp His Ile Asp Glu Thr Gly Glu Val Val Glu Ser Lys
    290             295             300
Gln Ile Gly Cys Arg His Leu Phe Leu Gly Ala Gly Ser Leu Gly Thr
305             310             315             320
Thr Glu Leu Leu Leu Arg Ala Arg Asp Thr Gly Ala Leu Pro Asp Leu
            325             330             335
```

```
Asp Ala Asp Val Gly Gln Gly Trp Gly Pro Asn Gly Asn Ile Met Ala
            340                     345             350
Gly Arg Ala Asn Asn Ala Ser Gln Pro Thr Gly Ala Arg Gln Ser Ala
        355                 360                 365
Ile Pro Val Leu Ala Ile Asp Asp Trp Asp Asn Glu Ala Ala Arg Val
        370             375             380
Xaa Ala Glu Ile Ala Pro Val Pro Ala Gly Phe Glu Thr Trp Ile Ser
385                 390             395                 400
Met Tyr Leu Ala Ile Thr Glu Asn Pro Glu Arg Ala Ser Phe Arg Tyr
            405                 410                 415
Asp Pro Ala Thr Asp Arg Ala Val Leu Asp Trp Arg Arg Asp Gln Asn
            420             425                 430
Thr Pro Ser Val Ala Ser Ala Lys Ser Leu Leu Asp Arg Ile Asn Glu
        435                 440             445
Thr Gln Lys Thr Thr Tyr Arg His Asp Leu Phe Gly Asp Asp Arg Ala
        450             455             460
Phe Ala Asp Asp Phe Cys Tyr His Pro Leu Gly Gly Cys Val Leu Gly
465                 470             475                 480
Asn Ala Thr Asp Asn Tyr Gly Arg Leu Lys Gly Tyr Arg Asn Leu Tyr
            485             490                 495
Ala Thr Asp Gly Ala Leu Ile Pro Gly Ser Leu Gly Val Asn Pro Phe
            500             505             510
Val Thr Ile Thr Ala Leu Ala Glu Arg Asn Met Ala Lys Ile Ile Ala
        515             520             525
Thr Asp Ile Thr Thr
        530
```

<210> 16
<211> 545
<212> PRT
<213> Streptomyces clavuligerus
<220>
<221> MISC_FEATURE
<222> (154)..(154)
<223> Xaa is Met, Phe, Leu, Val, Cys, Ile, Ala, Gln, Tyr, Lys, or Ser
<220>
<221> MISC_FEATURE
<222> (223)..(223)
<223> Xaa is Val, Met, Ile, Ala, Thr, Lys, Cys, Ser, Gly, Glu, Tyr, Pro, Asn,
Gln, Trp or His
<221> MISC_FEATURE
<222> (397)..(397)
<223> Xaa is Phe, Trp, ser, Thr, Lys, Ala, Asn, His or Asp, provided that when
xaa154 is Met, then Xaa397 is not Phe
<400> 16

```
Met Thr Pro His Leu Thr Arg Arg Gln Leu Met Gly Ala Ala Ala Leu
1               5                   10                      15
Gln Thr Ala Ala Val Leu Gly Phe Thr Arg Val Gly Leu Ser Ser Ala
            20                  25                      30
His Ala Val Glu Pro Pro Ala Ala Pro His Ala Pro Ala Val Val Ile
        35                  40                  45
Gly Ser Gly Tyr Gly Ala Ala Val Ala Ala Leu Arg Leu Gly Leu Ala
    50                  55                  60
Gly Val Pro Thr Leu Val Leu Glu Met Gly Arg Leu Trp Asp Thr Ala
65              70                  75                      80
Gly Pro Asp Gly Lys Val Phe Cys Pro Thr Ile Thr Pro Asp Arg Arg
            85                  90                  95
Ser Met Trp Phe Arg Thr Arg Thr Glu Ala Pro Leu Ser Thr Phe Leu
            100                 105                 110
Trp Leu Asp Val Val Asn Arg Arg Ile Asp Pro Tyr Pro Gly Val Leu
        115                 120                 125
Asp Arg Val Asn Tyr Gly Asp Met Ser Val Tyr Val Gly Arg Gly Val
    130                 135                 140
Gly Gly Gly Ser Leu Val Asn Gly Gly Xaa Ala Val Thr Pro Pro Arg
145                 150                 155                 160
Pro Tyr Phe Ser Glu Val Leu Pro Gly Val Asp Ala Glu Ala Met Tyr
            165                 170                 175
Gly Thr Tyr Phe Pro Arg Ala Arg Arg Met Leu Gly Val Asn Glu Val
            180                 185                 190
```

```
Asp Arg Ser Trp Phe Glu Ser Thr Pro Trp Tyr Arg Phe Ser Arg Val
        195                 200                 205
Ser Arg Thr His Ala Ala Arg Ala Gly Leu Gly Thr Val Phe Xaa Pro
    210                 215                 220
Asn Val Tyr Asp Phe Asp Tyr Met Arg Arg Glu Ala Ala Gly Glu Val
225                 230                 235                     240
Pro Arg Ser Ala Leu Ala Gly Glu Val Ile Tyr Gly Asn Asn His Gly
            245                 250                 255
Lys Arg Ser Leu Asp Arg Thr Tyr Leu Ala Ala Ala Leu Ala Thr Gly
            260                 265                 270
Arg Val Thr Val Glu Thr Met Ser Arg Val Arg Ala Leu Arg Pro Ala
        275                 280                 285
Asn Thr Gly Gly Thr Gly Gly Ala Gly Gly Tyr Val Leu Thr Val Glu
        290                 295                 300
Arg Leu Asp Leu Ser Gly Arg Val Thr Ala Val Asp Glu Ile Thr Thr
305                 310                 315                     320
Gly Arg Leu Phe Leu Gly Ala Gly Ser Leu Gly Thr Thr Glu Leu Leu
            325                 330                 335
Leu Arg Ala Arg Glu Thr Gly Ala Leu Pro Asp Leu Asp Pro Glu Val
            340                 345                 350
Gly Arg Gly Trp Gly His Asn Gly Asn Val Met Thr Ala Arg Ala Asn
        355                 360                 365
His Leu Trp Asp Thr Val Gly Ala Gln Gln Ser Thr Met Pro Val Leu
        370                 375                 380
Gly Ile Asp Asp Trp Asn Asn Pro Thr His Pro Val Xaa Ala Glu Ile
385                 390                 395                     400
Ala Pro Leu Pro Met Gly Leu Glu His Trp Ile Ser Leu Tyr Leu Ala
            405                 410                 415
Ile Thr Lys Asn Pro Glu Arg Gly His Phe Thr Tyr Asp Ala Ala Thr
            420                 425                 430
Asp Ser Ala Arg Leu Arg Trp Thr Arg Asp Gln Asn Glu Pro Ser Val
        435                 440                 445
Ala Ala Ala Arg Ser Leu Phe Asp Arg Ile Asn Arg Ala Asn Gly Thr
    450                 455                 460
Ile His Arg Tyr Asp Leu Phe Gly Gly Asn Arg Lys Phe Ala Asp Asp
465                 470                 475                     480
Phe Thr Tyr His Pro Leu Gly Gly Cys Val Leu Gly Arg Ala Thr Asp
            485                 490                 495
Gly Tyr Gly Arg Ala Lys Gly His Pro Gly Leu Tyr Val Val Asp Gly
            500                 505                 510
Ser Leu Val Pro Gly Ser Ile Gly Val Asn Pro Phe Val Thr Ile Thr
            515                 520                 525
Ala Leu Ala Glu Arg Asn Met Glu Arg Ile Val Gln Glu Asp Ile Leu
        530                 535                 540
Gly
545
```

<210> 17
<211> 559
<212> PRT
<213> Streptomyces clavuligerus
<220>
<221> MISC_FEATURE
<222> (168)..(168)
<223> Xaa is Met, Phe, Leu, Val, Cys, Ile, Ala, Gln, Tyr, Lys, or Ser
<220>
<221> MISC_FEATURE
<222> (237)..(237)
<223> Xaa is Val, Met, Ile, Ala, Thr, Lys, Cys, Ser, Gly, Glu, Tyr, Pro, Asn,
Gln, Trp or His
<221> MISC_FEATURE
<222> (411)..(411)
<223> Xaa is Phe, Trp, Ser, Thr, Lys, Ala, Asn, His or Asp, provided that when
xaa168 is Met, then Xaa411 is not Phe
<400> 17

```
Met Ala Leu Gly Ile Arg Pro Val Leu Val Asp Asp Gln His Met Thr
1               5                   10                  15
Pro His Leu Thr Arg Arg Gln Leu Met Gly Ala Ala Ala Leu Gln Thr
            20                  25                  30
```

```
Ala Ala Val Leu Gly Phe Thr Arg Val Gly Leu Ser Ser Ala His Ala
        35                  40                  45
Val Glu Pro Pro Ala Ala Pro His Ala Pro Ala Val Val Ile Gly Ser
    50                  55                  60
Gly Tyr Gly Ala Ala Val Ala Ala Leu Arg Leu Gly Leu Ala Gly Val
65                  70                  75                      80
Pro Thr Leu Val Leu Glu Met Gly Arg Leu Trp Asp Thr Ala Gly Pro
                85                  90                  95
Asp Gly Lys Val Phe Cys Pro Thr Ile Thr Pro Asp Arg Arg Ser Met
            100                 105                 110
Trp Phe Arg Thr Arg Thr Glu Ala Pro Leu Ser Thr Phe Leu Trp Leu
        115                 120                 125
Asp Val Val Asn Arg Arg Ile Asp Pro Tyr Pro Gly Val Leu Asp Arg
    130                 135                 140
Val Asn Tyr Gly Asp Met Ser Val Tyr Val Gly Arg Gly Val Gly Gly
145                 150                 155                     160
Gly Ser Leu Val Asn Gly Gly Xaa Ala Val Thr Pro Pro Arg Pro Tyr
                165                 170                 175
Phe Ser Glu Val Leu Pro Gly Val Asp Ala Glu Ala Met Tyr Gly Thr
            180                 185                 190
Tyr Phe Pro Arg Ala Arg Arg Met Leu Gly Val Asn Glu Val Asp Arg
        195                 200                 205
Ser Trp Phe Glu Ser Thr Pro Trp Tyr Arg Phe Ser Arg Val Ser Arg
    210                 215                 220
Thr His Ala Ala Arg Ala Gly Leu Gly Thr Val Phe Xaa Pro Asn Val
225                 230                 235                     240
Tyr Asp Phe Asp Tyr Met Arg Arg Glu Ala Ala Gly Glu Val Pro Arg
                245                 250                 255
Ser Ala Leu Ala Gly Glu Val Ile Tyr Gly Asn Asn His Gly Lys Arg
            260                 265                 270
Ser Leu Asp Arg Thr Tyr Leu Ala Ala Ala Leu Ala Thr Gly Arg Val
        275                 280                 285
Thr Val Glu Thr Met Ser Arg Val Arg Ala Leu Arg Pro Ala Asn Thr
    290                 295                 300
Gly Gly Thr Gly Gly Ala Gly Gly Tyr Val Leu Thr Val Glu Arg Leu
305                 310                 315                     320
Asp Leu Ser Gly Arg Val Thr Ala Val Asp Glu Ile Thr Thr Gly Arg
                325                 330                 335
Leu Phe Leu Gly Ala Gly Ser Leu Gly Thr Thr Glu Leu Leu Leu Arg
            340                 345                 350
Ala Arg Glu Thr Gly Ala Leu Pro Asp Leu Asp Pro Glu Val Gly Arg
        355                 360                 365
Gly Trp Gly His Asn Gly Asn Val Met Thr Ala Arg Ala Asn His Leu
    370                 375                 380
Trp Asp Thr Val Gly Ala Gln Gln Ser Thr Met Pro Val Leu Gly Ile
385                 390                 395                     400
Asp Asp Trp Asn Asn Pro Thr His Pro Val Xaa Ala Glu Ile Ala Pro
                405                 410                 415
Leu Pro Met Gly Leu Glu His Trp Ile Ser Leu Tyr Leu Ala Ile Thr
            420                 425                 430
Lys Asn Pro Glu Arg Gly His Phe Thr Tyr Asp Ala Ala Thr Asp Ser
        435                 440                 445
Ala Arg Leu Arg Trp Thr Arg Asp Gln Asn Glu Pro Ser Val Ala Ala
    450                 455                 460
Ala Arg Ser Leu Phe Asp Arg Ile Asn Arg Ala Asn Gly Thr Ile His
465                 470                 475                     480
Arg Tyr Asp Leu Phe Gly Gly Asn Arg Lys Phe Ala Asp Asp Phe Thr
                485                 490                 495
Tyr His Pro Leu Gly Gly Cys Val Leu Gly Arg Ala Thr Asp Gly Tyr
            500                 505                 510
Gly Arg Ala Lys Gly His Pro Gly Leu Tyr Val Val Asp Gly Ser Leu
        515                 520                 525
Val Pro Gly Ser Ile Gly Val Asn Pro Phe Val Thr Ile Thr Ala Leu
    530                 535                 540
Ala Glu Arg Asn Met Glu Arg Ile Val Gln Glu Asp Ile Leu Gly
545                 550                 555
```

<210> 18
<211> 534

```
<212> PRT
<213> Saccharopolyspora erythraea
<220>
<221> MISC_FEATURE
<222> (151)..(151)
<223> Xaa is Met, Phe, Leu, Val, Cys, Ile, Ala, Gln, Tyr, Lys, or Ser
<220>
<221> MISC_FEATURE
<222> (220)..(220)
<223> Xaa is Val, Met, Ile, Ala, Thr, Lys, Cys, Ser, Gly, Glu, Tyr, Pro, Asn,
Gln, Trp or His
<221> MISC_FEATURE
<222> (388)..(388)
<223> Xaa is Phe, Trp, Ser, Thr, Lys, Ala, Asn, His or Asp, provided that when
xaa151 is Met, then Xaa388 is not Phe
<400> 18
```

```
Met Thr Ser Ser Val Thr Arg Arg Arg Phe Leu Gly Met Ala Ala Met
1               5                   10                  15
Gln Ser Ala Ala Val Leu Gly Leu Gly Ala Val Ser Leu Gly Arg Ala
            20                  25                  30
Asp Ala Ala Glu Arg Pro Phe Val Pro Ala Val Val Gly Ser Gly
        35                  40                  45
Tyr Gly Ser Ala Ala Thr Ala Leu Arg Leu Gly Glu Ala Gly Val Ser
    50                  55                  60
Thr Leu Val Leu Glu Met Gly Arg Leu Trp Asp Arg Ala Gly Glu Asp
65                  70                  75                  80
Gly Ala Ile Phe Cys Ser Met Leu Gln Pro Asp His Arg Ala Met Trp
                85                  90                  95
Phe Lys Ala Arg Thr Glu Ala Pro Leu Ser Ser Leu Leu Trp Met Asp
            100                 105                 110
Leu Val Asn Arg Asp Ile Lys Pro Phe Ala Gly Val Leu Asp Arg Val
        115                 120                 125
His His Gly Asp Met Ser Val Tyr Val Gly Arg Gly Val Gly Gly Gly
    130                 135                 140
Ser Leu Val Asn Gly Gly Xaa Ala Val Thr Pro Arg Arg Gly Tyr Phe
145                 150                 155                 160
Glu Glu Val Leu Pro Arg Val Asp Ala Gly Glu Met Tyr Gly Arg Phe
                165                 170                 175
Phe Pro Leu Ala Asn Arg Met Leu Gly Val Asn Thr Val Asp Arg Thr
            180                 185                 190
Trp Phe Glu Glu Cys Glu Ser Tyr Arg Tyr Ala Arg Val Ser Arg Gly
        195                 200                 205
His Ala Gln Arg Ala Gly Leu Arg Thr Thr Phe Xaa Pro Asn Val Tyr
    210                 215                 220
Asp Phe Gly Tyr Met Arg Arg Glu Glu Arg Gly Glu Val Pro Lys Ser
225                 230                 235                 240
Ala Leu Ser Ser Glu Val Ile Tyr Gly Asn Asn His Gly Lys Arg Ser
                245                 250                 255
Leu Asp Arg Ser Tyr Leu Pro Ala Ala Val Gly Thr Gly Asn Val Thr
            260                 265                 270
Ile Gln Ser Leu His Arg Val Arg Ser Phe Arg Gln Glu Pro Asp Gly
        275                 280                 285
Thr Tyr Val Leu Thr Val Glu Arg Ile Asp Glu Leu Gly Asn Met Leu
    290                 295                 300
Gly Thr Thr Glu Ile Gly Cys Arg Trp Leu Phe Leu Gly Ala Gly Ser
305                 310                 315                 320
Met Gly Thr Thr Glu Leu Leu Leu Arg Ala Arg Glu Thr Gly Val Leu
                325                 330                 335
Pro Arg Leu Asp Asp Ser Val Gly His Gly Trp Gly Thr Asn Gly Asn
            340                 345                 350
Val Met Leu Gly Arg Ala Leu His Ser Trp Asp Arg Thr Gly Ser Val
        355                 360                 365
Gln Ser Gly Met Pro Ala Leu Gly Ile Asp Asn Trp Asp Asp Pro Val
    370                 375                 380
His Pro Val Xaa Ala Glu Ile Ala Pro Leu Pro Ala Gly Leu Glu Leu
385                 390                 395                 400
Leu Thr Ser Leu Ser Leu Ala Ile Thr Arg Asn Pro Glu Arg Gly Ser
                405                 410                 415
```

```
Phe Ser Tyr Asp Pro Ala Ala Asp Ala Ala Arg Leu His Trp Ser Ala
            420             425                 430
Ser Gln Gly Lys Pro Ser Val Glu Ala Ala Lys Ala Leu Phe Asp Pro
            435             440                 445
Ile Asn Arg Ala Asn Gly Thr Val Tyr Arg His Asp Leu Phe Gly Asp
450             455                 460
Ser Arg Ala Phe Glu Asp Arg Phe Thr Tyr His Pro Leu Gly Gly Cys
465             470             475                     480
Val Leu Gly Glu Ala Thr Asp Asp Phe Gly Arg Val Arg Gly Tyr Arg
                485             490                 495
Asn Leu Tyr Val Thr Asp Gly Ser Leu Ile Pro Gly Ser Thr Gly Val
            500             505                 510
Asn Pro Phe Val Thr Ile Thr Ala Leu Ala Glu Arg Asn Ile Asp Arg
            515             520                 525
Val Leu Ser Glu Asp Phe
            530
```

<210> 19
<211> 544
<212> PRT
<213> Streptomyces avermitilis

<220>
<221> MISC_FEATURE
<222> (159)..(159)
<223> Xaa is Met, Phe, Leu, Val, Cys, Ile, Ala, Gln, Tyr, Lys, or Ser
<220>
<221> MISC_FEATURE
<222> (228)..(228)
<223> Xaa is Val, Met, Ile, Ala, Thr, Lys, Cys, Ser, Gly, Glu, Tyr, Pro, Asn, Gln, Trp or His
<221> MISC_FEATURE
<222> (396)..(396)
<223> Xaa is Phe, Trp, Ser, Thr, Lys, Ala, Asn, His or Asp, provided that when xaa159 is Met, then Xaa396 is not Phe
<400> 19

```
Met Thr Glu Lys Leu Thr His Arg His Leu Thr Arg Arg Gln Ile Leu
1               5                   10              15
Gly Met Ala Ala Leu Gln Thr Ala Ala Thr Leu Gly Phe Thr Arg Ile
        20                  25                  30
Gly Leu Gln Ser Ala Arg Ala Ala Glu Pro Asp Ala Val Glu Thr Ala
        35                  40                  45
Pro Ala Ile Val Val Gly Ser Gly Tyr Gly Ala Ala Val Ala Ala Leu
    50                  55                  60
Arg Leu Gly Gln Ala Gly Leu Arg Thr Leu Val Ile Glu Met Gly Gly
65                  70                  75                  80
Leu Trp Asn Thr Pro Gly Ser Asp Gly Lys Val Phe Cys Ser Thr Ser
                85                  90                  95
Ala Pro Asp Arg Arg Ser Met Trp Phe Arg Thr Arg Thr Glu Ala Pro
            100                 105                 110
Leu Ala Glu Phe Leu Trp Leu Asp Val Val Asn Lys Asp Ile Ser Pro
            115                 120                 125
Tyr Pro Gly Val Leu Asp Arg Val His Phe Ala Asn Met Ser Val Tyr
    130                 135                 140
Val Gly Arg Gly Val Gly Gly Gly Ser Leu Val Asn Gly Gly Xaa Ala
145                 150                 155                 160
Val Thr Pro Leu Gln Ser Tyr Phe Ala Glu Gln Phe Pro Thr Val Asp
            165                 170                 175
Ala Ala Glu Met Tyr Ser Thr Tyr Phe Pro Arg Ala Arg Thr Met Leu
            180                 185                 190
Gly Val Asn Thr Val Asp Pro Ala Trp Phe Glu Ser Thr Glu Trp Tyr
            195                 200                 205
Arg Phe Thr Arg Thr Ser Arg Lys Ala Ala Thr Asn Thr Gly Leu Lys
    210                 215                 220
Thr Thr Phe Xaa Pro Asn Val Tyr Asp Phe Gly Tyr Met Gln Arg Glu
225                 230                 235                 240
Ala Ala Gly Thr Ala Thr Lys Ser Ala Leu Ala Gly Glu Val Ile Tyr
            245                 250                 255
Gly Asn Asn Tyr Gly Lys Arg Ser Leu Asp Lys Thr Tyr Leu Ala Ser
            260                 265                 270
```

```
Ala Leu Gly Thr Gly Asn Val Thr Ile His Thr Leu Glu Arg Val Arg
        275             280             285
Glu Ile Arg Arg Ala Ser Asp Gly Thr Tyr Leu Leu Thr Ala Asp Arg
        290             295             300
Ile Asp Thr Thr Gly Ala Val Val Glu Thr Lys Gln Tyr Ser Cys Thr
305             310             315             320
Tyr Leu Phe Leu Gly Gly Gly Ser Leu Gly Thr Ser Glu Leu Leu Val
            325             330             335
Arg Ala Arg Glu Thr Gly Ala Leu Pro Ala Leu Asp Ala Ser Val Gly
        340             345             350
Thr Gly Trp Gly Thr Asn Gly Asn Val Met Thr Gly Arg Ala Asn His
        355             360             365
Ile Trp Asp Thr Val Gly Ala Asn Gln Ser Thr Met Pro Val Met Gly
        370             375             380
Ile Asp Asp Trp Ala Asn Thr Ser Asn Pro Val Xaa Ala Glu Ile Ala
385             390             395             400
Pro Leu Pro Met Gly Leu Glu His Trp Ile Ser Leu Tyr Leu Ala Ile
            405             410             415
Thr Lys Asn Pro Glu Arg Ala Ser Phe Thr Tyr Asp Ala Ala Ser Asp
        420             425             430
Ser Ala Lys Leu Gly Trp Ser Ala Ala Gln Ser Ala Val Ser Val Ser
        435             440             445
Met Ala Lys Lys Leu Phe Asp Arg Ile Asn Ser Ala Asn Ala Thr Ile
    450             455             460
Tyr Arg Tyr Asp Leu Phe Gly Asn Asn Lys Thr Phe Ala Asp Asp Phe
465             470             475             480
Thr Tyr His Pro Leu Gly Gly Cys Val Leu Gly Lys Ser Thr Asp Asn
            485             490             495
Tyr Gly Arg Val Lys Gly Tyr Ser Lys Leu Tyr Val Thr Asp Gly Ser
        500             505             510
Leu Val Pro Gly Ser Ile Gly Val Asn Pro Phe Val Thr Ile Thr Ala
        515             520             525
Leu Ala Glu Arg Thr Met Ala Arg Val Leu Ala Glu Asp Thr Ala Pro
        530             535             540
```

<210> 20
<211> 522
<212> PRT
<213> Saccharopolyspora erythraea
<220>
<221> MISC_FEATURE
<222> (139)..(139)
<223> Xaa is Met, Phe, Leu, Val, Cys, Ile, Ala, Gln, Tyr, Lys, or Ser
<220>
<221> MISC_FEATURE
<222> (208)..(208)
<223> Xaa is Val, Met, Ile, Ala, Thr, Lys, Cys, Ser, Gly, Glu, Tyr, Pro, Asn, Gln, Trp or His
<221> MISC_FEATURE
<222> (376)..(376)
<223> Xaa is Phe, Trp, Ser, Thr, Lys, Ala, Asn, His or Asp, provided that when Xaa139 is Met, then Xaa376 is not Phe
<400> 20

```
Met Ala Ala Met Gln Ser Ala Ala Val Leu Gly Leu Gly Ala Val Ser
1               5                   10                  15
Leu Gly Arg Ala Asp Ala Ala Glu Arg Pro Phe Val Pro Ala Val Val
            20                  25                  30
Val Gly Ser Gly Tyr Gly Ser Ala Ala Thr Ala Leu Arg Leu Gly Glu
            35                  40                  45
Ala Gly Val Ser Thr Leu Val Leu Glu Met Gly Arg Leu Trp Asp Arg
50                  55                  60
Ala Gly Glu Asp Gly Ala Ile Phe Cys Ser Met Leu Gln Pro Asp His
65                  70                  75                  80
Arg Ala Met Trp Phe Lys Ala Arg Thr Glu Ala Pro Leu Ser Ser Leu
                85                  90                  95
Leu Trp Met Asp Leu Val Asn Arg Asp Ile Lys Pro Phe Ala Gly Val
            100                 105                 110
Leu Asp Arg Val His His Gly Asp Met Ser Val Tyr Val Gly Arg Gly
            115                 120                 125

Val Gly Gly Gly Ser Leu Val Asn Gly Gly Xaa Ala Val Thr Pro Arg
    130                 135                 140
Arg Gly Tyr Phe Glu Glu Val Leu Pro Arg Val Asp Ala Gly Glu Met
145                 150                 155                 160
Tyr Gly Arg Phe Phe Pro Leu Ala Asn Arg Met Leu Gly Val Asn Thr
                165                 170                 175
Val Asp Arg Thr Trp Phe Glu Glu Cys Glu Ser Tyr Arg Tyr Ala Arg
            180                 185                 190
Val Ser Arg Gly His Ala Gln Arg Ala Gly Leu Arg Thr Thr Phe Xaa
        195                 200                 205
Pro Asn Val Tyr Asp Phe Gly Tyr Met Arg Arg Glu Glu Arg Gly Glu
    210                 215                 220
Val Pro Lys Ser Ala Leu Ser Ser Glu Val Ile Tyr Gly Asn Asn His
225                 230                 235                 240
Gly Lys Arg Ser Leu Asp Arg Ser Tyr Leu Pro Ala Ala Val Gly Thr
                245                 250                 255
Gly Asn Val Thr Ile Gln Ser Leu His Arg Val Arg Ser Phe Arg Gln
            260                 265                 270
Glu Pro Asp Gly Thr Tyr Val Leu Thr Val Glu Arg Ile Asp Glu Leu
        275                 280                 285
Gly Asn Met Leu Gly Thr Thr Glu Ile Gly Cys Arg Trp Leu Phe Leu
    290                 295                 300
Gly Ala Gly Ser Met Gly Thr Thr Glu Leu Leu Leu Arg Ala Arg Glu
305                 310                 315                 320
Thr Gly Val Leu Pro Arg Leu Asp Asp Ser Val Gly His Gly Trp Gly
                325                 330                 335
Thr Asn Gly Asn Val Met Leu Gly Arg Ala Leu His Ser Trp Asp Arg
            340                 345                 350
Thr Gly Ser Val Gln Ser Gly Met Pro Ala Leu Gly Ile Asp Asn Trp
        355                 360                 365
Asp Asp Pro Val His Pro Val Xaa Ala Glu Ile Ala Pro Leu Pro Ala
    370                 375                 380
Gly Leu Glu Leu Leu Thr Ser Leu Ser Leu Ala Ile Thr Arg Asn Pro
385                 390                 395                 400
Glu Arg Gly Ser Phe Ser Tyr Asp Pro Ala Ala Asp Ala Ala Arg Leu
                405                 410                 415
His Trp Ser Ala Ser Gln Gly Lys Pro Ser Val Glu Ala Ala Lys Ala
            420                 425                 430
Leu Phe Asp Pro Ile Asn Arg Ala Asn Gly Thr Val Tyr Arg His Asp
            435                 440                 445
Leu Phe Gly Asp Ser Arg Ala Phe Glu Asp Arg Phe Thr Tyr His Pro
    450                 455                 460
Leu Gly Gly Cys Val Leu Gly Glu Ala Thr Asp Asp Phe Gly Arg Val
465                 470                 475                 480
Arg Gly Tyr Arg Asn Leu Tyr Val Thr Asp Gly Ser Leu Ile Pro Gly
                485                 490                 495
Ser Thr Gly Val Asn Pro Phe Val Thr Ile Thr Ala Leu Ala Glu Arg
            500                 505                 510
Asn Ile Asp Arg Val Leu Ser Glu Asp Phe
            515                 520
```

62

<210> 21
<211> 547
<212> PRT
<213> Streptomyces hygroscopicus
<220>
<221> MISC_FEATURE
<222> (161)..(161)
<223> Xaa is Met, Phe, Leu, Val, Cys, Ile, Ala, Gln, Tyr, Lys, or Ser
<220>
<221> MISC_FEATURE
<222> (230)..(230)
<223> Xaa is Val, Met, Ile, Ala, Thr, Lys, Cys, Ser, Gly, Glu, Tyr, Pro, Asn, Gln, Trp or His
<221> MISC_FEATURE
<222> (398)..(398)
<223> Xaa is Phe, Trp, Ser, Thr, Lys, Ala, Asn, His or Asp, provided that when xaa161 is Ile, then Xaa398 is not Phe
<400> 21

```
Met Ser His Ser Ala His Asp Ala Ala Met Glu Gly Lys Leu Thr Arg
1               5                   10                  15
Arg His Phe Leu Gly Leu Ala Ala Leu Gln Thr Ala Ala Ala Leu Gly
            20                  25                  30
Leu Thr Arg Ile Gly Leu Thr Pro Ala Ala Ala Ala Gln Ser Ala Asp
        35                  40                  45
His Thr Pro Ala Leu Val Ile Gly Ser Gly Tyr Gly Ala Ala Val Ala
    50                  55                  60
Ala Leu Arg Leu Gly Glu Ala Gly Ile Arg Thr Leu Val Ile Glu Met
65                  70                  75                  80
Gly Arg Leu Trp Glu Ala Pro Asp Ser Gly Gly Thr Val Phe Cys Ser
                85                  90                  95
Met Thr Ala Pro Asp Gln Arg Ser Met Trp Phe Lys Arg Arg Thr Glu
            100                 105                 110
Ala Pro Leu Ser Thr Phe Leu Trp Leu Asp Val Ile Asn Lys Asp Ile
        115                 120                 125
Thr Pro Tyr Pro Gly Val Leu Asp Arg Val Arg His Pro Asn Met Ser
    130                 135                 140
Val Tyr Val Gly Arg Gly Val Gly Gly Gly Ser Leu Val Asn Gly Gly
145                 150                 155                 160
Xaa Ala Val Thr Pro Pro Arg Ala Tyr Phe Gln Glu Val Leu Pro Gly
                165                 170                 175
Val Asp Ala Asp Ala Met Tyr Gly Thr Tyr Phe Pro Arg Ala Asn Arg
            180                 185                 190
Met Leu Gly Thr Ala Thr Ile Pro Ser Asp Trp Phe Glu Gly Thr Glu
        195                 200                 205
Trp Tyr Gln Tyr Ala Arg Val Ala Arg Ala Gln Ala Ser Ala Ala Gly
    210                 215                 220
Leu Lys Thr Val Phe Xaa Pro Ser Val Tyr Asp Phe Asp Tyr Met Arg
225                 230                 235                 240
Arg Glu Ala Ala Gly Thr Ala Thr Lys Ser Ala Leu Ala Gln Glu Val
                245                 250                 255
Ile Tyr Gly Asn Asn His Gly Lys Arg Ser Leu Asp Lys Thr Tyr Leu
            260                 265                 270
Ala Ala Ala Leu Gly Thr Gly Asn Val Thr Ile His Thr Leu Ser Arg
        275                 280                 285
Ala Arg Ala Ile Arg Arg Ala Ala Asp Gly Ser Tyr Thr Val Thr Val
    290                 295                 300
Asp Arg Ile Asp Thr Thr Gly Ala Val Thr Ala Thr Asp Glu Ile Ser
305                 310                 315                 320
Cys Arg Ala Leu Phe Leu Gly Ala Gly Ser Leu Gly Thr Thr Glu Leu
                325                 330                 335
Leu Leu Arg Ala Arg Glu Thr Gly Thr Leu Pro Gly Leu Asn Ala Glu
            340                 345                 350
Val Gly Arg Gly Trp Gly Gly Asn Gly Asn Val Met Leu Gly Arg Ala
        355                 360                 365
Asn His Val Trp Asn Pro Thr Gly Ala His Gln Ser Thr Ile Pro Val
    370                 375                 380
Thr Ala Ile Asp Asp Trp Ser Asn Ala Ala Asn Pro Val Xaa Ala Glu
385                 390                 395                 400
Ile Ala Pro Leu Pro Ala Gly Thr Glu Thr Trp Ala Ser Leu Tyr Leu
                405                 410                 415
Ala Ile Thr Lys Asn Pro Glu Arg Gly Thr Phe Thr Tyr Asp Ala Ala
            420                 425                 430
Lys Asp Ala Ala Val Leu Asn Trp Thr Ala Gly Gln Ser Ala Pro Ala
        435                 440                 445
Ile Ala Ala Ala Lys Ala Leu Phe Asp Arg Val Asn Ala Ala Asn Val
    450                 455                 460
Thr Ile Tyr Arg Tyr Asp Leu Phe Gly Asp Thr Arg Ala Phe Ala Ala
465                 470                 475                 480
Asp Phe Cys Tyr His Pro Leu Gly Gly Cys Val Leu Gly Arg Ala Thr
                485                 490                 495
Asp Ala Tyr Gly Arg Val Ala Gly Tyr Pro Arg Leu Tyr Val Thr Asp
            500                 505                 510
Gly Ser Leu Ile Pro Gly Ser Ile Gly Val Asn Pro Phe Val Thr Ile
        515                 520                 525
Thr Ala Leu Ala Glu Arg Thr Met Ala Arg Val Leu Ala Glu Asp Asp
    530                 535                 540
```

64

Val Gly Arg 545

<210> 22
<211> 545
<212> PRT
<213> Streptomyces viridochromogenes
<220>
<221> MISC_FEATURE
<222> (159)..(159)
<223> Xaa is Met, Phe, Leu, Val, Cys, Ile, Ala, Gln, Tyr, Lys, or Ser
<220>
<221> MISC_FEATURE
<222> (228)..(228)
<223> Xaa is Val, Met, Ile, Ala, Thr, Lys, Cys, Ser, Gly, Glu, Tyr, Pro, Asn, Gln, Trp or His
<221> MISC_FEATURE
<222> (396)..(396)
<223> Xaa is Phe, Trp, Ser, Thr, Lys, Ala, Asn, His or Asp, provided that when xaa159 is Met, then Xaa396 is not Phe
<400> 22

```
Met Thr Val Lys Leu Met Gln Arg Gln Leu Thr Arg Arg Gln Ile Leu
1               5                   10                  15
Gly Met Val Ala Leu Gln Gly Ala Ala Ala Ala Gly Leu Thr Arg Ile
            20                  25                  30
Gly Leu Gln Val Ala Ser Ala Ala Glu Pro Ala Ala Val Asp Asn Ala
        35                  40                  45
Pro Ala Ile Val Val Gly Ser Gly Tyr Gly Gly Ala Val Ala Ala Leu
    50                  55                  60
Arg Leu Gly Gln Ala Gly Ile Arg Thr Leu Val Leu Glu Met Gly Arg
65                  70                  75                  80
Leu Trp Asn Thr Pro Gly Pro Asp Gly Lys Val Phe Cys Ser Thr Arg
                85                  90                  95
Thr Pro Asp Gln Arg Ser Met Trp Phe Arg Thr Arg Thr Glu Ala Pro
            100                 105                 110
Leu Ala Thr Phe Leu Trp Leu Asp Leu Val Asn Gln Asp Ile Ser Ser
        115                 120                 125
Tyr Pro Gly Val Leu Asp Arg Val His Tyr Asp His Met Ser Val Tyr
    130                 135                 140
Val Gly Arg Gly Val Gly Gly Gly Ser Leu Val Asn Gly Gly Xaa Ala
145                 150                 155                 160
Val Thr Pro Leu Arg Ser Tyr Phe Ala Glu Gln Phe Pro Thr Val Asp
                165                 170                 175
Thr Ala Glu Met Tyr Asp Thr Tyr Phe Pro Arg Ala Arg Ser Met Leu
            180                 185                 190
Gly Val Asn Thr Val Asp Pro Ala Trp Phe Glu Ser Thr Glu Trp Tyr
        195                 200                 205
Arg Phe Thr Arg Ile Ser Arg Lys His Ala Asp Asn Ala Gly Leu Lys
    210                 215                 220
Thr Thr Phe Xaa Pro Asn Val Tyr Asp Phe Gly His Met Glu Arg Glu
225                 230                 235                 240
Ala Ala Gly Thr Ala Thr Lys Ser Ala Leu Ala Gly Glu Val Ile Tyr
            245                 250                 255
Gly Asn Asn Gln Gly Lys Arg Ser Leu Asp Lys Thr Tyr Leu Ala Ser
        260                 265                 270
Ala Leu Gly Thr Gly Asn Val Thr Leu His Thr Met Glu Arg Val Thr
    275                 280                 285
Ser Ile Ser Arg Ala Ala Asp Gly Thr Tyr Leu Leu Thr Ala Asp Arg
290                 295                 300
Ile Asp Asp Thr Gly Thr Val Val Glu Thr Lys Glu Tyr Ala Cys Thr
305                 310                 315                 320
Tyr Leu Phe Leu Gly Gly Gly Ser Ile Gly Thr Thr Glu Leu Leu Val
            325                 330                 335
Arg Ala Arg Glu Ser Gly Thr Leu Pro Arg Leu Asp Ala Ser Val Gly
            340                 345                 350
Thr Gly Trp Gly Thr Asn Gly Asn Val Met Leu Gly Arg Ala Asn His
        355                 360                 365
Val Trp Asp Thr Val Gly Ala Asn Gln Ser Thr Met Pro Val Met Gly
    370                 375                 380
```

```
Ile Asp Asp Trp Ala Asn Thr Ala Asn Pro Val Xaa Ala Glu Ile Ala
385                 390                 395                 400
Pro Leu Pro Met Gly Leu Glu His Trp Val Ser Leu Tyr Leu Ala Ile
            405                 410                 415
Thr Lys Asn Pro Glu Arg Ala Ser Phe Thr Tyr Asp Pro Ala Ser Gly
        420                 425                 430
Ala Val Arg Leu Gly Trp Ser Ala Ala Gln Ser Ala Val Ser Val Gly
        435                 440                 445
Met Ala Lys Lys Leu Phe Asp Arg Ile Asn Lys Ala Asn Ala Thr Ile
    450                 455                 460
Tyr Arg Tyr Asp Leu Phe Gly Ser Ser Asn Lys Val Phe Ala Asp Asp
465                 470                 475                 480
Phe Thr Tyr His Pro Leu Gly Gly Cys Val Leu Gly Arg Ser Thr Asp
            485                 490                 495
Ala Tyr Gly Arg Val Lys Gly Tyr Ser Arg Leu Tyr Val Thr Asp Gly
        500                 505                 510
Ser Leu Val Pro Gly Ser Ile Gly Val Asn Pro Phe Val Thr Ile Thr
        515                 520                 525
Ala Leu Ala Glu Arg Thr Met Ala Arg Val Leu Ala Glu Asp Thr Ala
    530                 535                 540
Pro
545
```

<210> 23
<211> 531
<212> PRT
<213> Microscilla marina
<220>
<221> MISC_FEATURE
<222> (146)..(146)
<223> Xaa is Met, Phe, Leu, Val, Cys, Ile, Ala, Gln, Tyr, Lys, or Ser
<220>
<221> MISC_FEATURE
<222> (215)..(215)
<223> Xaa is Val, Met, Ile, Ala, Thr, Lys, Cys, Ser, Gly, Glu, Tyr, Pro, Asn,
Gln, Trp or His
<221> MISC_FEATURE
<222> (383)..(383)
<223> Xaa is Phe, Trp, Ser, Thr, Lys, Ala, Asn, His or Asp, provided that when
xaa146 is Met, then Xaa383 is not Phe
<400> 23

```
Met Gly Ser Ala Ser Val Met Gly Leu Thr Thr Ile Ser Leu Ala Asn
1               5                   10                  15
Cys Phe Asn Pro Thr Leu Pro Gln Lys Glu Lys Asn Arg Asn Glu Thr
            20                  25                  30
Thr His Phe Thr Ser Ile Val Ile Gly Thr Gly Tyr Gly Gly Ala Val
            35                  40                  45
Ser Ala Leu Arg Leu Gly Glu Ala Gly Val Asp Thr Leu Met Leu Glu
    50                  55                  60
Met Gly Gln Leu Trp Asp Lys Pro Gly Pro Asp Gly Lys Val Phe Cys
65                  70                  75                  80
Lys Met Thr Lys Pro Asp Gly Arg Ala Met Trp Phe Lys Asn Arg Thr
            85                  90                  95
Glu Ala Pro Leu Ser Ser Phe Leu Trp Ile Asp Ala Ile Asn Arg Pro
            100                 105                 110
Ile Asp Tyr Tyr Ala Gly Val Leu Asp Arg Ile Asn Tyr Pro Asn Met
        115                 120                 125
Ser Val Tyr Val Gly Arg Gly Val Gly Gly Gly Ser Leu Val Asn Gly
    130                 135                 140
Gly Xaa Ala Val Thr Pro Pro Met Asn Tyr Phe Gln Glu Ile Leu Pro
145                 150                 155                 160
Glu Val Asn Thr His Glu Met Tyr Asn Lys Tyr Phe Pro Arg Ala Asn
                165                 170                 175
Gln Lys Leu Gln Val Asn Thr Ile Pro Asn Thr Leu Leu Glu Asn Ser
            180                 185                 190
Pro Tyr Tyr Arg Phe Thr Arg Val Gly Arg Gln Gln Ala Glu Lys Ala
        195                 200                 205
Gly Phe Lys Thr Val Thr Xaa Pro Asn Ile Tyr Asp Tyr Asn Tyr Met
210                 215                 220
```

```
Gln Gln Glu Glu Ala Gly Lys Val His Lys Ser Ala Phe Gly Lys Glu
225             230                 235                 240
Val Ile Tyr Gly Asn Asn Gly Gly Lys Arg Ser Leu Asp Lys Thr Tyr
            245                 250                 255
Leu Ala Asp Ala Leu Gly Thr Gly Lys Val Thr Leu Lys Tyr Leu His
            260                 265                 270
Arg Val Asp Ala Ile Thr Gln Asn Ser Gln Gly Leu Tyr Gln Ile Asp
            275                 280                 285
Val Ser Glu Ile Asn Thr Ser Gly Ala Thr Val Ala Lys Lys Thr Phe
    290                 295                 300
Thr Cys Lys His Leu Phe Met Cys Ala Gly Ser Val Gly Ser Thr Glu
305                 310                 315                 320
Met Leu Val Arg Ala Arg Glu Thr Gly Lys Leu Pro Ser Leu Pro Ser
            325                 330                 335
Glu Val Gly Thr His Trp Gly Asn Asn Gly Asn Val Met Thr Ala Arg
            340                 345                 350
Ala Asn His Met Trp His Pro Thr Gly Thr Lys Gln Ser Thr Ile Pro
            355                 360                 365
Ala Met Gly Ile Asn Asp Trp Asp Asn Ala Ser Asn Pro Val Xaa Ala
    370                 375                 380
Glu Ile Ala Pro Leu Pro Thr Gly Phe Glu Thr Trp Ile Ser Leu Tyr
385                 390                 395                 400
Leu Ala Ile Thr Lys Asn Pro Glu Arg Gly His Phe Glu Tyr Asp Ala
            405                 410                 415
Thr Lys Gln Gln Ala Val Leu Arg Trp Gly Ala His Gln Ser Gln Pro
            420                 425                 430
Ser Ile Asn Ser Ala Lys Ala Met Phe Asp Lys Ile Asn Lys Ala Asn
            435                 440                 445
Thr Thr Ile Tyr Arg Tyr Asp Leu Phe Gly Asn Asn Lys Ala Phe Ala
    450                 455                 460
Asp Asp Phe Thr Tyr His Pro Leu Gly Gly Cys Val Leu Gly Lys Ala
465                 470                 475                 480
Thr Asp Leu Tyr Gly Arg Ile Lys Gly Tyr Ser Asn Leu Tyr Val Asn
            485                 490                 495
Asp Gly Ala Leu Val Pro Gly Asn Thr Gly Val Asn Pro Phe Ile Thr
            500                 505                 510
Ile Thr Ala Met Ala Glu Arg Asn Ile Glu Lys Ile Ile Gln Glu Asp
        515                 520                 525
Met Leu Lys
        530
```

<210> 24

<211> 541

<212> PRT

<213> Kribbella flavida

<220>

<221> MISC_FEATURE

<222> (157)..(157)

<223> Xaa is Met, Phe, Leu, Val, Cys, Ile, Ala, Gln, Tyr, Lys, or Ser

<220>

<221> MISC_FEATURE

<222> (226)..(226)

<223> Xaa is Val, Met, Ile, Ala, Thr, Lys, Cys, Ser, Gly, Glu, Tyr, Pro, Asn, Gln, Trp or His

<221> MISC_FEATURE

<222> (393)..(393)

<223> Xaa is Phe, Trp, Ser, Thr, Lys, Ala, Asn, His or Asp, provided that when xaa157 is Met, then xaa393 is not Phe

<400> 24

```
Met Thr Ala Ser Asn Glu Thr Asn Gln Ser Val Val Thr Arg Arg Arg
1                   5                  10                      15
Phe Ala Gly Leu Ala Ala Phe Thr Ser Ala Ala Ala Leu Gly Leu Ser
            20                  25                  30
Arg Val Gly Asp Ala Val Ala Ala Glu Arg Ser Phe Val Pro Ala Val
            35                  40                  45
Val Val Gly Thr Gly Tyr Gly Ala Ala Val Thr Ala Leu Arg Leu Gly
    50                  55                  60
Glu Ala Gly Val Ala Thr Thr Met Leu Glu Met Gly Gln Pro Trp Asn
65                  70                  75                      80
```

```
Gln Pro Gly Ala Asp Gly Lys Val Phe Cys Ser Thr Leu Ala Pro Asp
            85                    90                    95
Arg Arg Ser Met Trp Phe His Arg Arg Thr Ala Ala Pro Leu Asp Thr
            100                   105                   110
Phe Leu Trp Leu Asp Val Val Asn Arg Asp Leu Gly Thr Pro Tyr Ala
        115                   120                   125
Gly Val Leu Asp Arg Ile Asp Phe Pro Ala Met Asp Val Tyr Val Gly
        130                   135                   140
Arg Gly Val Gly Gly Gly Ser Leu Val Asn Gly Gly Xaa Ala Pro Thr
145                   150                   155                   160
Pro Arg Arg Ser Tyr Phe Glu Gln Val Leu Pro Arg Val Asp Ala Asp
                165                   170                   175
Gln Met Tyr Arg Arg Trp Phe Pro Leu Ala Asn Arg Met Leu Gly Val
                180                   185                   190
Asn Ser Ile Asp Pro Arg Tyr Leu Glu Thr Thr Pro Ala Tyr Arg Tyr
        195                   200                   205
Ala Arg Val Ser Arg Arg His Ala His Gln Ala Gly Phe Arg Thr Ala
    210                   215                   220
Val Xaa Pro Asn Val Tyr Asp Phe Gly Tyr Leu Glu Gln Glu Glu Arg
225                   230                   235                   240
Arg Gln Val Pro Arg Ser Ala Leu Ala Gly Glu Val Ile Tyr Gly Asn
                245                   250                   255
Asn His Gly Lys Gln Ser Leu Asp Lys Thr Tyr Leu Ala Asp Ala Val
            260                   265                   270
Gly Thr Gly Arg Val Thr Ile Arg Thr Leu Thr Arg Val Val Ser Val
            275                   280                   285
Arg Ala Asp Arg Arg Gly Tyr Val Leu Gly Leu Glu Gln Ile Asp Ala
    290                   295                   300
Ser Gly Lys Val Val Arg Arg Ser Glu Leu Gly Cys Arg Gln Leu Phe
305                   310                   315                   320
Leu Gly Ala Gly Ser Ile Gly Thr Thr Glu Leu Leu Leu Arg Ala Arg
            325                   330                   335
Glu Thr Gly Thr Leu Pro Asp Leu Pro Asp Ala Ile Gly Glu Gly Trp
            340                   345                   350
Gly Thr Asn Gly Asn Val Met Thr Ala Arg Ala Asn His Ala Trp Asp
            355                   360                   365
Pro Thr Gly Ser Leu Gln Ser Thr Ile Pro Ala Val Ala Ile Asp Asn
370                   375                   380
Trp Asp Asp Pro Val His Pro Ala Xaa Ala Glu Ile Ala Pro Leu Pro
385                   390                   395                   400
Thr Gly Leu Glu Thr Trp Ala Gly Leu Tyr Leu Ala Ile Thr Ala Asn
            405                   410                   415
Pro Glu Arg Gly Arg Leu Ser Tyr Asp His Ala Thr Asp Arg Ala Ile
            420                   425                   430
Leu His Trp Gln Ala Ser Gln Ser Thr Pro Ser Ile Gln Ala Ala Lys
        435                   440                   445
Ala Leu Phe Asp Arg Ile Asn Arg Ala Thr Gly Thr Thr Tyr Arg Arg
    450                   455                   460
Asp Leu Phe Ser Gly Asn Arg Ala Phe Ala Ala Asp Phe Cys Tyr His
465                   470                   475                   480
Pro Leu Gly Gly Cys Val Leu Gly Arg Ala Thr Asp Asp Tyr Gly Arg
                485                   490                   495
Val Arg Gly His Arg Asn Leu Tyr Val Thr Asp Ser Ala Leu Leu Pro
            500                   505                   510
Gly Ser Ile Gly Val Asn Pro Phe Val Thr Ile Thr Ala Leu Ala Glu
        515                   520                   525
Arg Asn Ile Ala Arg Val Ile Ala Thr Asp Leu Thr Arg
        530                   535                   540
```

<210> 25

<211> 543

<212> PRT

<213> Streptomyces sviceus

<220>

<221> MISC_FEATURE

<222> (159)..(159)

<223> xaa is Met, Phe, Leu, Val, cys, Ile, Ala, Gln, Tyr, Lys, or Ser

<220>
<221> MISC_FEATURE
<222> (228)..(228)
<223> Xaa is Val, Met, Ile, Ala, Thr, Lys, Cys, Ser, Gly, Glu, Tyr, Pro, Asn, Gln, Trp or His
<221> MISC_FEATURE
<222> (396)..(396)
<223> Xaa is Phe, Trp, Ser, Thr, Lys, Ala, Asn, His or Asp, provided that when xaa159 is Met, then Xaa396 is not Phe
<400> 25

```
Met Ala Thr Lys Leu Ile Gln Arg Gln Leu Thr Arg Arg Gln Ile Leu
1               5                   10                  15
Gly Met Ala Ala Leu Gln Thr Ala Ala Thr Leu Gly Phe Thr Arg Val
            20                  25                  30
Gly Leu Gln Ser Ala Arg Ala Asp Glu Pro Ala Ala Val Glu Ser Ala
        35                  40                  45
Pro Ala Ile Val Val Gly Ser Gly Tyr Gly Ala Ser Val Ala Ala Leu
    50                  55                  60
Arg Leu Gly Gln Ala Gly Ile Arg Thr Leu Val Leu Glu Met Gly Arg
65                  70                  75                  80
Leu Trp Asn Thr Ala Gly Pro Asp Gly Lys Val Phe Cys Asn Thr Ala
                85                  90                  95
Asn Pro Asp Gln Arg Ser Met Trp Phe Arg Thr Arg Thr Glu Ala Pro
            100                 105                 110
Leu Ala Thr Phe Leu Trp Leu Asp Val Val Asn Lys Asp Val Ser Pro
        115                 120                 125
Tyr Pro Gly Val Leu Asp Arg Val His Phe Asp His Met Ser Val Phe
    130                 135                 140
Val Gly Arg Gly Val Gly Gly Gly Ser Leu Val Asn Gly Ser Xaa Ala
145                 150                 155                 160
Val Thr Pro Leu Gln Ser Tyr Phe Ala Glu Gln Phe Pro Thr Val Asp
            165                 170                 175
Thr Ala Glu Met Tyr Gly Thr Tyr Phe Pro Arg Ala Arg Ala Met Leu
            180                 185                 190
Gly Val Asn Thr Ile Asp Pro Ala Trp Phe Glu Ser Thr Glu Trp Tyr
        195                 200                 205
Lys Phe Thr Arg Val Ser Arg Lys His Ala Gln Asn Thr Gly Leu Lys
    210                 215                 220
Thr Thr Phe Xaa Pro Ser Val Tyr Asp Phe Gly Tyr Met Gln Arg Glu
225                 230                 235                 240
Ala Ala Gly Thr Ala Thr Lys Ser Ala Leu Gly Gln Glu Val Ile Tyr
            245                 250                 255
Gly Asn Asn Phe Gly Lys Lys Ser Leu Asp Lys Thr Tyr Leu Ala Ser
            260                 265                 270
Ala Leu Gly Thr Gly Asn Val Thr Ile His Thr Met Glu Lys Val Thr
        275                 280                 285
Gly Ile Ser Arg Ala Gly Asp Gly Ser Trp Val Leu Ser Ala Glu Arg
    290                 295                 300
Ile Asp Tyr Ser Gly Ala Val Val Glu Thr Lys Gln Tyr Ser Cys Thr
305                 310                 315                 320
Tyr Leu Phe Leu Gly Gly Gly Ser Leu Gly Thr Ser Glu Leu Leu Leu
            325                 330                 335
Arg Ser Arg Gln Ser Gly Thr Leu Pro Ala Leu Asp Ala Ser Val Gly
            340                 345                 350
Ala Gly Trp Gly Pro Asn Gly Asn Thr Met Leu Gly Arg Ala Asn His
        355                 360                 365
Leu Trp Asp Thr Val Gly Ala Asn Gln Ser Thr Met Pro Val Met Gly
    370                 375                 380
Ile Asp Asp Trp Ala Asn Thr Asp Asn Pro Val Xaa Ala Glu Ile Ala
385                 390                 395                 400
Pro Leu Pro Thr Gly Leu Glu His Trp Val Ser Leu Tyr Leu Ala Ile
            405                 410                 415
Thr Lys Asn Pro Gln Arg Ala Arg Phe Ser Tyr Gly Ser Gly Gly Leu
        420                 425                 430
Ser Leu Asp Trp Ser Gly Ala Gln Ser Ala Val Ser Ser Gly Met Ala
        435                 440                 445
Lys Lys Leu Phe Asp Arg Ile Asn Ser Ala Asn Ser Thr Ile Tyr Arg
    450                 455                 460
Tyr Asp Leu Phe Gly Ser Pro Ser Arg Val Phe Ala Asp Asp Phe Thr
465                 470                 475                 480
```

```
Tyr His Pro Leu Gly Gly Cys Val Leu Gly Lys Ala Thr Asp Asn Tyr
                    485                 490                 495
Gly Arg Val Lys Gly Tyr Ser Arg Leu Tyr Val Thr Asp Gly Ser Leu
                500                 505                 510
Ile Pro Gly Asn Ile Gly Val Asn Pro Phe Val Thr Ile Thr Ala Leu
                515                 520                 525
Ala Glu Arg Thr Met Ala Arg Val Leu Val Glu Asp Thr Ala Pro
        530                 535                 540
```

<210> 26
<211> 534
<212> PRT
<213> Saccharomonospora viridis
<220>
<221> MISC_FEATURE
<222> (150)..(150)
<223> Xaa is Met, Phe, Leu, Val, Cys, Ile, Ala, Gln, Tyr, Lys, or Ser
<220>
<221> MISC_FEATURE
<222> (219)..(219)
<223> Xaa is Val, Met, Ile, Ala, Thr, Lys, Cys, Ser, Gly, Glu, Tyr, Pro, Asn,
Gln, Trp or His
<221> MISC_FEATURE
<222> (387)..(387)
<223> Xaa is Phe, Trp, Ser, Thr, Lys, Ala, Asn, His or Asp, provided that when
xaa150 is Met, then Xaa387 is not Phe
<400> 26

```
Met Arg Pro Leu Ser Arg Arg Arg Leu Leu Gly Leu Leu Ala Val Asn
1               5               10              15
Thr Ala Ser Ala Leu Gly Leu Gly Thr Ile Ala Thr Pro Ser Ala Ala
            20              25              30
Ala Ala Ser Arg Arg Asp Phe Ser Pro Ala Val Val Val Gly Thr Gly
        35              40              45
Tyr Gly Ala Ala Val Thr Ala Leu Arg Leu Gly Gln Ala Gly Ile Pro
    50              55              60
Thr Val Met Leu Glu Met Gly Arg Leu Trp Asp Thr Pro Gly Asp Asp
65              70              75              80
Gly Arg Val Phe Cys Asp Met Leu Asn Pro Asp Arg Arg Ala Met Trp
                85              90              95
Leu Ala Thr Arg Thr Gln Met Pro Leu Ser Ser Phe Leu Trp Leu Asp
            100             105             110
Ile Asp Arg Arg Ile Glu Arg Phe Thr Gly Val Leu Asp Cys Val His
        115             120             125
His Gly Asp Ile Ser Val Tyr Val Gly Arg Gly Val Gly Gly Gly Ser
    130             135             140
Leu Val Asn Gly Ala Xaa Ala Val Thr Pro Lys Arg Ala Thr Phe Ala
145             150             155             160
Glu Ala Phe Pro Asp Val Asp Ser Asp Gly Met Tyr Arg Thr Tyr Phe
            165             170             175
Pro Arg Ala Ala Ala Met Leu Gly Val Asn His Ile Asp Pro Ala Trp
        180             185             190
Phe Glu Thr Cys Glu Ser Tyr Arg Tyr Ala Arg Val Ser Arg Ala His
    195             200             205
Ala His Asn Ala Gly Leu Thr Thr Thr Phe Xaa Pro Ser Val Tyr Asp
    210             215             220
Phe Ala Arg Met Arg Arg Glu Glu Ala Gly Glu Val Pro Arg Ser Ala
225             230             235             240
Leu Ala Ala Glu Val Ile Tyr Gly Asn Asn His Gly Lys Arg Ser Leu
            245             250             255
Asp Lys Thr Tyr Leu Ala Asp Ala Leu Gly Thr Gly Cys Val Ser Ile
        260             265             270
Arg Thr Leu His His Val Arg Ala Ile Glu Gln Asp Ala Asp Gly Thr
        275             280             285
Tyr Val Leu Thr Val Asp Glu Leu Asp Leu Asp Gly Thr Arg Val Ala
    290             295             300
Thr Arg Gln Leu Gly Ala Arg Tyr Leu Phe Leu Gly Ala Gly Ser Leu
305             310             315             320
Gly Ser Thr Glu Leu Leu Leu Arg Ala Arg Asp Thr Gly Ala Leu Pro
                325             330             335
```

```
Gly Leu Ser Pro Leu Ile Gly Arg Asp Trp Gly Pro Asn Gly Asn Val
            340             345             350
Met Val Gly Arg Ala Asn Arg Pro Arg Asp Arg Thr Gly Thr Val Gln
            355             360             365
Ser Gly Met Pro Ala Leu Gly Ile Asp Ala Trp Asp Asp Pro Arg His
    370             375             380
Pro Val Xaa Ala Glu Val Ala Pro Met Pro Ala Gly Val Glu Leu Trp
385             390             395             400
Val Ser Leu Tyr Leu Ala Val Thr Arg Asn Pro Glu Arg Gly Leu Leu
                405             410             415
Thr Tyr Asp Ala Gly Ser Asp Arg Val Arg Leu His Trp Leu Ser Gly
            420             425             430
Gln Ala Gln Pro Ser Val Asp Gln Ala Lys Ala Leu Phe Asp Arg Leu
            435             440             445
Asn Ala Ala Asn Gly Thr Glu Tyr Arg Ser Asp Leu Phe Gly Asp Thr
    450             455             460
Arg Val Phe Glu Thr Arg Leu Thr Tyr His Pro Leu Gly Gly Ala Val
465             470             475             480
Leu Gly Lys Ala Thr Asp Ala Tyr Gly Arg Val Arg Gly Gln Arg Arg
                485             490             495
Leu Tyr Val Thr Asp Gly Ser Leu Val Pro Gly Asn Ile Gly Val Asn
            500             505             510
Pro Phe Leu Thr Ile Thr Ala Leu Ala Glu Arg Asn Ile Glu Arg Ile
            515             520             525
Leu Ala Glu Asp Leu Arg
            530
```

<210> 27

<211> 552

<212> PRT

<213> Rhodococcus equi

<220>

<221> MISC_FEATURE

<222> (167)..(167)

<223> Xaa is Met, Phe, Leu, Val, Cys, Ile, Ala, Gln, Tyr, Lys, or Ser

<220>

<221> MISC_FEATURE

<222> (236)..(236)

<223> Xaa is Val, Met, Ile, Ala, Thr, Lys, Cys, Ser, Gly, Glu, Tyr, Pro, Asn, Gln, Trp or His

<221> MISC_FEATURE

<222> (404)..(404)

<223> Xaa is Phe, Trp, Ser, Thr, Lys, Ala, Asn, His or Asp, provided that when Xaa167 is Met, then Xaa404 is not Phe

<400> 27

```
Met Thr Asp Ser Arg Ala Asn Arg Ala Asp Ala Thr Arg Gly Val Ala
1               5                   10                  15
Ser Val Ser Arg Arg Arg Phe Leu Ala Gly Ala Gly Leu Thr Ala Gly
            20                  25                  30
Ala Ile Ala Leu Ser Ser Met Ser Thr Ser Ala Ser Ala Ala Pro Ser
        35                  40                  45
Arg Thr Leu Ala Asp Gly Asp Arg Val Pro Ala Leu Val Ile Gly Ser
    50                  55                  60
Gly Tyr Gly Gly Ala Val Ala Ala Leu Arg Leu Thr Gln Ala Gly Ile
65                  70                  75                  80
Pro Thr Gln Ile Val Glu Met Gly Arg Ser Trp Asp Thr Pro Gly Ser
                85                  90                  95
Asp Gly Lys Ile Phe Cys Gly Met Leu Asn Pro Asp Lys Arg Ser Met
            100                 105                 110
Trp Leu Ala Asp Lys Thr Asp Gln Pro Val Ser Asn Phe Met Gly Phe
        115                 120                 125
Gly Ile Asn Lys Ser Ile Asp Arg Tyr Val Gly Val Leu Asp Ser Glu
    130                 135                 140
Arg Phe Ser Gly Ile Lys Val Tyr Gln Gly Arg Gly Val Gly Gly Gly
145                 150                 155                 160
Ser Leu Val Asn Gly Gly Xaa Ala Val Thr Pro Lys Arg Asn Tyr Phe
                165                 170                 175
Glu Glu Ile Leu Pro Ser Val Asp Ser Asn Glu Met Tyr Asn Lys Tyr
            180                 185                 190
```

Phe Pro Arg Ala Asn Thr Gly Leu Gly Val Asn Asn Ile Asp Gln Ala
195                    200                    205

Trp Phe Glu Ser Thr Glu Trp Tyr Lys Phe Ala Arg Thr Gly Arg Lys
210                    215                    220

Thr Ala Gln Arg Ser Gly Phe Thr Thr Ala Phe Xaa Pro Asn Val Tyr
225                    230                    235                    240

Asp Phe Glu Tyr Met Lys Lys Glu Ala Ala Gly Gln Val Thr Lys Ser
245                    250                    255

Gly Leu Gly Gly Glu Val Ile Tyr Gly Asn Asn Ala Gly Lys Lys Ser
260                    265                    270

Leu Asp Lys Thr Tyr Leu Ala Gln Ala Ala Ala Thr Gly Lys Leu Thr
275                    280                    285

Ile Thr Thr Leu His Arg Val Thr Lys Val Ala Pro Ala Thr Gly Ser
290                    295                    300

Gly Tyr Ser Val Thr Met Glu Gln Ile Asp Glu Gln Gly Asn Val Val
305                    310                    315                    320

Ala Thr Lys Val Val Thr Ala Asp Arg Val Phe Phe Ala Ala Gly Ser
325                    330                    335

Val Gly Thr Ser Lys Leu Leu Val Ser Met Lys Ala Gln Gly His Leu
340                    345                    350

Pro Asn Leu Ser Ser Gln Val Gly Glu Gly Trp Gly Asn Asn Gly Asn
355                    360                    365

Ile Met Val Gly Arg Ala Asn His Met Trp Asp Ala Thr Gly Ser Lys
370                    375                    380

Gln Ala Thr Ile Pro Thr Met Gly Ile Asp Asn Trp Ala Asp Pro Thr
385                    390                    395                    400

Ala Pro Ile Xaa Ala Glu Ile Ala Pro Leu Pro Ala Gly Leu Glu Thr
405                    410                    415

Tyr Val Ser Leu Tyr Leu Ala Ile Thr Lys Asn Pro Glu Arg Ala Arg
420                    425                    430

Phe Gln Phe Asn Ser Gly Thr Gly Lys Val Asp Leu Thr Trp Ala Gln
435                    440                    445

Ser Gln Asn Gln Lys Gly Ile Asp Met Ala Lys Lys Val Phe Asp Lys
450                    455                    460

Ile Asn Gln Lys Glu Gly Thr Ile Tyr Arg Thr Asp Leu Phe Gly Val
465                    470                    475                    480

Tyr Phe Lys Thr Trp Gly Asp Asp Phe Thr Tyr His Pro Leu Gly Gly
485                    490                    495

Val Leu Leu Asn Lys Ala Thr Asp Asn Phe Gly Arg Leu Pro Glu Tyr
500                    505                    510

Pro Gly Leu Tyr Val Val Asp Gly Ser Leu Val Pro Gly Asn Val Gly
515                    520                    525

Val Asn Pro Phe Val Thr Ile Thr Ala Leu Ala Glu Arg Asn Met Asp
530                    535                    540

Lys Ile Ile Ser Ser Asp Ile Gln
545                    550

<210> 28
<211> 507
<212> PRT
<213> Brevibacterium sterolicum
<220>
<221> MISC_FEATURE
<222> (122)..(122)
<223> Xaa is Met, Phe, Leu, Val, Cys, Ile, Ala, Gln, Tyr, Lys, or Ser
<220>
<221> MISC_FEATURE
<222> (191)..(191)
<223> Xaa is Val, Met, Ile, Ala, Thr, Lys, Cys, Ser, Gly, Glu, Tyr, Pro, Asn, Gln, Trp or His
<221> MISC_FEATURE
<222> (359)..(359)
<223> Xaa is Phe, Trp, Ser, Thr, Lys, Ala, Asn, His or Asp, provided that when Xaa122 is Met, then Xaa359 is not Phe
<400> 28

Ala Pro Ser Arg Thr Leu Ala Asp Gly Asp Arg Val Pro Ala Leu Val
1                5                        10                        15
Ile Gly Ser Gly Tyr Gly Gly Ala Val Ala Ala Leu Arg Leu Thr Gln
                20                        25                        30

Ala Gly Ile Pro Thr Gln Ile Val Glu Met Gly Arg Ser Trp Asp Thr
        35                40                45
Pro Gly Ser Asp Gly Lys Ile Phe Cys Gly Met Leu Asn Pro Asp Lys
    50                55                60
Arg Ser Met Trp Leu Ala Asp Lys Thr Asp Gln Pro Val Ser Asn Phe
65                70                75                        80
Met Gly Phe Gly Ile Asn Lys Ser Ile Asp Arg Tyr Val Gly Val Leu
                85                90                        95
Asp Ser Glu Arg Phe Ser Gly Ile Lys Val Tyr Gln Gly Arg Gly Val
            100                105                110
Gly Gly Gly Ser Leu Val Asn Gly Gly Xaa Ala Val Thr Pro Lys Arg
            115                120                125
Asn Tyr Phe Glu Glu Ile Leu Pro Ser Val Asp Ser Asn Glu Met Tyr
    130                135                140
Asn Lys Tyr Phe Pro Arg Ala Asn Thr Gly Leu Gly Val Asn Asn Ile
145                150                155                        160
Asp Gln Ala Trp Phe Glu Ser Thr Glu Trp Tyr Lys Phe Ala Arg Thr
                165                170                175
Gly Arg Lys Thr Ala Gln Arg Ser Gly Phe Thr Thr Ala Phe Xaa Pro
            180                185                190
Asn Val Tyr Asp Phe Glu Tyr Met Lys Lys Glu Ala Ala Gly Gln Val
            195                200                205
Thr Lys Ser Gly Leu Gly Gly Glu Val Ile Tyr Gly Asn Asn Ala Gly
    210                215                220
Lys Lys Ser Leu Asp Lys Thr Tyr Leu Ala Gln Ala Ala Ala Thr Gly
225                230                235                        240
Lys Leu Thr Ile Thr Thr Leu His Arg Val Thr Lys Val Ala Pro Ala
                245                250                255
Thr Gly Ser Gly Tyr Ser Val Thr Met Glu Gln Ile Asp Glu Gln Gly
            260                265                270
Asn Val Val Ala Thr Lys Val Val Thr Ala Asp Arg Val Phe Phe Ala
    275                280                285
Ala Gly Ser Val Gly Thr Ser Lys Leu Leu Val Ser Met Lys Ala Gln
    290                295                300
Gly His Leu Pro Asn Leu Ser Ser Gln Val Gly Glu Gly Trp Gly Asn
305                310                315                        320
Asn Gly Asn Ile Met Val Gly Arg Ala Asn His Met Trp Asp Ala Thr
                325                330                335
Gly Ser Lys Gln Ala Thr Ile Pro Thr Met Gly Ile Asp Asn Trp Ala
            340                345                350
Asp Pro Thr Ala Pro Ile Xaa Ala Glu Ile Ala Pro Leu Pro Ala Gly
            355                360                365
Leu Glu Thr Tyr Val Ser Leu Tyr Leu Ala Ile Thr Lys Asn Pro Glu
    370                375                380
Arg Ala Arg Phe Gln Phe Asn Ser Gly Thr Gly Lys Val Asp Leu Thr
385                390                395                        400
Trp Ala Gln Ser Gln Asn Gln Lys Gly Ile Asp Met Ala Lys Lys Val
                405                410                415
Phe Asp Lys Ile Asn Gln Lys Glu Gly Thr Ile Tyr Arg Thr Asp Leu
            420                425                430
Phe Gly Val Tyr Tyr Lys Thr Trp Gly Asp Asp Phe Thr Tyr His Pro
            435                440                445
Leu Gly Gly Val Leu Leu Asn Lys Ala Thr Asp Asn Phe Gly Arg Leu
    450                455                460
Pro Glu Tyr Pro Gly Leu Tyr Val Val Asp Gly Ser Leu Val Pro Gly
465                470                475                        480
Asn Val Gly Val Asn Pro Phe Val Thr Ile Thr Ala Leu Ala Glu Arg
                485                490                495
Asn Met Asp Lys Ile Ile Ser Ser Asp Ile Gln
            500                505

<210> 29
<211> 552

<212> PRT
<213> Brevibacterium sterolicum
<220>
<221> MISC_FEATURE
<222> (167)..(167)
<223> Xaa is Met, Phe, Leu, Val, Cys, Ile, Ala, Gln, Tyr, Lys, or Ser
<220>
<221> MISC_FEATURE
<222> (236)..(236)
<223> Xaa is Val, Met, Ile, Ala, Thr, Lys, Cys, Ser, Gly, Glu, Tyr, Pro, Asn, Gln, Trp or His
<221> MISC_FEATURE
<222> (404)..(404)
<223> Xaa is Phe, Trp, Ser, Thr, Lys, Ala, Asn, His or Asp, provided that when Xaa167 is Met, then Xaa404 is not Phe
<400> 29

```
Met Thr Asp Ser Arg Ala Asn Arg Ala Asp Ala Thr Arg Gly Val Ala
1               5                   10                  15
Ser Val Ser Arg Arg Arg Phe Leu Ala Gly Ala Gly Leu Thr Ala Gly
            20                  25                  30
Ala Ile Ala Leu Ser Ser Met Ser Thr Ser Ala Ser Ala Ala Pro Ser
        35                  40                  45
Arg Thr Leu Ala Asp Gly Asp Arg Val Pro Ala Leu Val Ile Gly Ser
        50                  55                  60
Gly Tyr Gly Gly Ala Val Ala Ala Leu Arg Leu Thr Gln Ala Gly Ile
65                  70                  75                  80
Pro Thr Gln Ile Val Glu Met Gly Arg Ser Trp Asp Thr Pro Gly Ser
                85                  90                  95
Asp Gly Lys Ile Phe Cys Gly Met Leu Asn Pro Asp Lys Arg Ser Met
            100                 105                 110
Trp Leu Ala Asp Lys Thr Asp Gln Pro Val Ser Asn Phe Met Gly Phe
        115                 120                 125
Gly Ile Asn Lys Ser Ile Asp Arg Tyr Val Gly Val Leu Asp Ser Glu
        130                 135                 140
Arg Phe Ser Gly Ile Lys Val Tyr Gln Gly Arg Gly Val Gly Gly Gly
145                 150                 155                 160
Ser Leu Val Asn Gly Gly Xaa Ala Val Thr Pro Lys Arg Asn Tyr Phe
                165                 170                 175
Glu Glu Ile Leu Pro Ser Val Asp Ser Asn Glu Met Tyr Asn Lys Tyr
            180                 185                 190
Phe Pro Arg Ala Asn Thr Gly Leu Gly Val Asn Asn Ile Asp Gln Ala
            195                 200                 205
Trp Phe Glu Ser Thr Glu Trp Tyr Lys Phe Ala Arg Thr Gly Arg Lys
        210                 215                 220
Thr Ala Gln Arg Ser Gly Phe Thr Thr Ala Phe Xaa Pro Asn Val Tyr
225                 230                 235                 240
Asp Phe Glu Tyr Met Lys Lys Glu Ala Ala Gly Gln Val Thr Lys Ser
                245                 250                 255
Gly Leu Gly Gly Glu Val Ile Tyr Gly Asn Asn Ala Gly Lys Lys Ser
            260                 265                 270
Leu Asp Lys Thr Tyr Leu Ala Gln Ala Ala Ala Thr Gly Lys Leu Thr
        275                 280                 285
Ile Thr Thr Leu His Arg Val Thr Lys Val Ala Pro Ala Thr Gly Ser
        290                 295                 300
Gly Tyr Ser Val Thr Met Glu Gln Ile Asp Glu Gln Gly Asn Val Val
305                 310                 315                 320
Ala Thr Lys Val Val Thr Ala Asp Arg Val Phe Phe Ala Ala Gly Ser
                325                 330                 335
Val Gly Thr Ser Lys Leu Leu Val Ser Met Lys Ala Gln Gly His Leu
            340                 345                 350
Pro Asn Leu Ser Ser Gln Val Gly Glu Gly Trp Gly Asn Asn Gly Asn
        355                 360                 365
Ile Met Val Gly Arg Ala Asn His Met Trp Asp Ala Thr Gly Ser Lys
    370                 375                 380
Gln Ala Thr Ile Pro Thr Met Gly Ile Asp Asn Trp Ala Asp Pro Thr
385                 390                 395                 400
Ala Pro Ile Xaa Ala Glu Ile Ala Pro Leu Pro Ala Gly Leu Glu Thr
            405                 410                 415
Tyr Val Ser Leu Tyr Leu Ala Ile Thr Lys Asn Pro Glu Arg Ala Arg
            420                 425                 430
Phe Gln Phe Asn Ser Gly Thr Gly Lys Val Asp Leu Thr Trp Ala Gln
        435                 440                 445
Ser Gln Asn Gln Lys Gly Ile Asp Met Ala Lys Lys Val Phe Asp Lys
    450                 455                 460
```

81

```
Ile Asn Gln Lys Glu Gly Thr Ile Tyr Arg Thr Asp Leu Phe Gly Val
465                 470                 475                 480
Tyr Phe Lys Thr Trp Gly Asp Asp Phe Thr Tyr His Pro Leu Gly Gly
                485                 490                 495
Val Leu Leu Asn Lys Ala Thr Asp Asn Phe Gly Arg Leu Pro Glu Tyr
            500                 505                 510
Pro Gly Leu Tyr Val Val Asp Gly Ser Leu Val Pro Gly Asn Val Gly
        515                 520                 525
Val Asn Pro Phe Val Thr Ile Thr Arg Leu Ala Glu Arg Asn Met Asp
    530                 535                 540
Lys Ile Ile Ser Ser Asp Ile Gln
545                 550
```

<210> 30

<211> 551

<212> PRT

<213> Rhodococcus equi

<220>

<221> MISC_FEATURE

<222> (167)..(167)

<223> Xaa is Met, Phe, Leu, Val, Cys, Ile, Ala, Gln, Tyr, Lys, or Ser

<220>

<221> MISC_FEATURE

<222> (236)..(236)

<223> Xaa is Val, Met, Ile, Ala, Thr, Lys, Cys, Ser, Gly, Glu, Tyr, Pro, Asn, Gln, Trp or His

<221> MISC_FEATURE

<222> (404)..(404)

<223> Xaa is Phe, Trp, Ser, Thr, Lys, Ala, Asn, His or Asp, provided that when Xaa167 is Met, then Xaa404 is not Phe

<400> 30

EP 2 748 312 B1

```
Met Thr Asp Ser Arg Ala Asn Arg Ala Asp Ala Thr Arg Gly Val Ala
1               5               10              15
Ser Val Ser Arg Arg Arg Phe Leu Ala Gly Ala Gly Leu Thr Ala Gly
            20              25              30
Ala Ile Ala Leu Ser Ser Met Ser Thr Ser Ala Ser Ala Ala Pro Ser
        35              40              45
Arg Thr Leu Ala Asp Gly Asp Arg Val Pro Ala Leu Val Ile Gly Ser
    50              55              60
Gly Tyr Gly Gly Ala Val Ala Ala Leu Arg Leu Thr Gln Ala Gly Ile
65              70              75              80
Pro Thr Gln Ile Val Glu Met Gly Arg Ser Trp Asp Thr Pro Gly Ser
            85              90              95
Asp Gly Lys Ile Phe Cys Gly Met Leu Asn Pro Asp Lys Arg Ser Met
            100             105             110
Trp Leu Ala Asp Lys Thr Asp Gln Pro Val Ser Asn Phe Met Gly Phe
        115             120             125
Gly Ile Asn Lys Ser Ile Asp Arg Tyr Val Gly Val Leu Asp Ser Glu
    130             135             140
Arg Phe Ser Gly Ile Lys Val Tyr Gln Gly Arg Gly Val Gly Gly Gly
145             150             155             160
Ser Leu Val Asn Gly Gly Xaa Ala Val Thr Pro Lys Arg Asn Tyr Phe
            165             170             175
Glu Glu Ile Leu Pro Ser Val Asp Ser Asn Glu Met Tyr Asn Lys Tyr
            180             185             190
Phe Pro Arg Ala Asn Thr Gly Leu Gly Val Asn Asn Ile Asp Gln Ala
        195             200             205
Trp Phe Glu Ser Thr Glu Trp Tyr Lys Phe Ala Arg Thr Gly Arg Lys
    210             215             220
Thr Ala Gln Arg Ser Gly Phe Thr Thr Ala Phe Xaa Pro Asn Val Tyr
225             230             235             240
Asp Phe Glu Tyr Met Lys Lys Glu Ala Ala Gly Gln Val Thr Lys Ser
            245             250             255
Gly Leu Gly Gly Glu Val Ile Tyr Gly Asn Asn Ala Gly Lys Lys Ser
            260             265             270
Leu Asp Lys Thr Tyr Leu Ala Gln Ala Ala Ala Thr Gly Lys Leu Thr
        275             280             285
Ile Thr Thr Leu His Arg Val Thr Lys Val Ala Pro Ala Thr Gly Ser
    290             295             300
```

83

```
Gly Tyr Ser Val Thr Met Glu Gln Ile Asp Glu Gln Gly Asn Val Val
305                 310                 315                 320
Ala Thr Lys Val Val Thr Ala Asp Arg Val Phe Phe Ala Ala Gly Ser
            325                 330                 335
Val Gly Thr Ser Lys Leu Leu Val Ser Met Lys Ala Gln Gly His Leu
            340                 345                 350
Pro Asn Leu Ser Ser Gln Val Gly Glu Gly Trp Gly Asn Asn Gly Asn
            355                 360                 365
Ile Met Val Gly Arg Ala Asn His Met Trp Asp Ala Thr Gly Ser Lys
        370                 375                 380
Gln Ala Thr Ile Pro Thr Met Gly Ile Asp Asn Trp Ala Asp Pro Thr
385                 390                 395                 400
Ala Pro Ile Xaa Ala Glu Ile Ala Pro Leu Pro Ala Gly Leu Glu Thr
            405                 410                 415
Tyr Val Ser Leu Tyr Leu Ala Ile Thr Lys Asn Pro Glu Arg Ala Arg
            420                 425                 430
Phe Gln Phe Asn Ser Gly Thr Gly Lys Val Asp Leu Thr Trp Ala Gln
        435                 440                 445
Ser Gln Asn Gln Lys Gly Ile Asp Met Ala Lys Lys Val Phe Asp Lys
    450                 455                 460
Ile Asn Gln Lys Glu Gly Thr Ile Tyr Arg Thr Asp Leu Phe Gly Val
465                 470                 475                 480
Tyr Lys Thr Trp Gly Asp Asp Phe Thr Tyr His Pro Leu Gly Gly Val
            485                 490                 495
Leu Leu Asn Lys Ala Thr Asp Asn Phe Gly Arg Leu Pro Glu Tyr Pro
        500                 505                 510
Gly Leu Tyr Val Val Asp Gly Ser Leu Val Pro Gly Asn Val Gly Val
        515                 520                 525
Asn Pro Phe Val Thr Ile Thr Ala Leu Ala Glu Arg Asn Met Asp Lys
    530                 535                 540
Ile Ile Ser Ser Asp Ile Gln
545                 550
```

<210> 31

<211> 551

<212> PRT

<213> Brevibacterium sp.

<220>

<221> MISC_FEATURE

<222> (167)..(167)

<223> Xaa is Met, Phe, Leu, Val, Cys, Ile, Ala, Gln, Tyr, Lys, or Ser

<220>

<221> MISC_FEATURE

<222> (236)..(236)

<223> Xaa is Val, Met, Ile, Ala, Thr, Lys, Cys, Ser, Gly, Glu, Tyr, Pro, Asn, Gln, Trp or His

<221> MISC_FEATURE

<222> (404)..(404)

<223> Xaa is Phe, Trp, Ser, Thr, Lys, Ala, Asn, His or Asp, provided that when Xaa167 is Met, then Xaa404 is not Phe

<400> 31

```
Met Thr Asp Ser Arg Ala Asn Arg Ala Asp Ala Thr Arg Gly Val Ala
1               5                   10                  15
Ser Val Ser Arg Arg Arg Phe Leu Ala Gly Ala Gly Leu Thr Ala Gly
            20                  25                  30
Ala Ile Ala Leu Ser Ser Met Ser Thr Ser Ala Ser Ala Ala Pro Ser
        35                  40  ·           45
Arg Thr Leu Ala Asp Gly Asp Arg Val Pro Ala Leu Val Ile Gly Ser
    50                  55                  60
Gly Tyr Gly Gly Ala Val Ala Ala Leu Arg Leu Thr Gln Ala Gly Ile
65                  70                  75                  80
Pro Thr Gln Ile Val Glu Met Gly Arg Ser Trp Asp Thr Pro Gly Ser
            85                  90                  95
Asp Gly Lys Ile Phe Cys Gly Met Leu Asn Pro Asp Lys Arg Ser Met
            100                 105                 110
Arg Leu Ala Asp Lys Thr Asp Gln Pro Val Ser Asn Phe Met Gly Phe
        115                 120                 125
Gly Ile Asn Lys Ser Ile Asp Arg Tyr Val Gly Val Leu Asp Ser Glu
    130                 135                 140
```

```
Arg Phe Ser Gly Ile Lys Val Tyr Gln Gly Arg Gly Val Gly Gly Gly
145             150             155             160
Ser Leu Val Asn Gly Gly Xaa Ala Val Thr Pro Lys Arg Asn Tyr Phe
            165             170             175
Glu Glu Ile Leu Pro Ser Val Asp Ser Asn Glu Met Tyr Asn Lys Tyr
        180             185             190
Phe Pro Arg Ala Asn Thr Gly Leu Gly Val Asn Asn Ile Asp Gln Ala
        195             200             205
Trp Phe Glu Ser Thr Glu Trp Tyr Lys Phe Ala Arg Thr Gly Arg Lys
    210             215             220
Thr Ala Gln Arg Ser Gly Phe Thr Thr Ala Phe Xaa Pro Asn Val Tyr
225             230             235             240
Asp Phe Glu Tyr Met Lys Lys Glu Ala Ala Gly Gln Val Thr Lys Ser
            245             250             255
Gly Leu Gly Gly Glu Val Ile Tyr Gly Asn Asn Ala Gly Lys Lys Ser
        260             265             270
Leu Asp Lys Thr Tyr Leu Ala Gln Ala Ala Ala Thr Gly Lys Leu Thr
        275             280             285
Ile Thr Thr Leu His Arg Val Thr Lys Val Ala Pro Ala Thr Gly Ser
    290             295             300
Gly Tyr Ser Val Thr Met Glu Gln Ile Asp Glu Gln Gly Asn Val Val
305             310             315             320
Ala Thr Lys Val Val Thr Ala Asp Arg Val Phe Phe Ala Ala Gly Ser
            325             330             335
Val Gly Thr Ser Lys Leu Leu Val Ser Met Lys Ala Gln Gly His Leu
            340             345             350
Pro Asn Leu Ser Ser Gln Val Gly Glu Gly Trp Gly Asn Asn Gly Asn
        355             360             365
Ile Met Val Gly Arg Ala Asn His Met Trp Asp Ala Thr Gly Ser Lys
    370             375             380
Gln Ala Thr Ile Pro Thr Met Gly Ile Asp Asn Trp Ala Asp Pro Ala
385             390             395             400
Ala Pro Ile Xaa Ala Glu Ile Ala Pro Leu Pro Ala Gly Leu Glu Thr
            405             410             415
Tyr Val Ser Leu Tyr Leu Ala Ile Thr Lys Asn Pro Glu Arg Ala Arg
            420             425             430
Phe Gln Phe Asn Ser Gly Thr Gly Lys Val Asp Leu Thr Trp Ala Gln
        435             440             445
Ser Gln Asn Gln Lys Gly Ile Asp Met Ala Lys Lys Val Phe Asp Lys
    450             455             460
Ile Asn Gln Lys Glu Gly Thr Ile Tyr Arg Thr Asp Leu Phe Gly Val
465             470             475             480
Tyr Lys Thr Trp Gly Asp Asp Phe Thr Tyr His Pro Leu Gly Gly Val
            485             490             495
Leu Leu Asn Lys Ala Thr Asp Asn Phe Gly Arg Leu Pro Glu Tyr Pro
        500             505             510
Gly Leu Tyr Val Val Asp Gly Ser Leu Val Pro Gly Asn Val Gly Val
        515             520             525
Asn Pro Phe Val Thr Ile Thr Ala Leu Ala Glu Arg Asn Met Asp Lys
    530             535             540
Ile Ile Ser Ser Asp Ile Gln
545             550
```

<210> 32

<211> 551

<212> PRT

<213> Rhodococcus sp.

<220>

<221> MISC_FEATURE

<222> (167)..(167)

<223> Xaa is Met, Phe, Leu, Val, Cys, Ile, Ala, Gln, Tyr, Lys, or Ser

<220>

<221> MISC_FEATURE

<222> (236)..(236)

<223> Xaa is Val, Met, Ile, Ala, Thr, Lys, Cys, Ser, Gly, Glu, Tyr, Pro, Asn, Gln, Trp or His

<221> MISC_FEATURE
<222> (405)..(405)
<223> Xaa is Phe, Trp, Ser, Thr, Lys, Ala, Asn, His or Asp, provided that when Xaa167 is Met, then Xaa405 is not Phe
<400> 32

```
Met Thr Asp Ser Arg Ala Asn Arg Ala Asp Ala Thr Arg Gly Val Ala
1               5                   10                  15
Ser Val Ser Arg Arg Arg Phe Leu Ala Gly Ala Gly Leu Thr Ala Gly
            20                  25                  30
Ala Ile Ala Leu Ser Ser Met Ser Thr Ser Ala Ser Ala Ala Pro Ser
        35                  40                  45
Arg Thr Leu Ala Asp Gly Asp Arg Val Pro Ala Leu Val Ile Gly Ser
    50                  55                  60
Gly Tyr Gly Gly Ala Val Ala Ala Leu Arg Leu Thr Gln Ala Gly Ile
65                  70                  75                  80
Pro Thr Gln Ile Val Glu Met Gly Arg Ser Trp Asp Thr Pro Gly Ser
                85                  90                  95
Asp Gly Lys Ile Phe Cys Gly Met Leu Asn Pro Asp Lys Arg Ser Met
            100                 105                 110
Trp Leu Ala Asp Lys Thr Asp Gln Pro Val Ser Asn Phe Met Gly Phe
        115                 120                 125
Gly Ile Asn Lys Ser Ile Asp Arg Tyr Val Gly Val Leu Asp Ser Glu
        130                 135                 140
Arg Phe Ser Gly Ile Lys Val Tyr Gln Gly Arg Gly Val Gly Gly Gly
145                 150                 155                 160
Ser Leu Val Asn Gly Gly Xaa Ala Val Thr Pro Lys Arg Asn Tyr Phe
                165                 170                 175
Glu Glu Ile Leu Pro Ser Val Asp Ser Asn Glu Met Tyr Asn Lys Tyr
            180                 185                 190
Phe Pro Arg Ala Asn Thr Gly Leu Gly Val Asn His Ile Asp Gln Ala
        195                 200                 205
Trp Phe Glu Ser Thr Glu Trp Tyr Lys Phe Ala Arg Thr Gly Arg Lys
        210                 215                 220
Thr Ala Gln Arg Ser Gly Phe Thr Thr Ala Phe Xaa Pro Asn Val Tyr
225                 230                 235                 240
Asp Phe Glu Tyr Met Lys Lys Glu Ala Ala Gly Gln Val Thr Lys Ser
            245                 250                 255
Gly Leu Gly Gly Glu Val Ile Tyr Gly Asn Asn Ala Gly Lys Lys Ser
        260                 265                 270
Leu Asp Lys Thr Tyr Leu Ala Gln Ala Ala Ala Thr Gly Lys Leu Thr
    275                 280                 285
Ile Thr Thr Leu His Arg Val Thr Lys Val Ala Pro Ala Thr Gly Ser
    290                 295                 300
Gly Tyr Ser Val Thr Met Glu Gln Ile Asp Glu Gln Gly Asn Val Val
305                 310                 315                 320
Ala Ala Thr Lys Val Val Thr Ala Asp Arg Val Phe Phe Ala Ala Gly
            325                 330                 335
Ser Val Gly Thr Ser Lys Leu Leu Val Ser Met Lys Ala Gln Gly His
        340                 345                 350
Leu Pro Asn Leu Ser Ser Gln Val Gly Glu Gly Trp Gly Asn Asn Gly
        355                 360                 365
Asn Ile Met Val Gly Arg Ala Asn His Met Trp Asp Ala Thr Gly Ser
    370                 375                 380
Lys Gln Ala Thr Ile Pro Thr Met Gly Ile Asp Asn Trp Ala Asp Pro
385                 390                 395                 400
Thr Ala Pro Ile Xaa Ala Glu Ile Ala Pro Leu Pro Ala Gly Leu Glu
            405                 410                 415
Thr Tyr Val Ser Leu Tyr Leu Ala Ile Thr Lys Asn Pro Glu Arg Ala
        420                 425                 430
Arg Phe Gln Phe Asn Ser Gly Thr Gly Lys Val Asp Leu Thr Trp Ala
    435                 440                 445
Gln Ser Gln Asn Gln Lys Gly Ile Asp Met Ala Lys Lys Val Phe Asp
    450                 455                 460
Lys Ile Asn Gln Lys Glu Gly Thr Ile Tyr Arg Thr Asp Leu Phe Gly
465                 470                 475                 480
Val Tyr Lys Thr Trp Gly Asp Asp Phe Thr Tyr His Pro Leu Gly Gly
            485                 490                 495
Val Leu Leu Asn Lys Ala Thr Asp Asn Phe Gly Arg Leu Pro Glu Tyr
        500                 505                 510
Pro Gly Leu Tyr Val Val Asp Gly Ser Leu Val Pro Gly Asn Val Gly
        515                 520                 525
```

88

```
Val Asn Pro Phe Val Thr Ile Thr Ala Leu Ala Arg Asn Met Asp Lys
    530                     535                 540
Ile Ile Ser Ser Asp Ile Gln
545                 550
```

<210> 33
<211> 533
<212> PRT
<213> Rhodococcus sp.
<220>
<221> MISC_FEATURE
<222> (1) .. (1)
<223> Xaa is unknown
<220>
<221> MISC_FEATURE
<222> (158)..(158)
<223> Xaa is Met, Phe, Leu, Val, Cys, Ile, Ala, Gln, Tyr, Lys, or Ser
<220>
<221> MISC_FEATURE
<222> (227)..(227)
<223> Xaa is Val, Met, Ile, Ala, Thr, Lys, Cys, Ser, Gly, Glu, Tyr, Pro, Asn,
Gln, Trp or His
<221> MISC_FEATURE
<222> (395)..(395)
<223> Xaa is Phe, Trp, Ser, Thr, Lys, Ala, Asn, His or Asp, provided that when
xaa158 is Met, then xaa395 is not Phe
<400> 33

```
Xaa Ala Thr Arg Gly Val Ala Ser Val Ser Arg Arg Arg Phe Leu Ala
1               5                   10                  15
Gly Ala Gly Leu Thr Ala Gly Ala Ile Ala Leu Ser Ser Met Ser Thr
            20                  25                  30
Ser Ala Ser Ala Ala Pro Ser Arg Thr Leu Ala Asp Gly Asp Arg Val
        35                  40                  45
Pro Ala Leu Val Ile Gly Ser Gly Tyr Gly Gly Ala Val Ala Ala Leu
    50                  55                  60
Arg Leu Thr Gln Ala Gly Ile Pro Thr Gln Ile Val Glu Met Gly Arg
65                  70                  75                  80
Ser Trp Asp Thr Pro Gly Ser Asp Gly Lys Ile Phe Cys Gly Met Leu
                85                  90                  95
Asn Pro Asp Lys Arg Ser Met Trp Leu Ala Asp Lys Thr Asp Gln Pro
            100                 105                 110
Val Ser Asn Phe Met Gly Phe Gly Ile Asn Lys Ser Ile Asp Arg Tyr
            115                 120                 125
Val Gly Val Leu Asp Ser Glu Arg Phe Ser Gly Ile Lys Val Tyr Gln
    130                 135                 140
Gly Arg Gly Val Gly Gly Gly Ser Leu Val Asn Gly Gly Xaa Ala Val
145                 150                 155                 160
Thr Pro Lys Arg Asn Tyr Phe Glu Glu Ile Leu Pro Ser Val Asp Ser
                165                 170                 175
Asn Glu Met Tyr Asn Lys Tyr Phe Pro Arg Ala Asn Thr Gly Leu Gly
            180                 185                 190
Val Asn Asn Ile Asp Gln Ala Trp Phe Glu Ser Thr Glu Trp Tyr Lys
            195                 200                 205
Phe Ala Arg Thr Gly Arg Lys Thr Ala Gln Arg Ser Gly Phe Thr Thr
    210                 215                 220
Ala Phe Xaa Pro Asn Val Tyr Asp Phe Glu Tyr Met Lys Lys Glu Ala
225                 230                 235                 240
Ala Gly Gln Val Thr Lys Ser Gly Leu Gly Gly Glu Val Ile Tyr Gly
                245                 250                 255
Asn Asn Ala Gly Lys Lys Ser Leu Asp Lys Thr Tyr Leu Ala Gln Ala
            260                 265                 270
Ala Ala Thr Gly Lys Leu Thr Ile Thr Thr Leu His Arg Val Thr Lys
        275                 280                 285
Val Ala Pro Ala Thr Gly Ser Gly Tyr Ser Val Thr Met Glu Gln Ile
    290                 295                 300
Asp Glu Gln Gly Asn Val Val Ala Thr Lys Val Val Thr Ala Asp Arg
305                 310                 315                 320
Val Phe Phe Ala Ala Gly Ser Val Gly Thr Ser Lys Leu Leu Val Ser
                325                 330                 335
```

90

```
Met Lys Ala Gln Gly His Leu Pro Asn Leu Ser Ser Gln Val Gly Glu
        340                     345                 350
Gly Trp Gly Asn Asn Gly Asn Ile Met Val Gly Arg Ala Asn His Met
        355                 360                 365
Trp Asp Ala Thr Gly Ser Lys Gln Ala Thr Ile Pro Thr Met Gly Ile
    370             375                 380
Asp Asn Trp Ala Asp Pro Thr Ala Pro Ile Xaa Ala Glu Ile Ala Pro
385                 390                 395                     400
Leu Pro Ala Gly Leu Glu Thr Tyr Val Ser Leu Tyr Leu Ala Ile Thr
                405                 410                 415
Lys Asn Pro Glu Arg Ala Arg Phe Gln Phe Asn Ser Gly Thr Gly Lys
            420                 425                 430
Val Asp Leu Thr Trp Ala Gln Ser Gln Asn Gln Lys Gly Ile Asp Met
        435                 440                 445
Ala Lys Lys Val Phe Asp Lys Ile Asn Gln Lys Glu Gly Thr Ile Tyr
        450                 455                 460
Arg Thr Asp Leu Phe Gly Val Tyr Lys Thr Trp Gly Asp Asp Phe Thr
465                 470                 475                     480
Tyr His Pro Leu Gly Val Leu Leu Asn Lys Ala Thr Asp Asn Phe
                485                 490                 495
Gly Arg Leu Pro Glu Tyr Pro Gly Leu Tyr Val Val Asp Gly Ser Leu
            500                 505                 510
Val Pro Gly Asn Val Gly Val Asn Pro Phe Val Thr Ile Thr Ala Leu
        515                 520                 525
Ala Glu Arg Asn Met
        530
```

<210> 34
<211> 552
<212> PRT
<213> Rhodococcus erythropolis
<220>
<221> MISC_FEATURE
<222> (167)..(167)
<223> Xaa is Met, Phe, Leu, Val, Cys, Ile, Ala, Gln, Tyr, Lys, or Ser
<220>
<221> MISC_FEATURE
<222> (236)..(236)
<223> Xaa is Val, Met, Ile, Ala, Thr, Lys, Cys, Ser, Gly, Glu, Tyr, Pro, Asn, Gln, Trp or His
<221> MISC_FEATURE
<222> (405)..(405)
<223> Xaa is Phe, Trp, Ser, Thr, Lys, Ala, Asn, His or Asp, provided that when Xaa167 is Met, then xaa405 is not Phe
<400> 34

```
Met Ser Ile Arg Ala Gly Ser Asn Glu Arg His Ala Arg Thr Asn Ser
1               5                   10                  15
Thr Leu Ser Arg Arg Asn Phe Leu Ala Ala Thr Gly Leu Ala Val Gly
            20                  25                  30
Ala Ala Ala Leu Ser Ser Ser Trp Thr Thr Ala Ala Ala Ala Pro Arg
            35                  40                  45
Arg Ala Leu Ser Asp Gly Asp Arg Val Pro Ala Leu Val Ile Gly Ser
    50                  55                  60
Gly Tyr Gly Gly Ala Val Ala Ala Leu Arg Leu Thr Gln Ala Gly Ile
65                  70                  75                  80
Asp Thr His Met Val Glu Met Gly Lys Ser Trp Thr Thr Pro Gly Ser
            85                  90                  95
Asp Gly Lys Val Phe Cys Pro Met Leu Ser Pro Asp Gly Arg Ser Phe
            100                 105                 110
Trp Leu Arg Asp Arg Thr Val Gln Pro Val Ser His Phe Ser Gly Gly
        115                 120                 125
Ser Val Asp Lys Asn Ile Ser Arg Tyr Val Gly Val Leu Asp Ala Glu
    130                 135                 140
Asp Phe Gly Gly Ile Lys Val Tyr Gln Gly Arg Gly Val Gly Gly Gly
145                 150                 155                 160
Ser Leu Val Asn Gly Gly Xaa Ala Val Thr Pro Lys Arg Asn Tyr Phe
                165                 170                 175
Glu Glu Ile Leu Pro Gly Val Asp Ser Asn Glu Met Tyr Ser Thr Tyr
            180                 185                 190
```

```
Phe Pro Arg Ala Asn Ala Ala Leu Gly Val Asn Asn Ile Asp Pro Ala
        195             200             205
Trp Phe Glu Ser Thr Glu Tyr Tyr Lys Phe Ala Arg Thr Gly Arg Lys
    210             215             220
Thr Ala Glu Arg Ser Gly Tyr Thr Thr Thr Phe Xaa Pro Asn Val Tyr
225             230             235             240
Asp Phe Asn Tyr Met Lys Gln Glu Ala Ala Gly Gln Val Thr Lys Ser
            245             250             255
Ala Leu Val Ser Glu Val Ile Tyr Gly Asn Asn Ala Gly Lys Lys Ser
        260             265             270
Leu Asp Lys Thr Tyr Leu Ala Ala Ala Ser Ala Thr Gly Lys Leu Thr
        275             280             285
Ile Ser Ala Leu His Val Val Thr Ser Val Ala Pro Ala Ala Thr Gly
    290             295             300
Gly Gly Tyr Gln Val Val Met Asn Gln Ile Asn Glu Gln Gly Asn Thr
305             310             315             320
Val Gly Thr Lys Thr Val Thr Ala Asp Lys Val Phe Phe Ala Ala Gly
            325             330             335
Ser Ile Gly Thr Ser Lys Leu Leu Val Ala Met Lys Ala Gln Gly Gln
        340             345             350
Leu Ala Asn Leu Pro Gly Ala Val Gly Gln Glu Trp Gly His Asn Gly
        355             360             365
Asn Val Met Val Gly Arg Ala Asn His Met Trp Asp Ala Thr Gly Ala
    370             375             380
Lys Gln Ser Ala Ile Pro Val Met Gly Ile Asp Asn Trp Ala Asp Thr
385             390             395             400
Ser Ala Pro Val Xaa Ala Glu Ile Ala Pro Phe Pro Ala Gly Thr Glu
            405             410             415
Leu Trp Val Ser Leu Tyr Leu Ala Ile Ala Lys Asn Pro Gln Arg Ala
        420             425             430
Gln Phe Gln Phe Asn Ser Ala Thr Gly Lys Val Gly Leu Asn Trp Gln
    435             440             445
Arg Ser Gln Asn Gln Pro Ser Ile Asp Met Ala Lys Lys Leu Phe Asp
    450             455             460
Lys Ile Asn Lys Lys Glu Gly Thr Ile Tyr Arg Thr Asp Leu Phe Gly
465             470             475             480
Pro Val Gln Thr Trp Gly Asp Gln Leu Thr Tyr His Pro Leu Gly Gly
            485             490             495
Cys Val Leu Gly Lys Ala Thr Asp Gly Tyr Gly Arg Leu Pro Glu Tyr
        500             505             510
Pro Gly Leu Tyr Val Met Asp Gly Ser Leu Val Pro Gly Asn Val Gly
        515             520             525
Val Asn Pro Phe Val Thr Ile Thr Ala Leu Ala Glu Arg Asn Ile Glu
    530             535             540
Asn Ile Ile Ala Asn Asp Met Asn
545             550
```

<210> 35

<211> 522

<212> PRT

<213> Rhodococcus erythropolis

<220>

<221> MISC_FEATURE

<222> (137)..(137)

<223> Xaa is Met, Phe, Leu, Val, cys, Ile, Ala, Gln, Tyr, Lys, or Ser

<220>

<221> MISC_FEATURE

<222> (206)..(206)

<223> Xaa is Val, Met, Ile, Ala, Thr, Lys, Cys, Ser, Gly, Glu, Tyr, Pro, Asn, Gln, Trp or His

<221> MISC_FEATURE

<222> (375)..(375)

<223> Xaa is Phe, Trp, Ser, Thr, Lys, Ala, Asn, His or Asp, provided that when xaal37 is Met, then Xaa375 is not Phe

<400> 35

EP 2 748 312 B1

```
Met Gly Ala Ala Ala Leu Ser Ser Ser Trp Thr Thr Ala Ala Ala Ala
1               5                   10              15
Pro Arg Arg Ala Leu Asn Asp Gly Asp Arg Val Pro Ala Leu Val Ile
            20              25              30

Gly Ser Gly Tyr Gly Gly Ala Val Ala Ala Leu Arg Leu Thr Gln Ala
        35              40              45
Gly Ile Asp Thr His Met Val Glu Met Gly Lys Ser Trp Thr Thr Pro
    50              55              60
Gly Ser Asp Gly Lys Val Phe Cys Pro Met Leu Ser Pro Asp Gly Arg
65              70              75              80
Ser Phe Trp Leu Arg Asp Arg Thr Val Gln Pro Val Ser His Phe Ser
            85              90              95
Gly Gly Ser Val Asp Lys Asn Ile Ser Arg Tyr Val Gly Val Leu Asp
            100             105             110
Ala Glu Asp Phe Gly Gly Ile Lys Val Tyr Gln Gly Arg Gly Val Gly
            115             120             125
Gly Gly Ser Leu Val Asn Gly Gly Xaa Ala Val Thr Pro Lys Arg Asn
        130             135             140
Tyr Phe Glu Glu Ile Leu Pro Gly Val Asp Ser Asn Glu Met Tyr Ser
145             150             155             160
Thr Tyr Phe Pro Arg Ala Asn Ala Ala Leu Gly Val Asn Asn Ile Asp
                165             170             175
Pro Ala Trp Phe Glu Ser Thr Glu Tyr Tyr Lys Phe Ala Arg Thr Gly
            180             185             190
Arg Lys Thr Ala Glu Arg Ser Gly Tyr Thr Thr Thr Phe Xaa Pro Asn
        195             200             205
Val Tyr Asp Phe Asn Tyr Met Lys Gln Glu Ala Ala Gly Gln Val Thr
    210             215             220
Lys Ser Ala Leu Val Ser Glu Val Ile Tyr Gly Asn Asn Ala Gly Lys
225             230             235             240
Lys Ser Leu Asp Lys Thr Tyr Leu Ala Ala Ala Ser Ala Thr Gly Lys
            245             250             255
Leu Thr Ile Ser Ala Leu His Val Val Thr Ser Val Ala Pro Ala Ala
            260             265             270
Thr Gly Gly Gly Tyr Gln Val Val Met Asn Gln Ile Asn Glu Gln Gly
        275             280             285
Asn Thr Val Gly Thr Lys Thr Val Thr Ala Asp Lys Val Phe Phe Ala
    290             295             300
Ala Gly Ser Ile Gly Thr Ser Lys Leu Leu Val Ala Met Lys Ala Gln
305             310             315             320
Gly Gln Leu Ala Asn Leu Pro Gly Ala Val Gly Gln Glu Trp Gly His
            325             330             335
Asn Gly Asn Val Met Val Gly Arg Ala Asn His Met Trp Asp Ala Thr
            340             345             350
Gly Ala Lys Gln Ser Ala Ile Pro Val Met Gly Ile Asp Asn Trp Ala
        355             360             365
Asp Thr Ser Ala Pro Val Xaa Ala Glu Ile Ala Pro Phe Pro Ala Gly
    370             375             380
Thr Glu Leu Trp Val Ser Leu Tyr Leu Ala Ile Ala Lys Asn Pro Gln
385             390             395             400
Arg Ala Gln Phe Gln Phe Asn Ser Ala Thr Gly Lys Val Gly Leu Asn
            405             410             415
Trp Gln Arg Ser Gln Asn Gln Pro Ser Ile Asp Met Ala Lys Lys Leu
            420             425             430
Phe Asp Lys Ile Asn Lys Lys Glu Gly Thr Ile Tyr Arg Thr Asp Leu
        435             440             445
Phe Gly Pro Thr Gln Thr Trp Gly Asp Gln Leu Thr Tyr His Pro Leu
    450             455             460
Gly Gly Cys Val Leu Gly Lys Ala Thr Asp Gly Tyr Gly Arg Leu Pro
465             470             475             480
Glu Tyr Pro Gly Leu Tyr Val Met Asp Gly Ser Leu Val Pro Gly Asn
            485             490             495
Val Gly Val Asn Pro Phe Val Thr Ile Thr Ala Leu Ala Glu Arg Asn
        500             505             510
Ile Glu Asn Ile Ile Ala Asn Asp Met Asn
        515             520
```

94

<210> 36
<211> 523
<212> PRT
<213> Amycolatopsis mediterranei
<220>
<221> MISC_FEATURE
<222> (142)..(142)
<223> Xaa is Met, Phe, Leu, Val, Cys, Ile, Ala, Gln, Tyr, Lys, or Ser
<220>
<221> MISC_FEATURE
<222> (211)..(211)
<223> Xaa is Val, Met, Ile, Ala, Thr, Lys, Cys, Ser, Gly, Glu, Tyr, Pro, Asn, Gln, Trp or His
<221> MISC_FEATURE
<222> (377)..(377)
<223> Xaa is Phe, Trp, Ser, Thr, Lys, Ala, Asn, His or Asp, provided that when Xaa142 is Met, then Xaa377 is not Phe
<400> 36

```
Met Ala Ser Ala Ala Leu Ala Gly Arg Ala Thr Ala Ala Ser Ser Gln
1               5                   10                  15
Pro Ala Val Ala Gly Ser Ala Ala Ala Ile Ser Asp Gly Ala Arg Val
            20                  25                  30
Thr Ala Leu Val Ile Gly Thr Gly Tyr Gly Gly Ser Val Ala Ala Leu
        35                  40                  45
Arg Leu Ala Gln Ala Gly Val Asp Val Gln Met Val Glu Met Gly Met
    50                  55                  60
Ala Trp Asp Thr Pro Gly Ala Asp Gly Lys Ile Phe Cys Thr Thr Pro
65                  70                  75                  80
Asn Pro Asp Gln Arg Ser Phe Trp Leu Arg Thr Arg Thr Lys Gln Pro
                85                  90                  95
Leu Ser Asn Phe Leu Gly Phe Pro Ile Asp Lys Asp Ile Pro Arg Tyr
            100                 105                 110
Thr Gly Ile Leu Asp Ala Glu Glu Phe Ser Gly Ile Thr Val Tyr Gln
        115                 120                 125
Gly Arg Gly Val Gly Gly Gly Ser Leu Val Asn Gly Gly Xaa Ala Val
    130                 135                 140
Thr Pro Lys Arg Glu Asn Phe Gly Ala Ile Leu Pro Thr Val Asp Ala
145                 150                 155                 160
Asn Glu Met Tyr Asp Val Tyr Tyr Pro Arg Ala Asn Ala Gly Leu Gly
                165                 170                 175
Val Ala Ser Val Arg Pro Ala Trp Phe Glu Thr Thr Asp Trp Tyr Gln
            180                 185                 190
Phe Ala Arg Val Gly Arg Lys Gln Ala Gln Arg Ser Gly Phe Pro Phe
        195                 200                 205
Val Phe Xaa Pro Asp Val Tyr Asp Trp Asp Tyr Met Glu Arg Glu Ala
    210                 215                 220
Ala Gly Thr Ala Thr Lys Ser Ala Leu Ala Gly Glu Ile Leu Phe Gly
225                 230                 235                 240
Asn Asn Tyr Gly Lys Lys Ser Leu Gln Lys Thr Tyr Leu Pro Lys Ile
                245                 250                 255
Ala Ala Thr Gly Arg Val Thr Ile Ser Pro Leu His Arg Val Thr Gln
            260                 265                 270
Val Val Pro Ala Ser Gly Gly Gly Tyr Thr Val Thr Ile Glu Gln Leu
        275                 280                 285
Thr Thr Asp Gly Ala Val Ser Ala Ile Lys Thr Val Thr Ala Ala Lys
    290                 295                 300
Val Phe Phe Ala Ala Gly Ser Val Gly Thr Ser Lys Leu Leu Val Lys
305                 310                 315                 320
Leu Lys Ala Thr Gly Ala Leu Pro Asn Leu Asn Gly Glu Val Gly Lys
                325                 330                 335
Gly Trp Gly Asp Asn Gly Asn Val Met Val Gly Arg Ala Asn Gln Ile
            340                 345                 350
Trp Asp Pro Thr Gly Ala Ser Gln Ser Thr Ile Pro Cys Gly Gly Ile
        355                 360                 365
Asp Asn Trp Ala Ala Gly Gly Ala Xaa Ala Glu Val Ala Pro Leu Pro
    370                 375                 380
Thr Gly Ile Glu Thr Trp Ala Ser Phe Tyr Leu Ser Ile Thr Lys Asn
385                 390                 395                 400
Pro Asn Arg Ala Gln Phe Thr Trp Asn Pro Thr Thr Arg Ala Val Asp
                405                 410                 415
Leu Asn Trp Gln Thr Ala Trp Lys Gln Pro Gly Ile Asp Met Ala Lys
            420                 425                 430
Thr Ile Phe Asp Lys Ile Asn Ala Thr Glu Gly Thr Ile Tyr Arg Thr
        435                 440                 445
```

```
Asp Leu Phe Gly Thr Tyr Lys Thr Trp Gly Asp His Leu Thr Tyr His
    450                 455                 460
Pro Leu Gly Gly Ala Val Leu Gly Lys Ala Thr Asp Asn Tyr Gly Arg
465                 470                 475                 480
Leu Ala Gly His Pro Gly Leu Tyr Ala Ile Asp Gly Ser Leu Ile Pro
                485                 490                 495
Gly Asn Thr Ser Val Asn Pro Phe Val Thr Ile Thr Ala Leu Ala Glu
            500                 505                 510
Arg Asn Ile Glu Lys Ile Ile Ala Gln Asp Phe
        515                 520
```

<210> 37
<211> 547
<212> PRT
<213> Streptosporangium roseum
<220>
<221> MISC_FEATURE
<222> (166)..(166)
<223> Xaa is Met, Phe, Leu, Val, Cys, Ile, Ala, Gln, Tyr, Lys, or Ser
<220>
<221> MISC_FEATURE
<222> (235)..(235)
<223> Xaa is Val, Met, Ile, Ala, Thr, Lys, Cys, Ser, Gly, Glu, Tyr, Pro, Asn,
Gln, Trp or His
<221> MISC_FEATURE
<222> (401)..(401)
<223> Xaa is Phe, Trp, Ser, Thr, Lys, Ala, Asn, His or Asp, provided that when
xaa166 is Met, then Xaa401 is not Phe
<400> 37

```
Met Ser Asp Asn Thr Ser Gly Ser Thr Asp Ser Lys Gly Ile Ser Arg
1               5                   10              15
Arg Gly Phe Ile Ala Gly Thr Gly Ser Ile Leu Gly Val Ala Ala Leu
            20                  25              30
Thr Gly Arg Ala Thr Ala Ala Gln Ala Ala Ala Leu Pro Ala Ala Ala
            35                  40              45
Pro Ile Ser Ser Gly Ala His Val Pro Ala Leu Val Ile Gly Thr Gly
    50              55                  60
Tyr Gly Gly Ser Val Ala Ala Leu Arg Leu Ala Gln Ala Gly Val Asp
65              70                  75                      80
Val His Met Ile Glu Met Gly Met Ala Trp Asp Thr Pro Gly Ser Asp
                85                  90                  95
Gly Lys Ile Phe Cys Asn Thr Arg Glu Pro Asp Tyr Arg Ser Tyr Trp
            100                 105                 110
Leu Arg Thr Lys Ser Lys Ala Pro Leu Asn Tyr Phe Leu Gly Phe Pro
            115                 120                 125
Ile Asp Arg Asn Ile Pro Arg Tyr Thr Gly Ile Leu Asp Ala Glu Asp
    130                 135                 140
Phe Ser Gly Ile Thr Val Tyr Gln Gly Arg Gly Val Gly Gly Gly Ser
145                 150                 155                 160
Leu Val Asn Gly Gly Xaa Ala Val Thr Pro Lys Arg Glu Asn Phe Gly
                165                 170                 175
Ala Val Leu Pro Ser Val Asn Ala Ala Glu Met Tyr Asp Ile Tyr Tyr
            180                 185                 190
Pro Arg Ala Asn Ala Gly Leu Gly Val Ser Ser Ile Asp Pro Ala Trp
            195                 200                 205
Phe Asp Ser Thr Ala Cys Tyr Gln Tyr Ala Arg Val Gly Arg Lys His
    210                 215                 220
Ala Gln Arg Ser Gly Phe Pro Phe Val Phe Xaa Pro Asp Val Tyr Asp
225                 230                 235                 240
Trp Asp Tyr Met Lys Gln Glu Ala Ala Gly Thr Val Thr Lys Ser Ala
                245                 250                 255
Leu Ala Gly Glu Ile Leu Tyr Gly Asn Asn His Gly Lys Lys Ser Leu
            260                 265                 270
Gln Gln Thr Tyr Ile Ala Arg Ala Lys Ala Thr Gly Arg Val Ala Ile
            275                 280                 285
Ser Pro Leu His Lys Val Thr Ser Val Ala Pro Ala Ala Gly Gly Gly
    290                 295                 300
Tyr Thr Val Val Ile Asp Gln Ile Asn Thr Asn Gly Asp Thr Thr Ala
305                 310                 315                 320
```

```
Thr Lys Thr Val Thr Ala Asp Arg Val Phe Phe Ala Ala Gly Ser Val
            325                     330                 335
Gly Thr Ser Lys Leu Leu Val Lys Leu Lys Ala Thr Gly Ala Leu Pro
            340                 345                 350
Asn Leu Asn Asp Glu Ile Gly Lys Gly Trp Gly Asp Asn Gly Asn Val
            355                 360                 365
Met Cys Gly Arg Ala Asn His Met Trp Asp Pro Thr Gly Ser Leu Gln
    370                 375                 380
Ser Ala Ile Pro Cys Ala Gly Ile Asp Asn Trp Ala Ala Gly Gly Ala
385                 390                 395                     400
Xaa Ala Glu Val Ala Pro Leu Pro Thr Gly Ile Glu Thr Tyr Ala Ser
            405                 410                 415
Phe Tyr Leu Ser Ile Thr Lys Asn Pro Asn Arg Ala Gln Phe Ser Trp
            420                 425                 430
Asn Ala Ala Thr Gly Lys Val Asp Leu Asn Trp Gln Thr Ser Trp Lys
            435                 440                 445
Gln Pro Ser Ile Asp Met Ala Lys Thr Ile Phe Asp Lys Ile Asn Ser
    450                 455                 460
Lys Glu Gly Thr Ile Tyr Arg Thr Asp Leu Phe Gly Thr Tyr Lys Ile
465                 470                 475                     480
Trp Gly Asp His Leu Thr Tyr His Pro Leu Gly Gly Ala Val Leu Asn
            485                 490                 495
Lys Ala Thr Asp Asn Tyr Gly Arg Leu Ala Gly His Pro Gly Leu Tyr
            500                 505                 510
Val Ile Asp Gly Ser Leu Ile Pro Gly Asn Thr Ser Val Asn Pro Phe
    515                 520                 525
Val Thr Ile Thr Ala Leu Ala Glu Arg Asn Ile Glu Lys Ile Ile Ala
    530                 535                 540
Thr Asp Leu
545
```

<210> 38
<211> 549
<212> PRT
<213> Streptomyces sp.
<220>
<221> MISC_FEATURE
<222> (168)..(168)
<223> Xaa is Met, Phe, Leu, Val, Cys, Ile, Ala, Gln, Tyr, Lys, or Ser
<220>
<221> MISC_FEATURE
<222> (237)..(237)
<223> Xaa is Val, Met, Ile, Ala, Thr, Lys, Cys, Ser, Gly, Glu, Tyr, Pro, Asn, Gln, Trp or His
<221> MISC_FEATURE
<222> (403)..(403)
<223> Xaa is Phe, Trp, Ser, Thr, Lys, Ala, Asn, His or Asp, provided that when Xaa168 is Met, then Xaa403 is not Phe
<400> 38

Met Ser Asp Lys Ser Leu His Ser Lys Val Ser Gly Gly Val Ser Arg
1                   5                   10                  15
Arg Gly Phe Ile Ala Gly Thr Gly Ser Ile Leu Gly Ala Val Ala Leu
            20                  25                  30
Thr Val Asn Val Thr Pro Ala His Ala Glu Pro Ala Thr Thr Thr Ala
            35                  40                  45
Ser Gly Pro Ile Glu Ser Gly Ala Arg Val Pro Val Leu Val Ile Gly
    50                  55                  60
Thr Gly Tyr Gly Gly Ser Val Ala Ala Leu Arg Leu Ala Gln Ala Gly
65                  70                  75                  80
Val Pro Val His Met Val Glu Met Gly Met Ala Trp Asp Thr Pro Gly
                85                  90                  95
Ser Asp Gly Lys Ile Phe Ala Asn Thr Thr Lys Pro Asp Tyr Arg Ser
            100                 105                 110
Tyr Trp Leu Arg Thr Arg Thr Lys Ala Pro Leu Ser Asn Phe Leu Gly
            115                 120                 125
Phe Pro Ile Asp Lys Asp Val Pro Arg Tyr Thr Gly Ile Leu Asp Ala
    130                 135                 140
Glu Glu Met Gly Gly Ile Ile Val Tyr Gln Gly Arg Gly Val Gly Gly
145                 150                 155                 160

```
Gly Ser Leu Val Asn Gly Gly Xaa Ala Val Thr Pro Lys Arg Gln Asn
            165                 170                 175
Phe Ala Ala Ile Leu Pro Ser Val Asp Ala Glu Glu Met Tyr Arg Thr
            180                 185                 190
Tyr Tyr Pro Arg Ala Asn Ala Gly Leu Gly Val Gly Leu Ile Asp Pro
        195                 200                 205
Val Trp Phe Glu Ala Val Asp Cys Tyr Gln Phe Ala Arg Val Gly Arg
    210                 215                 220
Lys His Ala Gln Arg Ser Gly Phe Pro Phe Val Phe Xaa Pro Asp Val
225                 230                 235                 240
Tyr Asp Trp Asp Tyr Met Lys Gln Glu Val Ala Gly Thr Val Pro Lys
            245                 250                 255
Ser Ala Val Asp Gly Glu Ile Leu Tyr Gly Asn Asn Ala Gly Lys Lys
        260                 265                 270
Ser Leu Gln Gln Thr Tyr Leu Ala Ala Ala Arg Ala Thr Gly Lys Val
        275                 280                 285
Thr Ile Ser Pro Leu His Arg Val Thr Thr Val Ser Pro Ser Asp Gly
    290                 295                 300
Gly Gly Tyr Thr Val Val Met Glu Gln Leu Ser Thr Ser Gly Asp Val
305                 310                 315                 320
Leu Ala Thr Lys Thr Val Thr Ala Gly Arg Val Phe Phe Ala Ala Gly
            325                 330                 335
Ser Val Gly Thr Ser Lys Leu Leu Val Arg Leu Lys Ala Thr Gly Ala
            340                 345                 350
Leu Pro Asn Leu Asn Asp Glu Val Gly Lys Gly Trp Gly Asp Asn Gly
        355                 360                 365
Asn Val Met Cys Gly Arg Ala Asn His Met Trp Asp Pro Thr Gly Lys
    370                 375                 380
Val Gln Ala Ser Ile Pro Cys Gly Gly Ile Asp Asn Trp Asp Ala Gly
385                 390                 395                 400
Gly Ala Xaa Ala Glu Val Ala Pro Leu Pro Thr Gly Ile Glu Thr Tyr
            405                 410                 415
Ala Ser Phe Tyr Leu Ser Ile Thr Lys Asn Pro Asn Arg Ala Arg Phe
        420                 425                 430
Ser Trp Asn Ala Ala Ala Gly Lys Val Glu Leu Asp Trp Gln Thr Ala
    435                 440                 445
Trp Lys Gln Pro Ser Ile Asp Met Ala Lys Thr Ile Phe Asp Lys Ile
    450                 455                 460
Asn Ala Lys Glu Gly Thr Ile Tyr Arg Thr Asp Leu Phe Gly Thr Asn
465                 470                 475                 480
Lys Val Trp Gly Asp His Leu Thr Tyr His Pro Leu Gly Gly Ala Val
            485                 490                 495
Leu Gly Lys Ala Thr Asp Asn Tyr Gly Arg Leu His Gly His Pro Gly
        500                 505                 510
Leu Tyr Val Ile Asp Gly Ala Leu Ile Pro Gly Asn Thr Ser Val Asn
        515                 520                 525
Pro Phe Ala Thr Ile Thr Ala Leu Ala Glu Arg Asn Ile Glu Lys Ile
530                 535                 540
Ile Ala Thr Asp Leu
545
```

<210> 39

<211> 546

<212> PRT

<213> catenulispora acidiphila

<220>

<221> MISC_FEATURE

<222> (165)..(165)

<223> Xaa is Met, Phe, Leu, Val, Cys, Ile, Ala, Gln, Tyr, Lys, or Ser

<220>

<221> MISC_FEATURE

<222> (234)..(234)

<223> Xaa is Val, Met, Ile, Ala, Thr, Lys, Cys, Ser, Gly, Glu, Tyr, Pro, Asn, Gln, Trp or His

<221> MISC_FEATURE

<222> (400)..(400)

<223> Xaa is Phe, Trp, Ser, Thr, Lys, Ala, Asn, His or Asp, provided that when xaa165 is Met, then xaa400 is not Phe
<400> 39

```
Met Ser Ala Thr Ser Arg His Asp Pro Gly Ala Arg Gly Leu Ser Arg
1               5                   10                  15
Arg Gly Phe Leu Ala Gly Thr Gly Thr Val Leu Gly Ala Ala Ala Leu
            20                  25                  30
Gly Gly Leu Ser Ala Ser Arg Ala Ser Ala Ala Gln Arg Ser Thr Pro
        35                  40                  45
Ile Ser Asn Gly Ala His Val Gln Ala Leu Ile Ile Gly Thr Gly Tyr
        50                  55                  60
Gly Gly Ser Val Ala Ala Leu Arg Leu Ala Gln Ala Gly Ile Ala Val
65                  70                  75                  80
Glu Met Ile Glu Met Gly Met Ala Trp Asp Thr Pro Gly Ser Asp Gly
                85                  90                  95
Lys Ile Phe Cys Asn Leu Thr Ser Pro Asp Gln Arg Ser Phe Trp Leu
            100                 105                 110
Arg Thr Gln Thr Lys Gln Pro Val Gly Tyr Phe Leu Gly Ile Pro Ile
        115                 120                 125
Asp Arg Ala Ile Pro Asn Tyr Thr Gly Ile Leu Asp Ala Glu Asp Phe
        130                 135                 140
Ala Gly Ile Thr Val Tyr Gln Gly Arg Gly Ile Gly Gly Gly Ser Leu
145                 150                 155                 160
Val Asn Gly Gly Xaa Ala Val Thr Pro Lys Gln Glu Asn Phe Gly Ala
                165                 170                 175
Ile Leu Pro Ser Val Asn Pro Ala Glu Met Tyr Asn Val Tyr Tyr Pro
            180                 185                 190
Arg Ala Asn Ala Gly Leu Gly Ala Gly Val Val Pro Gln Ser Trp Phe
        195                 200                 205
Thr Lys Thr Asp Trp Tyr Gln Phe Ala Arg Val Gly Gln Lys Gln Ala
    210                 215                 220
Gly Arg Ser Gly Phe Pro Phe Gln Phe Xaa Pro Asp Val Tyr Asp Trp
225                 230                 235                 240
Asn Tyr Met Gln Gln Glu Asp Ala Gly Thr Val Pro Lys Ser Ala Leu
                245                 250                 255
Gly Gln Glu Leu Leu Tyr Gly Asn Asn Tyr Gly Lys Lys Ser Leu Gln
            260                 265                 270
Lys Thr Tyr Ile Pro Ala Ala Leu Ala Thr Gly Lys Val Asn Ile Ser
        275                 280                 285
Pro Leu His Lys Val Thr Ser Val Ser Pro Ala Ser Gly Gly Gly Tyr
    290                 295                 300
Thr Val Leu Met Asn Gln Leu Asp Thr Ser Gly Asn Val Val Val Thr
305                 310                 315                 320
Lys Glu Val Thr Ala Asp Lys Val Val Phe Ala Ala Gly Ser Val Gly
                325                 330                 335
Thr Ser Lys Leu Leu Val Gln Met Arg Asp Thr Gly Gln Leu Pro His
            340                 345                 350
Leu Asn Asp Gln Val Gly Gln Gly Trp Gly Asp Asn Gly Asn Ile Met
        355                 360                 365
Val Gly Arg Ala Asn Gln Ile Trp Asp Pro Thr Gly Ser Lys Gln Ser
    370                 375                 380
Thr Val Pro Cys Gly Gly Ile Asp Asn Trp Thr Lys Gly Gly Ala Xaa
385                 390                 395                 400
Ala Glu Val Ala Pro Leu Pro Ile Gly Ile Glu Thr Trp Ala Ser Leu
                405                 410                 415
Tyr Leu Ser Ile Thr Lys Asn Pro His Arg Ala Gln Phe Thr Trp Asn
            420                 425                 430
Ala Ala Thr Gln Lys Val Asp Leu Ser Trp Gln Leu Ala Trp Lys Gln
        435                 440                 445
Asp Gly Ile Thr Met Ala Lys Ser Ile Phe Asp Lys Ile Asn Ser Thr
    450                 455                 460
Glu Gly Thr Ile Tyr Arg Thr Asp Leu Phe Gly Ser Tyr Lys Thr Trp
465                 470                 475                 480
Gln Asp Gln Leu Thr Tyr His Pro Leu Gly Gly Ala Val Leu Asn Gln
                485                 490                 495
Ala Thr Asp Asn Tyr Gly Arg Leu Thr Ala Tyr Pro Gly Leu Tyr Val
            500                 505                 510
Met Asp Gly Ala Leu Ile Pro Gly Asn Thr Ser Val Asn Pro Phe Val
        515                 520                 525
Thr Ile Thr Ala Leu Ala Glu Arg Asn Ile Glu Asn Ile Ile Ala Asn
    530                 535                 540
```

102

Gly Gly
545

<210> 40
<211> 550
<212> PRT
<213> Streptomyces roseosporus
<220>
<221> MISC_FEATURE
<222> (169)..(169)
<223> Xaa is Met, Phe, Leu, Val, Cys, Ile, Ala, Gln, Tyr, Lys, or Ser
<220>
<221> MISC_FEATURE
<222> (238)..(238)
<223> Xaa is Val, Met, Ile, Ala, Thr, Lys, Cys, Ser, Gly, Glu, Tyr, Pro, Asn, Gln, Trp or His
<221> MISC_FEATURE
<222> (404)..(404)
<223> Xaa is Phe, Trp, Ser, Thr, Lys, Ala, Asn, His or Asp, provided that when xaa169 is Met, then Xaa404 is not Phe
<400> 40

```
Met Cys His Met Asn Asp Thr Ser Met Gln Asn Ser Glu Thr Lys Gly
1               5               10              15
Val Ser Arg Arg Arg Phe Ile Thr Gly Thr Gly Ser Leu Leu Gly Ala
        20              25              30
Ala Ala Ile Ala Gly His Ala Pro Arg Ala Trp Ala Asp Val Arg Ala
        35              40              45
Val Ala Ala Pro Ile Gly Ser Gly Ala His Val Pro Val Leu Val Val
    50              55              60
Gly Thr Gly Tyr Gly Gly Ser Val Ala Ala Leu Arg Leu Ala Glu Ala
65              70              75              80
Gly Thr Asp Val His Met Val Glu Met Gly Met Ala Trp Asp Thr Pro
            85              90              95
Gly Ala Asp Gly Lys Ile Phe Ala Asn Thr Thr Arg Pro Asp Asp Arg
            100             105             110
Ser Phe Trp Leu Arg Thr Arg Thr Lys Gln Pro Leu Ser Asn Phe Leu
        115             120             125
Gly Phe Pro Leu Asp Lys Asp Val Asn Arg Tyr Thr Gly Ile Leu Asp
    130             135             140
Ala Glu Glu Phe Gly Gly Ile Thr Val Tyr Gln Gly Arg Gly Val Gly
145             150             155             160
Gly Gly Ser Leu Val Asn Gly Gly Xaa Ala Val Thr Pro Arg Arg Glu
            165             170             175
Asn Phe Gly Ala Ile Leu Pro Thr Val Asn Ala Ala Glu Met Tyr Ser
            180             185             190
Thr Tyr Tyr Pro Arg Ala Asn Ser Gly Leu Gly Val Thr Thr Ile Asp
    195             200             205
Pro Ala Trp Phe Asp Ser Val Asp Cys Tyr Gln Tyr Ala Arg Val Gly
    210             215             220
Arg Lys His Ala Gln Arg Ser Gly Phe Pro Phe Leu Phe Xaa Pro Ala
225             230             235             240
Val Tyr Asp Trp Asp Tyr Met Lys Gln Glu Ala Ala Gly Thr Val Pro
            245             250             255
Lys Ser Ala Leu Asp Gly Glu Ile Leu Tyr Gly Asn Asn His Gly Lys
            260             265             270
Lys Ser Leu Gln Lys Thr Tyr Ile Asp Arg Ile Arg Ala Thr Gly Arg
    275             280             285
Val Thr Ile Ser Pro Leu His Lys Val Thr Thr Val Thr Pro Ala Pro
    290             295             300
Gly Gly Gly Tyr Thr Val Leu Ile Asp Gln Leu Asp Thr Gly Gly Arg
305             310             315             320
Thr Thr Ala Thr Lys Thr Val Thr Ala Asp Lys Val Phe Phe Ala Ala
            325             330             335
Gly Ser Val Gly Thr Ser Lys Leu Leu Val Gly Leu Lys Ala Thr Gly
            340             345             350
Ala Leu Pro Leu Leu Asn Asp Glu Ile Gly Arg Gly Trp Gly Asp Asn
        355             360             365
Gly Asn Val Met Cys Gly Arg Ala Asn His Leu Trp Asp Pro Thr Gly
    370             375             380
```

```
Lys Val Gln Ser Ser Ile Pro Thr Gly Gly Ile Asp Asn Trp Asp Ala
385                 390             395                 400
Gly Gly Ala Xaa Ala Glu Ile Ala Pro Leu Pro Thr Gly Ile Glu Thr
            405             410                 415
Trp Ala Ser Phe Tyr Leu Ser Ile Thr Lys Asn Pro His Arg Ala Arg
            420             425                 430
Phe Thr Trp Asn Ala Ala Ala Gly Lys Ala Glu Leu Asp Trp Arg Thr
        435             440             445
Ala Trp Lys Gln Pro Ser Ile Asp Ala Ala Lys Thr Ile Phe Asp Lys
    450             455             460
Ile Asn Gln Lys Glu Gly Thr Ile Tyr Arg Thr Asp Leu Phe Gly Val
465             470             475                 480
Tyr Lys Ile Trp Gly Asp His Leu Thr Tyr His Pro Leu Gly Gly Ala
            485             490                 495
Val Leu Asp Lys Ala Thr Asp Asn Tyr Gly Arg Leu His Gly Tyr Ser
            500             505             510
Gly Leu Tyr Val Ile Asp Gly Ala Leu Ile Pro Gly Asn Thr Ser Val
        515             520             525
Asn Pro Phe Val Thr Ile Thr Ala Leu Ala Glu Arg Asn Ile Glu Arg
    530             535             540
Ile Ile Ala Thr Asp Leu
545             550
```

<210> 41
<211> 502
<212> PRT
<213> Streptomyces roseosporus
<220>
<221> MISC_FEATURE
<222> (121)..(121)
<223> Xaa is Met, Phe, Leu, Val, Cys, Ile, Ala, Gln, Tyr, Lys, or Ser
<220>
<221> MISC_FEATURE
<222> (190)..(190)
<223> Xaa is Val, Met, Ile, Ala, Thr, Lys, Cys, Ser, Gly, Glu, Tyr, Pro, Asn, Gln, Trp or His
<221> MISC_FEATURE
<222> (356)..(356)
<223> Xaa is Phe, Trp, Ser, Thr, Lys, Ala, Asn, His or Asp, provided that when Xaa121 is Met, then Xaa356 is not Phe
<400> 41

```
Met Ala Ala Pro Ile Gly Ser Gly Ala His Val Pro Val Leu Val Val
1               5                   10                  15
Gly Thr Gly Tyr Gly Gly Ser Val Ala Ala Leu Arg Leu Ala Glu Ala
            20                  25                  30
Gly Thr Asp Val His Met Val Glu Met Gly Met Ala Trp Asp Thr Pro
        35                  40                  45
Gly Ala Asp Gly Lys Ile Phe Ala Asn Thr Thr Arg Pro Asp Asp Arg
    50                  55                  60
Ser Phe Trp Leu Arg Thr Arg Thr Lys Gln Pro Leu Ser Asn Phe Leu
65                  70                  75                  80
Gly Phe Pro Leu Asp Lys Asp Val Asn Arg Tyr Thr Gly Ile Leu Asp
                85                  90                  95
Ala Glu Glu Phe Gly Gly Ile Thr Val Tyr Gln Gly Arg Gly Val Gly
            100                 105                 110
Gly Gly Ser Leu Val Asn Gly Gly Xaa Ala Val Thr Pro Arg Arg Glu
        115                 120                 125
Asn Phe Gly Ala Ile Leu Pro Thr Val Asn Ala Ala Glu Met Tyr Ser
    130                 135                 140
Thr Tyr Tyr Pro Arg Ala Asn Ser Gly Leu Gly Val Thr Thr Ile Asp
145                 150                 155                 160
Pro Ala Trp Phe Asp Ser Val Asp Cys Tyr Gln Tyr Ala Arg Val Gly
                165                 170                 175
Arg Lys His Ala Gln Arg Ser Gly Phe Pro Phe Leu Phe Xaa Pro Ala
            180                 185                 190
Val Tyr Asp Trp Asp Tyr Met Lys Gln Glu Ala Ala Gly Thr Val Pro
        195                 200                 205
Lys Ser Ala Leu Asp Gly Glu Ile Leu Tyr Gly Asn Asn His Gly Lys
    210                 215                 220
```

```
Lys Ser Leu Gln Lys Thr Tyr Ile Asp Arg Ile Arg Ala Thr Gly Arg
225                 230                 235                 240
Val Thr Ile Ser Pro Leu His Lys Val Thr Thr Val Thr Pro Ala Pro
            245                 250                 255
Gly Gly Gly Tyr Thr Val Leu Ile Asp Gln Leu Asp Thr Gly Gly Arg
        260                 265                 270
Thr Thr Ala Thr Lys Thr Val Thr Ala Asp Lys Val Phe Phe Ala Ala
    275                 280                 285
Gly Ser Val Gly Thr Ser Lys Leu Leu Val Gly Leu Lys Ala Thr Gly
    290                 295                 300
Ala Leu Pro Leu Leu Asn Asp Glu Ile Gly Arg Gly Trp Gly Asp Asn
305                 310                 315                 320
Gly Asn Val Met Cys Gly Arg Ala Asn His Leu Trp Asp Pro Thr Gly
                325                 330                 335
Lys Val Gln Ser Ser Ile Pro Thr Gly Gly Ile Asp Asn Trp Asp Ala
        340                 345                 350
Gly Gly Ala Xaa Ala Glu Ile Ala Pro Leu Pro Thr Gly Ile Glu Thr
        355                 360                 365
Trp Ala Ser Phe Tyr Leu Ser Ile Thr Lys Asn Pro His Arg Ala Arg
    370                 375                 380
Phe Thr Trp Asn Ala Ala Ala Gly Lys Ala Glu Leu Asp Trp Arg Thr
385                 390                 395                 400
Ala Trp Lys Gln Pro Ser Ile Asp Ala Ala Lys Thr Ile Phe Asp Lys
            405                 410                 415
Ile Asn Gln Lys Glu Gly Thr Ile Tyr Arg Thr Asp Leu Phe Gly Val
        420                 425                 430
Tyr Lys Ile Trp Gly Asp His Leu Thr Tyr His Pro Leu Gly Gly Ala
        435                 440                 445
Val Leu Asp Lys Ala Thr Asp Asn Tyr Gly Arg Leu His Gly Tyr Ser
    450                 455                 460
Gly Leu Tyr Val Ile Asp Gly Ala Leu Ile Pro Gly Asn Thr Ser Val
465                 470                 475                 480
Asn Pro Phe Val Thr Ile Thr Ala Leu Ala Glu Arg Asn Ile Glu Arg
                485                 490                 495
Ile Ile Ala Thr Asp Leu
                500
```

<210> 42

<211> 547
<212> PRT
<213> Streptomyces sp.
<220>
<221> MISC_FEATURE
<222> (166)..(166)
<223> Xaa is Met, Phe, Leu, Val, Cys, Ile, Ala, Gln, Tyr, Lys, or Ser
<220>
<221> MISC_FEATURE
<222> (235)..(235)
<223> Xaa is Val, Met, Ile, Ala, Thr, Lys, Cys, Ser, Gly, Glu, Tyr, Pro, Asn, Gln, Trp or His
<221> MISC_FEATURE
<222> (401)..(401)
<223> Xaa is Phe, Trp, Ser, Thr, Lys, Ala, Asn, His or Asp, provided that when xaa166 is Met, then Xaa401 is not Phe
<400> 42

```
Met Gly Asp Thr Thr Val His Lys Gly Gly Thr Gln Gly Val Ser Arg
1               5               10              15
Arg Arg Phe Ile Thr Gly Thr Gly Ser Leu Leu Gly Gly Ala Ala Ile
            20              25              30
Ala Gly His Thr Ala Pro Ala Trp Ala Thr Val Arg Ala Ala Ala Ala
        35              40              45
Pro Ile Gly Ser Gly Ala Arg Val Pro Ala Leu Val Ile Gly Thr Gly
    50              55              60
Tyr Gly Gly Ser Val Ala Ala Leu Arg Leu Ala Gln Ala Gly Thr Asp
65              70              75              80
Val His Met Val Glu Met Gly Met Ala Trp Asp Thr Pro Gly Ala Asp
                85              90              95
Gly Lys Ile Phe Ala Asn Thr Thr Arg Pro Asp Asp Arg Ser Phe Trp
            100             105             110
```

```
Leu Arg Thr Arg Thr Lys Gln Pro Leu Ser Asn Phe Leu Gly Phe Pro
    115                 120                 125
Ile Asp Arg Ser Val Asn Arg Tyr Thr Gly Ile Leu Asp Ala Glu Glu
    130                 135                 140
Phe Ala Gly Ile Thr Val Tyr Gln Gly Arg Gly Val Gly Gly Gly Ser
145                 150                 155                 160
Leu Val Asn Gly Gly Xaa Ala Val Thr Pro Arg Arg Glu Asn Phe Gly
                165                 170                 175
Ala Ile Leu Pro Thr Val Asn Ala Gln Glu Met Tyr Ser Thr Tyr Tyr
            180                 185                 190
Pro Arg Ala Asn Ser Gly Leu Gly Val Thr Thr Ile Asp Pro Ala Trp
        195                 200                 205
Phe Asp Ser Val Asp Cys Tyr Gln Tyr Ala Arg Val Gly Arg Lys His
    210                 215                 220
Ala Gln Arg Ser Gly Phe Pro Phe Leu Phe Xaa Pro Ala Val Tyr Asp
225                 230                 235                 240
Trp Asp Tyr Met Lys Gln Glu Ala Ala Gly Thr Val Pro Arg Ser Ala
                245                 250                 255
Leu Asp Ala Glu Ile Leu Tyr Gly Asn Asn Tyr Gly Lys Lys Ser Leu
            260                 265                 270
Gln Lys Thr Tyr Ile Asp Arg Ile Arg Ala Thr Gly Arg Val Thr Ile
        275                 280                 285
Ser Pro Leu His Arg Val Thr Arg Val Thr Pro Ala Pro Gly Gly Gly
    290                 295                 300
Tyr Thr Val Leu Ile Asp Gln Leu Asn Thr Ala Gly Gln Thr Thr Ala
305                 310                 315                 320
Thr Lys Thr Val Thr Ala Asp Lys Val Phe Phe Ala Ala Gly Ser Val
                325                 330                 335
Gly Thr Ser Lys Leu Leu Val Gly Leu Lys Ala Thr Gly Ala Leu Pro
            340                 345                 350
Leu Leu Asn Asp Glu Ile Gly Lys Gly Trp Gly Asp Asn Gly Asn Val
        355                 360                 365
Met Cys Gly Arg Ala Asn His Leu Trp Asp Pro Thr Gly Lys Val Gln
    370                 375                 380
Ser Ser Ile Pro Thr Gly Gly Ile Asp Asn Trp Asp Ala Gly Gly Ala
385                 390                 395                 400
Xaa Ala Glu Val Ala Pro Leu Pro Thr Gly Ile Glu Thr Trp Ala Ser
                405                 410                 415
Phe Tyr Leu Ser Ile Thr Lys Asn Pro His Arg Ala Arg Phe Thr Trp
            420                 425                 430
Asn Ala Ala Ala Gly Lys Ala Glu Leu Asp Trp Gln Thr Ala Trp Lys
        435                 440                 445
Gln Pro Ser Ile Asp Ala Ala Lys Thr Ile Phe Asp Lys Ile Asn Gln
    450                 455                 460
Lys Glu Gly Thr Ile Tyr Arg Thr Asp Leu Phe Gly Val His Lys Ile
465                 470                 475                 480
Trp Gly Asp His Leu Thr Tyr His Pro Leu Gly Gly Ala Val Leu Asp
                485                 490                 495
Lys Ala Thr Asp Asn Tyr Gly Arg Leu His Gly Tyr Thr Gly Leu Tyr
            500                 505                 510
Val Ile Asp Gly Ala Leu Ile Pro Gly Asn Thr Ser Val Asn Pro Phe
        515                 520                 525
Val Thr Ile Thr Ala Leu Ala Glu Arg Asn Ile Glu Arg Ile Ile Ala
530                 535                 540
Thr Asp Leu
545
```

<210> 43

<211> 547

<212> PRT

<213> Streptomyces griseus

<220>

<221> MISC_FEATURE

<222> (166)..(166)

<223> Xaa is Met, Phe, Leu, Val, Cys, Ile, Ala, Gln, Tyr, Lys, or Ser

<220>

<221> MISC_FEATURE

<222> (235)..(235)
<223> Xaa is Val, Met, Ile, Ala, Thr, Lys, Cys, Ser, Gly, Glu, Tyr, Pro, Asn, Gln, Trp or His
<221> MISC_FEATURE
<222> (401)..(401)
<223> Xaa is Phe, Trp, Ser, Thr, Lys, Ala, Asn, His or Asp, provided that when Xaa166 is Met, then Xaa401 is not Phe
<400> 43

```
Met Gly Asp Thr Thr Val His Lys Gly Gly Thr Gln Gly Val Ser Arg
1               5                   10                  15
Arg Arg Phe Ile Thr Gly Thr Gly Ser Leu Leu Gly Gly Ala Ala Ile
            20              25                  30
Ala Gly His Thr Ala Pro Ala Trp Ala Thr Val Arg Ala Ala Ala Ala
        35              40                  45
Pro Ile Gly Ser Gly Ala Arg Val Pro Ala Leu Val Ile Gly Thr Gly
    50              55                  60
Tyr Gly Gly Ser Val Ala Ala Leu Arg Leu Ala Gln Ala Gly Thr Asp
65              70                  75                  80
Val His Met Val Glu Met Gly Met Ala Trp Asp Thr Pro Gly Ala Asp
            85              90                  95
Gly Lys Ile Phe Ala Asn Thr Thr Arg Pro Asp Asp Arg Ser Phe Trp
        100                 105                 110
Leu Arg Thr Arg Thr Lys Gln Pro Leu Ser Asn Phe Leu Gly Phe Pro
        115                 120                 125
Ile Asp Arg Ser Val Asn Arg Tyr Thr Gly Ile Leu Asp Ala Glu Glu
    130                 135                 140
Phe Ala Gly Ile Thr Val Tyr Gln Gly Arg Gly Val Gly Gly Gly Ser
145                 150                 155                 160
Leu Val Asn Gly Gly Xaa Ala Val Thr Pro Arg Arg Glu Asn Phe Gly
                165                 170                 175
Ala Ile Leu Pro Thr Val Asn Ala Gln Glu Met Tyr Ser Thr Tyr Tyr
        180                 185                 190
Pro Arg Ala Asn Ser Gly Leu Gly Val Thr Thr Ile Asp Pro Ala Trp
        195                 200                 205
Phe Asp Ser Val Asp Cys Tyr Gln Tyr Ala Arg Val Gly Arg Lys His
    210                 215                 220
Ala Gln Arg Ser Gly Phe Pro Phe Leu Phe Xaa Pro Ala Val Tyr Asp
225                 230                 235                 240
Trp Asp Tyr Met Lys Gln Glu Ala Ala Gly Thr Val Pro Arg Ser Ala
            245                 250                 255
Leu Asp Ala Glu Ile Leu Tyr Gly Asn Asn Tyr Gly Lys Lys Ser Leu
            260                 265                 270
Gln Lys Thr Tyr Ile Asp Arg Ile Arg Ala Thr Gly Arg Val Thr Ile
        275                 280                 285
Ser Pro Leu His Lys Val Thr Arg Val Thr Pro Ala Pro Gly Gly Gly
    290                 295                 300
Tyr Thr Val Leu Ile Asp Gln Leu Asn Thr Ala Gly Gln Thr Thr Ala
305                 310                 315                 320
Thr Lys Thr Val Thr Ala Asp Lys Val Phe Phe Ala Ala Gly Ser Val
            325                 330                 335
Gly Thr Ser Lys Leu Leu Val Gly Leu Lys Ala Thr Gly Ala Leu Pro
        340                 345                 350
Leu Leu Asn Asp Glu Ile Gly Lys Gly Trp Gly Asp Asn Gly Asn Val
        355                 360                 365
Met Cys Gly Arg Ala Asn His Leu Trp Asp Pro Thr Gly Lys Val Gln
    370                 375                 380
Ser Ser Ile Pro Thr Gly Gly Ile Asp Asn Trp Asp Ala Gly Gly Ala
385                 390                 395                 400
Xaa Ala Glu Val Ala Pro Leu Pro Thr Gly Ile Glu Thr Trp Ala Ser
                405                 410                 415
Phe Tyr Leu Ser Ile Thr Lys Asn Pro His Arg Ala Arg Phe Thr Trp
        420                 425                 430
Asn Ala Ala Ala Gly Lys Ala Glu Leu Asp Trp Gln Thr Ala Trp Lys
        435                 440                 445
Gln Pro Ser Ile Asp Ala Ala Lys Thr Ile Phe Asp Lys Ile Asn Gln
    450                 455                 460
Lys Glu Gly Thr Ile Tyr Arg Thr Asp Leu Phe Gly Val His Lys Ile
465                 470                 475                 480
Trp Gly Asp His Leu Thr Tyr His Pro Leu Gly Gly Ala Val Leu Asp
                485                 490                 495
```

```
Lys Ala Thr Asp Asn Tyr Gly Arg Leu His Gly Tyr Thr Gly Leu Tyr
              500                     505                     510
Val Ile Asp Gly Ala Leu Ile Pro Gly Asn Thr Ser Val Asn Pro Phe
              515                     520                     525
Val Thr Ile Thr Ala Leu Ala Glu Arg Asn Ile Glu Arg Ile Ile Ala
              530                     535                     540
Thr Asp Leu
545
```

<210> 44
<211> 546
<212> PRT
<213> Streptomyces sp.
<220>
<221> MISC_FEATURE
<222> (165)..(165)
<223> Xaa is Met, Phe, Leu, Val, Cys, Ile, Ala, Gln, Tyr, Lys, or Ser
<220>
<221> MISC_FEATURE
<222> (234)..(234)
<223> Xaa is Val, Met, Ile, Ala, Thr, Lys, Cys, Ser, Gly, Glu, Tyr, Pro, Asn,
Gln, Trp or His
<221> MISC_FEATURE
<222> (400)..(400)
<223> Xaa is Phe, Trp, Ser, Thr, Lys, Ala, Asn, His or Asp, provided that when
Xaa165 is Met, then Xaa400 is not Phe
<400> 44

```
Met Pro Asp Lys Gly Ser Lys Gly Phe Ser Arg Arg Gly Phe Ile Ala
1               5                   10                  15
Arg Thr Ser Ser Ile Leu Gly Ala Val Ala Val Ala Gly Gly Ala Ala
            20                  25                  30
Ala Thr Thr Ala Arg Ala Ala Val Ala Thr Ser Ala Thr Ala Ala Pro
        35                  40                  45
Ile Asp Ser Gly Ala His Val Pro Val Leu Ile Ile Gly Thr Gly Tyr
    50                  55                  60
Gly Gly Ser Val Ala Ala Leu Arg Leu Ala Gln Ala Gly Val Asp Val
65                  70                  75                  80
His Met Ile Glu Met Gly Met Ala Trp Asp Thr Pro Gly Ser Asp Gly
                85                  90                  95
Lys Ile Phe Ala Asn Thr Thr Arg Pro Asp Tyr Arg Ser Phe Trp Leu
        100                 105                 110
Arg Thr Arg Thr Lys Ala Pro Ile Ser Asn Phe Leu Gly Phe Pro Ile
        115                 120                 125
Asp Lys Asp Val Ala Arg Tyr Thr Gly Ile Leu Asp Ala Glu Glu Phe
    130                 135                 140
Asn Gly Ile Thr Val Tyr Gln Gly Arg Gly Val Gly Gly Gly Ser Leu
145                 150                 155                 160
Val Asn Gly Gly Xaa Ala Val Thr Pro Lys Arg Glu Arg Phe Gly Ala
                165                 170                 175
Val Leu Pro Ser Val Asp Ala Asp Glu Met Tyr Asp Val Tyr Tyr Pro
            180                 185                 190
Arg Ala Asn Ala Gly Leu Gly Val Thr Asn Val Asp Gln Ala Trp Trp
        195                 200                 205
Glu Thr Ala Pro Cys Tyr Gln Tyr Ala Arg Val Gly Arg Lys His Ala
    210                 215                 220
Gln Arg Ser Gly Phe Pro Phe Val Phe Xaa Pro Asn Val Tyr Asp Trp
225                 230                 235                 240
Glu Tyr Met Lys Gln Glu Glu Ala Gly Thr Val Pro Arg Ser Ser Leu
            245                 250                 255
Asp Gly Glu Val Leu Tyr Gly Asn Asn Tyr Gly Lys Lys Ser Val Gln
        260                 265                 270
Lys Thr Tyr Ile Ala Gln Ala Lys Ala Thr Gly Arg Val Ser Ile Ser
        275                 280                 285
Pro Gln His Lys Val Thr Ser Val Ala Pro Ala Thr Gly Gly Gly Tyr
    290                 295                 300
Thr Val Ser Ile Asp Gln Ile Asn Thr Thr Gly Asp Thr Thr Ala Thr
305                 310                 315                 320
Lys Thr Val Thr Ala Asp Arg Val Phe Phe Ala Ala Gly Ser Val Gly
            325                 330                 335
```

```
Thr Ser Lys Leu Leu Val Arg Leu Lys Ala Thr Gly Arg Leu Pro Leu
            340                 345                 350
Leu Asn Asp Glu Val Gly Lys Gly Trp Gly Asp Asn Gly Asn Val Met
        355                 360                 365
Cys Gly Arg Ala Asn His Ile Trp Asp Ala Thr Gly Lys Leu Gln Ala
370                 375                 380
Ser Met Pro Thr Ala Gly Ile Asp Asn Trp Asp Ala Gly Gly Ala Xaa
385                 390                 395                 400
Ala Glu Val Ala Pro Leu Pro Thr Gly Ile Glu Thr Tyr Ala Ser Leu
                405                 410                 415
Tyr Leu Ser Ile Thr Lys Asn Pro His Arg Ala Glu Phe Ser Trp Asn
            420                 425                 430
Ala Ala Thr Gly Asn Val Asp Leu Asn Trp Gln Arg Ala Trp Lys Gln
        435                 440                 445
Pro Ala Ile Asp Met Ala Lys Ser Ile Phe Asp Lys Ile Asn Ser Lys
        450                 455                 460
Glu Gly Thr Ile Tyr Arg Ser Asp Leu Phe Gly Gly Asn Lys Val Trp
465                 470                 475                 480
Gly Asp His Leu Thr Tyr His Pro Leu Gly Gly Ala Val Leu Asp Lys
            485                 490                 495
Ala Thr Asp Asn Tyr Gly Arg Leu His Gly Tyr Ser Gly Leu Tyr Val
        500                 505                 510
Ile Asp Gly Ser Leu Ile Pro Gly Asn Thr Ser Val Asn Pro Phe Val
        515                 520                 525
Thr Ile Thr Ala Leu Ala Glu Arg Asn Ile Glu Lys Ile Ile Ala Thr
    530                 535                 540
Asp Leu
545
```

<210> 45
<211> 547
<212> PRT
<213> Streptomyces pristinaespiralis
<220>
<221> MISC_FEATURE
<222> (166)..(166)
<223> Xaa is Met, Phe, Leu, Val, Cys, Ile, Ala, Gln, Tyr, Lys, or Ser
<220>
<221> MISC_FEATURE
<222> (235)..(235)
<223> Xaa is Val, Met, Ile, Ala, Thr, Lys, Cys, Ser, Gly, Glu, Tyr, Pro, Asn, Gln, Trp or His
<221> MISC_FEATURE
<222> (401)..(401)
<223> Xaa is Phe, Trp, Ser, Thr, Lys, Ala, Asn, His or Asp, provided that when Xaa166 is Met, then Xaa401 is not Phe
<400> 45

```
Met Asn Ala Ala Pro Leu Pro Arg Pro Ser Gly Arg Asp Pro Ser Arg
1               5               10              15
Arg Gln Leu Leu Ala Gly Thr Gly Ser Ile Leu Gly Ala Val Ile Leu
        20              25              30
Ala Gly His Gly Pro Ala Ala Ala Arg Ala Lys Ala Ala Pro Ala Ala
        35              40              45
Ala Ile Pro Asp Gly Ala His Val Pro Ala Leu Val Ile Gly Thr Gly
    50              55              60
Tyr Gly Gly Ser Val Ala Ala Leu Arg Leu Ala Arg Ala Gly Val Asp
65              70              75              80
Val His Met Ile Glu Met Gly Met Ser Trp Asp Thr Pro Gly Pro Asp
            85              90              95
Gly Lys Val Phe Ala Asn Thr Thr Arg Pro Asp His Arg Ser Phe Trp
            100             105             110
Leu Arg Thr Arg Thr Lys Gln Pro Leu Ser Asp Phe Leu Gly Phe Pro
        115             120             125
Leu Asp Lys Asp Val Pro Arg Tyr Thr Gly Ile Leu Asp Ala Glu Glu
    130             135             140
Phe Gly Gly Ile Thr Val Tyr Gln Gly Arg Gly Val Gly Gly Gly Ser
145             150             155             160
Leu Val Asn Gly Gly Xaa Ala Val Thr Pro Arg Arg Glu Asn Phe Gly
            165             170             175
```

```
Ala Val Leu Pro Ser Val Asn Ala Asp Glu Met Tyr Gly Ile Tyr Tyr
            180                   185                   190
Pro Arg Ala Asn Ala Ala Leu Gly Val Gly Val Val Asp Gln Gly Trp
            195               200               205
Trp Glu Ser Ala Ala Cys Tyr Gln Tyr Ala Arg Val Gly Arg Lys His
    210               215                   220
Ala Glu Arg Ser Gly Phe Pro Phe Val Leu Xaa Pro Gly Val Tyr Asp
225               230                   235                   240
Trp Asp Tyr Leu Glu Gln Glu Ala Ala Gly Thr Val Pro Ala Ser Ala
                245               250               255
Leu Glu Gly Glu Val Leu Phe Gly Asn Asn His Gly Lys Lys Ser Leu
            260               265               270
Pro Lys Thr Tyr Leu Ala Arg Ala Ala Ala Thr Gly Arg Val Val Ile
            275               280               285
Ser Pro Leu His Lys Val Thr Ser Val Ala Pro Ala Gly Gly Gly Gly
    290               295               300
Tyr Thr Val Val Met Glu Gln Leu Asn Thr Gly Gly Asp Val Thr Ala
305               310               315               320
Val Lys Ala Val Thr Ala Asp Arg Val Phe Phe Ala Ala Gly Ser Val
            325               330               335
Gly Thr Ser Lys Leu Leu Thr Arg Leu Lys Ala Thr Gly Val Leu Pro
            340               345               350
Gly Leu Asn Gly Glu Ile Gly Lys Gly Trp Gly Asp Asn Gly Asn Val
            355               360               365
Met Cys Gly Arg Ala Asn His Met Trp Asp Ala Thr Gly Arg Leu Gln
    370               375               380
Ala Ser Met Pro Thr Ala Gly Ile Asp Asn Trp Gln Ala Gly Gly Ala
385               390               395               400
Xaa Ala Glu Val Ala Pro Leu Pro Thr Gly Ile Glu Thr Tyr Ala Ser
            405               410               415
Phe Tyr Leu Ser Ile Thr Arg Asn Pro His Arg Ala Ala Phe Ser Trp
            420               425               430
Asp Ala Ala Ala Gly Lys Val Val Leu Asp Trp Arg Thr Ala Trp Lys
    435               440               445
Gln Pro Ser Ile Asp Ala Ala Arg Thr Ile Phe Asp Arg Ile Asn Ala
    450               455               460
Lys Glu Gly Thr Ile Tyr Arg Thr Asp Leu Phe Gly Ala Tyr Lys Ile
465               470               475               480
Trp Gly Asp His Leu Thr Tyr His Pro Leu Gly Gly Ala Val Leu Asn
            485               490               495
Arg Ala Thr Asp Asn Tyr Gly Arg Leu His Gly His Pro Gly Leu Tyr
            500               505               510
Val Ile Asp Gly Ser Leu Ile Pro Gly Asn Thr Ser Val Asn Pro Phe
            515               520               525
Val Thr Ile Thr Ala Leu Ala Glu Arg Asn Ile Glu Lys Ile Ile Ala
    530               535               540
Thr Asp Leu
545
```

<210> 46

<211> 543

<212> PRT

<213> Actinosynnema mirum

<220>

<221> MISC_FEATURE

<222> (163)..(163)

<223> Xaa is Met, Phe, Leu, Val, Cys, Ile, Ala, Gln, Tyr, Lys, or Ser

<220>

<221> MISC_FEATURE

<222> (232)..(232)

<223> Xaa is Val, Met, Ile, Ala, Thr, Lys, Cys, Ser, Gly, Glu, Tyr, Pro, Asn,
Gln, Trp or His

<221> MISC_FEATURE

<222> (397)..(397)

<223> Xaa is Phe, Trp, Ser, Thr, Lys, Ala, Asn, His or Asp, provided that when
Xaa163 is Met, then Xaa397 is not Phe

<400> 46

Met Thr Arg Ser Val Ser Arg Arg Ser Phe Leu Ala Gly Ala Gly Thr
1               5                   10                  15

EP 2 748 312 B1

```
Ala Leu Gly Ala Thr Ala Phe Ser Gly Gly Ala Ser Ala Ser Gly Ser
        20              25              30
Thr Ala Pro Gly Ala Pro Ala Ser Gly Arg Ser Pro Ala Gly Ile Pro
        35              40              45
Asp Gly Ala Arg Val Pro Ala Leu Val Val Gly Ser Gly Tyr Gly Gly
    50              55              60
Ala Val Ala Ala Leu Arg Leu Ala Gln Ala Gly Val Pro Val His Val
65              70              75              80
Val Glu Lys Gly Arg Ser Trp Asp Glu Pro Gly Trp Asp Gly Lys Val
            85              90              95
Phe Ala Asn Met Leu Asn Pro Asp Glu Arg Ser Tyr Trp Leu Arg Thr
            100             105             110
Trp Thr Lys Gln Pro Leu Ser Asn Phe Leu Gly Leu Pro Val Asp Arg
        115             120             125
Ala Val Pro Arg Arg Thr Gly Ile Leu Asp Ala Glu Glu Phe Ala Gly
    130             135             140
Ile Thr Val Tyr Gln Gly Arg Gly Val Gly Gly Ser Leu Val Asn
145             150             155             160
Gly Gly Xaa Ala Val Thr Pro Arg Arg Glu Arg Phe Ala Ala Val Leu
            165             170             175
Pro Gly Val Asp Pro Glu Glu Met Tyr Ser Thr Tyr Tyr Pro Leu Ala
            180             185             190
Asn Ala Glu Leu Gly Thr Gly Leu Val Asp Pro Asp Trp Trp Glu Gln
            195             200             205
Ala Glu Cys Tyr Arg Tyr Ala Arg Val Gly Arg Ala Gln Ala Gln Arg
    210             215             220
Ser Gly Phe Pro Phe Glu Leu Xaa Pro Gly Val Tyr Asp Trp Ala His
225             230             235             240
Leu Glu Arg Glu Glu Ala Gly Thr Ala Pro Arg Ser Ala Leu Ala Ala
            245             250             255
Glu Val Ile Tyr Gly Asn Asn His Gly Lys Leu Ser Leu Pro Arg Thr
            260             265             270
Tyr Leu Ala Arg Ala Leu Ala Thr Gly Arg Val Thr Ile Ser Ala Leu
        275             280             285
His Glu Val Thr Ser Val Arg Ala Val Gly Gly Gly Tyr Glu Ala Leu
        290             295             300
Leu Asp Val Leu Asp Thr Asn Gly Arg Val Thr Ser Thr Lys Arg Val
305             310             315             320
Glu Ala Glu Arg Val Phe Phe Ala Ala Gly Ser Val Gly Thr Ser Lys
            325             330             335
Leu Leu Thr Arg Leu Arg Asp Thr Gly Ala Leu Pro Ala Leu Ser Pro
            340             345             350
Glu Val Gly Leu Gly Trp Gly Glu Asn Gly Asn Val Met Val Gly Arg
        355             360             365
Ala Asn Lys Ala Ser Asp Pro Thr Gly Ala Leu Gln Ser Cys Ile Pro
    370             375             380
Thr Gly Gly Ile Asp Asn Trp Ala Ala Gly Gly Ala Xaa Ala Glu Val
385             390             395             400
Ala Pro Leu Pro Thr Gly Val Glu Thr Phe Thr Ser Phe Tyr Leu Ala
            405             410             415
Ile Thr Ala Asn Pro Arg Arg Gly Arg Phe Thr Trp Asn Pro Glu Ala
            420             425             430
Gly Arg Val Glu Leu Asp Trp Arg Gln Glu Trp Lys Gln Pro Ser Val
        435             440             445
Asp Met Ala Arg Thr Ile Phe Asp Arg Ile Asn Ser Val Glu Gly Thr
    450             455             460
Val Tyr Arg Ala Asp Leu Phe Gly Ala Gly Lys Val Trp Gly Asp Gly
465             470             475             480
Leu Thr Tyr His Pro Leu Gly Gly Val Val Leu Gly Arg Ala Thr Asp
            485             490             495
Gly His Gly Arg Leu Ala Gly Tyr Arg Gly Leu Tyr Val Val Asp Gly
            500             505             510
Ser Leu Ile Pro Gly Asn Thr Ser Val Asn Pro Phe Val Thr Ile Thr
        515             520             525
Ala Leu Ala Glu Arg Asn Leu Ala Arg Ile Val Ala Arg Asp Leu
    530             535             540
```

<210> 47
<211> 528

117

<212> PRT
<213> chryseobacterium gleum
<220>
<221> MISC_FEATURE
<222> (142)..(142)
<223> Xaa is Met, Phe, Leu, Val, Cys, Ile, Ala, Gln, Tyr, Lys, or Ser
<220>
<221> MISC_FEATURE
<222> (211)..(211)
<223> Xaa is Val, Met, Ile, Ala, Thr, Lys, Cys, Ser, Gly, Glu, Tyr, Pro, Asn, Gln, Trp or His
<221> MISC_FEATURE
<222> (380)..(380)
<223> Xaa is Phe, Trp, Ser, Thr, Lys, Ala, Asn, His or Asp, provided that when Xaa142 is Met, then Xaa380 is not Phe
<400> 47

```
Met Asp Arg Lys Lys Phe Ile Arg Thr Ser Ala Leu Ala Ile Ser Gly
1               5                   10                  15
Phe Tyr Phe Leu Gln Ser Gly Leu Leu His Ala Thr Asn Arg Lys Asn
            20              25                  30
Ser Leu Glu Lys Glu Asn Ser Asp Ala Pro Ile Val Ile Ile Gly Ser
        35              40                  45
Gly Tyr Gly Gly Ala Val Ser Ala Leu Arg Leu Cys Glu Ala Gly Lys
    50              55                  60
Lys Val Val Met Leu Glu Met Gly Leu Asn Trp Glu Lys Ala Gly Ile
65              70                  75                      80
Pro Phe Ser Asn Leu Leu Lys Pro Gly Lys Ser Ser Ala Trp Leu Lys
            85                  90                      95
Lys Lys Ser Ile Ala Pro Phe Met Asn Ile Phe Ser Leu Thr Pro Phe
            100                 105                 110
Thr Gly Thr Leu Asp Arg Leu Asp Phe Lys His Ile Asn Ile Trp Val
        115                 120                 125
Gly Arg Gly Val Gly Gly Gly Ser Leu Val Asn Gly Gly Xaa Ala Val
    130                 135                 140
Thr Pro Lys Glu Ser Tyr Phe Arg Glu Val Phe Pro Asp Leu Asp Ala
145             150                 155                     160
Glu Arg Phe Tyr Ser His Tyr Phe Pro Leu Val Arg Glu Glu Leu Lys
            165                 170                 175
Val Asn Val Ile Asp Glu Gln Phe Leu Lys Asp Cys Pro Tyr Tyr Gln
            180                 185                 190
Phe Thr Arg Val Gly Glu Lys Glu Ala His Lys Ala Gly Phe Lys Thr
        195                 200                 205
Ile Arg Xaa Pro Asn Val Tyr Asp Phe Lys Tyr Met Glu Lys Glu Phe
    210                 215                 220
Arg Asn Glu Val Pro Arg Ser Ala Leu Asn Thr Glu Val Ile Tyr Gly
225                 230                 235                 240
Asn Asn Tyr Gly Lys Asn Ser Leu Asp Lys Thr Tyr Leu Arg Lys Ala
            245                 250                 255
Leu Glu Thr Gly Asn Leu Glu Ile Leu Asp Leu His Arg Val Gln Thr
            260                 265                 270
Val Lys Leu Asn Asp Asp Lys Ser Tyr Thr Leu His Val Arg Gln Ile
    275                 280                 285
Asp Thr Ser Gly Ser Val Ile Ala Asp Lys Val Phe Asn Cys Lys Lys
    290                 295                 300
Leu Ile Leu Ser Ala Gly Thr Met Gly Thr Leu Gln Ile Leu Leu Gln
305                 310                 315                 320
Ser Asn Ala Glu Asn Gly Phe Pro Ile His Glu Lys Ile Gly Lys Asn
            325                 330                 335
Trp Gly Asn Asn Gly Asn Phe Met Thr Gly Arg Asn Trp Val Lys Pro
            340                 345                 350
Leu Ser Gly Gly Thr Gly Ala Lys Gln Ser Thr Ile Pro Val Gly Gly
    355                 360                 365
Ile Asp Asn Trp Asp Asp Pro Glu His Gln Phe Xaa Thr Glu Ile Ala
    370                 375                 380
Pro Leu Pro Met Gly Met Asp Val Ala Thr Ala Leu Tyr Leu Leu Ile
385             390                 395                     400
Asn Arg Val Asp Lys Lys Gly Glu Val Thr Tyr Asn Lys Ala Ser Gln
            405                 410                 415
```

```
Ser Leu Thr Leu Asn Trp Asp Glu Ser Asn Thr Ala Lys Met Lys Glu
            420                 425                 430
Asn Ala Gln Tyr Phe Ile Arg Lys Met Asn Lys Ala Asn Gly Gly Thr
        435                 440                 445
Arg Ser His Leu Leu Phe Asn Asn Gly Phe Gly Ala Glu Ile Cys Tyr
    450                 455                 460
His Pro Leu Gly Gly Cys Val Leu Gly Glu Ala Thr Asn Glu Tyr Gly
465             470                 475                     480
Lys Leu Arg Asp His Glu Asn Leu Tyr Val Leu Asp Gly Ser Leu Ile
            485                 490                 495
Pro Gly Thr Ile Gly Val Asn Pro Phe Val Thr Ile Thr Ala Ile Ala
    500                 505                 510
Glu Tyr Cys Ile Glu Asn Leu Ile Arg Gln Asn Glu Phe Asn Leu Gly
    515                 520                 525
```

EP 2 748 312 B1

<210> 48
<211> 525
<212> PRT
<213> Weeksella virosa
<220>
<221> MISC_FEATURE
<222> (139)..(139)
<223> Xaa is Met, Phe, Leu, Val, Cys, Ile, Ala, Gln, Tyr, Lys, or Ser
<220>
<221> MISC_FEATURE
<222> (208)..(208)
<223> Xaa is Val, Met, Ile, Ala, Thr, Lys, Cys, Ser, Gly, Glu, Tyr, Pro, Asn, Gln, Trp or His
<221> MISC_FEATURE
<222> (377)..(377)
<223> Xaa is Phe, Trp, Ser, Thr, Lys, Ala, Asn, His or Asp, provided that when Xaa139 is Met, then Xaa377 is not Phe
<400> 48

```
Met Asn Arg Lys Ser Phe Ile Arg Lys Thr Ser Leu Gly Leu Gly Gly
1               5                   10                  15
Leu Phe Leu Tyr Lys Pro Met Ser Leu Phe Ser Lys Thr Lys Pro Lys
            20                  25                  30
Lys Glu Pro Ile Glu Lys Pro Ile Ile Ile Ile Gly Ser Gly Tyr Gly
            35                  40                  45
Gly Ala Val Ala Ala Leu Arg Leu Cys Glu Ala Gly Lys Lys Val Cys
    50                  55                  60
Leu Leu Glu Met Gly Leu Asn Trp Glu Lys Ser Gly Glu Lys Phe Ser
65                  70                  75                  80
Pro Met Thr His Pro Gly Lys Ser Ala Ala Trp Leu Arg Lys Lys Thr
                85                  90                  95
Ile Ala Pro Phe Phe Asn Ile Phe Pro Leu Lys Pro Phe Thr Gly Thr
            100                 105                 110
Leu Asp Arg Leu Asp Tyr Lys Asn Ile Lys Ile Trp Val Gly Arg Gly
            115                 120                 125
Val Gly Gly Gly Ser Leu Val Asn Gly Gly Xaa Ala Val Leu Pro Lys
    130                 135                 140
Lys Asn Tyr Phe Lys Glu Ile Phe Pro Thr Leu Asp Val Asp Leu Phe
145                 150                 155                 160
Tyr Asn Lys Tyr Phe Pro Leu Ala Gln Gln Glu Leu Lys Val Asn Val
                165                 170                 175
Ala Asp Glu Glu Phe Leu Gln Ser Cys Ser Tyr Tyr Lys Phe Asn Lys
            180                 185                 190
Val Gly Glu Lys Glu Ala Gln Lys Ala Gly Tyr Lys Thr Ile Arg Xaa
    195                 200                 205
Pro Asn Val Tyr Asn Phe Lys Tyr Met Glu Ala Glu Tyr Glu Asn Lys
    210                 215                 220
Val Pro Arg Ser Ala Leu Ala Gly Glu Val Ile Tyr Gly Asn Asn His
225                 230                 235                 240
Gly Lys Tyr Ser Leu Asp Lys Thr Tyr Leu Lys Lys Ala Asp Ala Thr
                245                 250                 255
Gly Asn Leu Glu Ile Leu Asp Leu His Gln Val Lys Ser Ile Ala Leu
            260                 265                 270
Asn Ser Asp His Ser Tyr Thr Leu Ser Val Asp Gln Ile Asn Thr Ser
            275                 280                 285
```

```
Gly Glu Ile Val Gln Val Lys Glu Met Arg Cys Gln Lys Leu Ile Leu
    290                 295                 300
Ala Ala Gly Thr Met Gly Ser Leu Glu Leu Leu Leu Arg Ser Gln Ala
305                 310                 315                 320
Lys Asn Gln Leu Pro Leu Asp Glu His Ile Gly Lys Met Trp Gly Asn
            325                 330                 335
Asn Gly Asn Phe Met Thr Gly Arg Asn Trp Val Lys Ala Phe Ser Gly
            340                 345                 350
Gly Asn Gly Tyr Leu His Ser Thr Ile Pro Val Gly Gly Ile Asp Asn
            355                 360                 365
Trp Asp Asp Pro Lys Tyr Pro Phe Xaa Ala Glu Ile Ala Pro Leu Pro
    370                 375                 380
Met Gly Met Asn Val Ala Thr Ser Leu Tyr Leu Ile Ile Asn Lys Leu
385                 390                 395                 400
Asp Lys Tyr Gly Glu Val Thr Tyr His Pro Thr Glu Asp Lys Leu Asp
            405                 410                 415
Leu Lys Trp Asp Leu Ser His Thr Lys Lys Met Lys Glu Asn Ala Arg
            420                 425                 430
His Phe Ile Lys Lys Met Asn Arg Thr Asn Gly Gly Thr Arg Ala His
        435                 440                 445
Phe Leu Phe His Asn Gly Phe Gly His Asp Ile Cys Tyr His Pro Leu
    450                 455                 460
Gly Gly Ile Val Leu Glu Lys Ala Thr Asn Pro Tyr Gly Lys Leu Asn
465                 470                 475                 480
Leu His Lys Asn Leu Phe Val Leu Asp Gly Ser Leu Ile Pro Gly Ser
            485                 490                 495
Ile Gly Val Asn Pro Phe Leu Thr Ile Thr Ala Leu Val Glu Tyr Cys
            500                 505                 510
Ile Glu His Leu Leu Gln Ser Lys Glu Phe Glu Thr Val
            515                 520                 525
```

<210> 49
<211> 28
<212> DNA
<213> Artificial Sequence
<220>
<223> primer for point mutation
<400> 49
ggttaacggt ggcgcggcgg tggaaccg          28
<210> 50
<211> 28
<212> DNA
<213> Artificial Sequence
<220>
<223> primer for point mutation
<400> 50
ggttaacggt ggctgcgcgg tggaaccg          28
<210> 51
<211> 28
<212> DNA
<213> Artificial Sequence
<220>
<223> primer for point mutation
<400> 51
ggttaacggt ggcgacgcgg tggaaccg          28
<210> 52
<211> 28
<212> DNA
<213> Artificial Sequence
<220>
<223> primer for point mutation
<400> 52

ggttaacggt ggcgaagcgg tggaaccg      28

<210> 53
<211> 28
<212> DNA
<213> Artificial Sequence
<220>
<223> primer for point mutation
<400> 53

ggttaacggt ggcttcgcgg tggaaccg      28

<210> 54
<211> 28
<212> DNA
<213> Artificial Sequence
<220>
<223> primer for point mutation
<400> 54

ggttaacggt ggcggtgcgg tggaaccg      28

<210> 55
<211> 28
<212> DNA
<213> Artificial Sequence
<220>
<223> primer for point mutation
<400> 55

ggttaacggt ggccacgcgg tggaaccg      28

<210> 56
<211> 28
<212> DNA
<213> Artificial Sequence
<220>
<223> primer for point mutation
<400> 56

ggttaacggt ggcatcgcgg tggaaccg      28

<210> 57
<211> 28
<212> DNA
<213> Artificial Sequence
<220>
<223> primer for point mutation
<400> 57

ggttaacggt ggcaaagcgg tggaaccg      28

<210> 58
<211> 28
<212> DNA
<213> Artificial Sequence
<220>
<223> primer for point mutation
<400> 58

ggttaacggt ggcctggcgg tggaaccg      28

<210> 59
<211> 28
<212> DNA
<213> Artificial Sequence
<220>
<223> primer for point mutation
<400> 59

ggttaacggt ggcaacgcgg tggaaccg      28

<210> 60

<211> 28
<212> DNA
<213> Artificial Sequence
<220>
<223> primer for point mutation
<400> 60
ggttaacggt ggcccggcgg tggaaccg          28
<210> 61
<211> 28
<212> DNA
<213> Artificial Sequence
<220>
<223> primer for point mutation
<400> 61
ggttaacggt ggccaggcgg tggaaccg          28
<210> 62
<211> 28
<212> DNA
<213> Artificial sequence
<220>
<223> primer for point mutation
<400> 62
ggttaacggt ggcttcgcgg tggaaccg          28
<210> 63
<211> 28
<212> DNA
<213> Artificial Sequence
<220>
<223> primer for point mutation
<400> 63
ggttaacggt ggctctgcgg tggaaccg          28
<210> 64
<211> 28
<212> DNA
<213> Artificial Sequence
<220>
<223> primer for point mutation
<400> 64
ggttaacggt ggcaccgcgg tggaaccg          28
<210> 65
<211> 28
<212> DNA
<213> Artificial Sequence
<220>
<223> primer for point mutation
<400> 65
ggttaacggt ggcgttgcgg tggaaccg          28
<210> 66
<211> 28
<212> DNA
<213> Artificial Sequence
<220>
<223> primer for point mutation
<400> 66
ggttaacggt ggcggcgcgg tggaaccg          28
<210> 67
<211> 28
<212> DNA

<210> 67

<211> 28

<212> DNA

<213> Artificial Sequence

<220>

<223> primer for point mutation

<400> 67

ggttaacggt ggctacgcgg tggaaccg    28

<210> 68

<211> 28

<212> DNA

<213> Artificial sequence

<220>

<223> primer for point mutation

<400> 68

cggttccacc gccgcgccac cgttaacc    28

<210> 69

<211> 28

<212> DNA

<213> Artificial Sequence

<220>

<223> primer for point mutation

<400> 69

cggttccacc gcgcagccac cgttaacc    28

<210> 70

<211> 28

<212> DNA

<213> Artificial Sequence

<220>

<223> primer for point mutation

<400> 70

cggttccacc gcgtcgccac cgttaacc    28

<210> 71

<211> 28

<212> DNA

<213> Artificial Sequence

<220>

<223> primer for point mutation

<400> 71

cggttccacc gcttcgccac cgttaacc    28

<210> 72

<211> 28

<212> DNA

<213> Artificial Sequence

<220>

<223> primer for point mutation

<400> 72

cggttccacc gcgaagccac cgttaacc    28

<210> 73

<211> 28

<212> DNA

<213> Artificial Sequence

<220>

<223> primer for point mutation

<400> 73

cggttccacc gcaccgccac cgttaacc    28

<210> 74

<211> 28

<212> DNA

<213> Artificial Sequence

<220>

<223> primer for point mutation
<400> 74
cggttccacc gcgtggccac cgttaacc     28
<210> 75
<211> 28
<212> DNA
<213> Artificial Sequence
<220>
<223> primer for point mutation
<400> 75
cggttccacc gcgatgccac cgttaacc     28
<210> 76
<211> 28
<212> DNA
<213> Artificial Sequence
<220>
<223> primer for point mutation
<400> 76
cggttccacc gctttgccac cgttaacc     28
<210> 77
<211> 28
<212> DNA
<213> Artificial Sequence
<220>
<223> primer for point mutation
<400> 77
cggttccacc gccaggccac cgttaacc     28
<210> 78
<211> 28
<212> DNA
<213> Artificial Sequence
<220>
<223> primer for point mutation
<400> 78
cggttccacc gcgttgccac cgttaacc     28
<210> 79
<211> 28
<212> DNA
<213> Artificial Sequence
<220>
<223> primer for point mutation
<400> 79
cggttccacc gccgggccac cgttaacc     28
<210> 80
<211> 28
<212> DNA
<213> Artificial Sequence
<220>
<223> primer for point mutation
<400> 80
cggttccacc gcctggccac cgttaacc     28
<210> 81
<211> 28
<212> DNA
<213> Artificial Sequence
<220>
<223> primer for point mutation
<400> 81

cggttccacc gcgaagccac cgttaacc          28
<210> 82
<211> 28
<212> DNA
<213> Artificial Sequence
<220>
<223> primer for point mutation
<400> 82
cggttccacc gcagagccac cgttaacc          28
<210> 83
<211> 28
<212> DNA
<213> Artificial Sequence
<220>
<223> primer for point mutation
<400> 83
cggttccacc gcggtgccac cgttaacc          28
<210> 84
<211> 28
<212> DNA
<213> Artificial Sequence
<220>
<223> primer for point mutation
<400> 84
cggttccacc gcaacgccac cgttaacc          28
<210> 85
<211> 28
<212> DNA
<213> Artificial Sequence
<220>
<223> primer for point mutation
<400> 85
cggttccacc gcgccgccac cgttaacc          28
<210> 86
<211> 28
<212> DNA
<213> Artificial Sequence
<220>
<223> primer for point mutation
<400> 86
cggttccacc gcgtagccac cgttaacc          28
<210> 87
<211> 29
<212> DNA
<213> Artificial Sequence
<220>
<223> primer for point mutation
<400> 87
gggtaccgtg tttgcgccga acgtgtatg          29
<210> 88
<211> 29
<212> DNA
<213> Artificial Sequence
<220>
<223> primer for point mutation
<400> 88
gggtaccgtg ttttgcccga acgtgtatg          29
<210> 89

<211> 29
<212> DNA
<213> Artificial Sequence
<220>
<223> primer for point mutation
<400> 89
gggtaccgtg tttgacccga acgtgtatg    29
<210> 90
<211> 29
<212> DNA
<213> Artificial Sequence
<220>
<223> primer for point mutation
<400> 90
gggtaccgtg tttgaaccga acgtgtatg    29
<210> 91
<211> 29
<212> DNA
<213> Artificial Sequence
<220>
<223> primer for point mutation
<400> 91
gggtaccgtg tttttcccga acgtgtatg    29
<210> 92
<211> 29
<212> DNA
<213> Artificial Sequence
<220>
<223> primer for point mutation
<400> 92
gggtaccgtg tttggtccga acgtgtatg    29
<210> 93
<211> 29
<212> DNA
<213> Artificial Sequence
<220>
<223> primer for point mutation
<400> 93
gggtaccgtg tttcacccga acgtgtatg    29
<210> 94
<211> 29
<212> DNA
<213> Artificial Sequence
<220>
<223> primer for point mutation
<400> 94
gggtaccgtg tttatcccga acgtgtatg    29
<210> 95
<211> 29
<212> DNA
<213> Artificial Sequence
<220>
<223> primer for point mutation
<400> 95
gggtaccgtg tttaaaccga acgtgtatg    29
<210> 96
<211> 29
<212> DNA

<213> Artificial Sequence
<220>
<223> primer for point mutation
<400> 96
gggtaccgtg tttctgccga acgtgtatg     29
<210> 97
<211> 29
<212> DNA
<213> Artificial Sequence
<220>
<223> primer for point mutation
<400> 97
gggtaccgtg tttatgccga acgtgtatg     29
<210> 98
<211> 29
<212> DNA
<213> Artificial Sequence
<220>
<223> primer for point mutation
<400> 98
gggtaccgtg tttaacccga acgtgtatg 29
<210> 99
<211> 29
<212> DNA
<213> Artificial Sequence
<220>
<223> primer for point mutation
<400> 99
gggtaccgtg tttccgccga acgtgtatg     29
<210> 100
<211> 29
<212> DNA
<213> Artificial Sequence
<220>
<223> primer for point mutation
<400> 100
gggtaccgtg tttcagccga acgtgtatg     29
<210> 101
<211> 29
<212> DNA
<213> Artificial sequence
<220>
<223> primer for point mutation
<400> 101
gggtaccgtg tttttcccga acgtgtatg     29
<210> 102
<211> 29
<212> DNA
<213> Artificial Sequence
<220>
<223> primer for point mutation
<400> 102
gggtaccgtg ttttctccga acgtgtatg     29
<210> 103
<211> 29
<212> DNA
<213> Artificial Sequence
<220>

<223> primer for point mutation

<400> 103

gggtaccgtg tttaccccga acgtgtatg     29

<210> 104

<211> 29

<212> DNA

<213> Artificial Sequence

<220>

<223> primer for point mutation

<400> 104

gggtaccgtg tttggcccga acgtgtatg     29

<210> 105

<211> 29

<212> DNA

<213> Artificial Sequence

<220>

<223> primer for point mutation

<400> 105

gggtaccgtg ttttacccga acgtgtatg     29

<210> 106

<211> 29

<212> DNA

<213> Artificial sequence

<220>

<223> primer for point mutation

<400> 106

catacacgtt cggcgcaaac acggtaccc     29

<210> 107

<211> 29

<212> DNA

<213> Artificial Sequence

<220>

<223> primer for point mutation

<400> 107

catacacgtt cgggcaaaac acggtaccc     29

<210> 108

<211> 29

<212> DNA

<213> Artificial Sequence

<220>

<223> primer for point mutation

<400> 108

catacacgtt cgggtcaaac acggtaccc     29

<210> 109

<211> 29

<212> DNA

<213> Artificial Sequence

<220>

<223> primer for point mutation

<400> 109

catacacgtt cggttcaaac acggtaccc     29

<210> 110

<211> 29

<212> DNA

<213> Artificial Sequence

<220>

<223> primer for point mutation

<400> 110

**129**

catacacgtt cggaccaaac acggtaccc          29
<210> 111
<211> 29
<212> DNA
<213> Artificial Sequence
<220>
<223> primer for point mutation
<400> 111
catacacgtt cggaccaaac acggtaccc          29
<210> 112
<211> 29
<212> DNA
<213> Artificial Sequence
<220>
<223> primer for point mutation
<400> 112
catacacgtt cgggtgaaac acggtaccc          29
<210> 113
<211> 29
<212> DNA
<213> Artificial Sequence
<220>
<223> primer for point mutation
<400> 113
catacacgtt cgggataaac acggtaccc          29
<210> 114
<211> 29
<212> DNA
<213> Artificial Sequence
<220>
<223> primer for point mutation
<400> 114
catacacgtt cggtttaaac acggtaccc          29
<210> 115
<211> 29
<212> DNA
<213> Artificial Sequence
<220>
<223> primer for point mutation
<400> 115
catacacgtt cggcagaaac acggtaccc          29
<210> 116
<211> 29
<212> DNA
<213> Artificial Sequence
<220>
<223> primer for point mutation
<400> 116
catacacgtt cggcataaac acggtaccc          29
<210> 117
<211> 29
<212> DNA
<213> Artificial Sequence
<220>
<223> primer for point mutation
<400> 117
catacacgtt cgggttaaac acggtaccc          29
<210> 118

<211> 29
<212> DNA
<213> Artificial Sequence
<220>
<223> primer for point mutation
<400> 118
catacacgtt cggcggaaac acggtaccc          29
<210> 119
<211> 29
<212> DNA
<213> Artificial Sequence
<220>
<223> primer for point mutation
<400> 119
catacacgtt cggctgaaac acggtaccc          29
<210> 120
<211> 29
<212> DNA
<213> Artificial Sequence
<220>
<223> primer for point mutation
<400> 120
catacacgtt cgggaaaaac acggtaccc          29
<210> 121
<211> 29
<212> DNA
<213> Artificial Sequence
<220>
<223> primer for point mutation
<400> 121
catacacgtt cggagaaaac acggtaccc          29
<210> 122
<211> 29
<212> DNA
<213> Artificial Sequence
<220>
<223> primer for point mutation
<400> 122
catacacgtt cggggtaaac acggtaccc          29
<210> 123
<211> 29
<212> DNA
<213> Artificial Sequence
<220>
<223> primer for point mutation
<400> 123
catacacgtt cgggccaaac acggtaccc          29
<210> 124
<211> 29
<212> DNA
<213> Artificial Sequence
<220>
<223> primer for point mutation
<400> 124
catacacgtt cgggtaaaac acggtaccc          29
<210> 125
<211> 29
<212> DNA

<213> Artificial Sequence
<220>
<223> primer for point mutation
<400> 125
gatagctctg ttgcggccga aattgcacc     29
<210> 126
<211> 29
<212> DNA
<213> Artificial Sequence
<220>
<223> primer for point mutation
<400> 126
gatagctctg tttgcgccga aattgcacc     29
<210> 127
<211> 29
<212> DNA
<213> Artificial Sequence
<220>
<223> primer for point mutation
<400> 127
gatagctctg ttgacgccga aattgcacc     29
<210> 128
<211> 29
<212> DNA
<213> Artificial Sequence
<220>
<223> primer for point mutation
<400> 128
gatagctctg ttgaagccga aattgcacc     29
<210> 129
<211> 29
<212> DNA
<213> Artificial Sequence
<220>
<223> primer for point mutation
<400> 129
gatagctctg ttggtgccga aattgcacc     29
<210> 130
<211> 29
<212> DNA
<213> Artificial Sequence
<220>
<223> primer for point mutation
<400> 130
gatagctctg ttcacgccga aattgcacc     29
<210> 131
<211> 29
<212> DNA
<213> Artificial Sequence
<220>
<223> primer for point mutation
<400> 131
gatagctctg ttatcgccga aattgcacc     29
<210> 132
<211> 29
<212> DNA
<213> Artificial Sequence
<220>

<223> primer for point mutation
<400> 132
gatagctctg ttaaagccga aattgcacc      29
<210> 133
<211> 29
<212> DNA
<213> Artificial Sequence
<220>
<223> primer for point mutation
<400> 133
gatagctctg ttctggccga aattgcacc      29
<210> 134
<211> 29
<212> DNA
<213> Artificial Sequence
<220>
<223> primer for point mutation
<400> 134
gatagctctg ttatggccga aattgcacc      29
<210> 135
<211> 29
<212> DNA
<213> Artificial Sequence
<220>
<223> primer for point mutation
<400> 135
gatagctctg ttaacgccga aattgcacc      29
<210> 136
<211> 29
<212> DNA
<213> Artificial Sequence
<220>
<223> primer for point mutation
<400> 136
gatagctctg ttccggccga aattgcacc      29
<210> 137
<211> 29
<212> DNA
<213> Artificial Sequence
<220>
<223> primer for point mutation
<400> 137
gatagctctg ttcaggccga aattgcacc      29
<210> 138
<211> 29
<212> DNA
<213> Artificial Sequence
<220>
<223> primer for point mutation
<400> 138
gatagctctg ttttcgccga aattgcacc      29
<210> 139
<211> 29
<212> DNA
<213> Artificial Sequence
<220>
<223> primer for point mutation
<400> 139

gatagctctg tttctgccga aattgcacc          29
<210> 140
<211> 29
<212> DNA
<213> Artificial Sequence
<220>
<223> primer for point mutation
<400> 140
gatagctctg ttaccgccga aattgcacc          29
<210> 141
<211> 29
<212> DNA
<213> Artificial Sequence
<220>
<223> primer for point mutation
<400> 141
gatagctctg ttgttgccga aattgcacc          29
<210> 142
<211> 29
<212> DNA
<213> Artificial Sequence
<220>
<223> primer for point mutation
<400> 142
gatagctctg ttggcgccga aattgcacc          29
<210> 143
<211> 29
<212> DNA
<213> Artificial Sequence
<220>
<223> primer for point mutation
<400> 143
gatagctctg tttacgccga aattgcacc          29
<210> 144
<211> 29
<212> DNA
<213> Artificial Sequence
<220>
<223> primer for point mutation
<400> 144
ggtgcaattt cggccgcaac agagctatc          29
<210> 145
<211> 29
<212> DNA
<213> Artificial Sequence
<220>
<223> primer for point mutation
<400> 145
ggtgcaattt cggcgcaaac agagctatc          29
<210> 146
<211> 29
<212> DNA
<213> Artificial Sequence
<220>
<223> primer for point mutation
<400> 146
ggtgcaattt cggcgtcaac agagctatc          29
<210> 147

<211> 29
<212> DNA
<212> DNA
<213> Artificial Sequence
<220>
<223> primer for point mutation
<400> 147
ggtgcaattt cggcttcaac agagctatc    29
<210> 148
<211> 29
<212> DNA
<213> Artificial Sequence
<220>
<223> primer for point mutation
<400> 148
ggtgcaattt cggcaccaac agagctatc    29
<210> 149
<211> 29
<212> DNA
<213> Artificial Sequence
<220>
<223> primer for point mutation
<400> 149
ggtgcaattt cggcgtgaac agagctatc    29
<210> 150
<211> 29
<212> DNA
<213> Artificial Sequence
<220>
<223> primer for point mutation
<400> 150
ggtgcaattt cggcgataac agagctatc    29
<210> 151
<211> 29
<212> DNA
<213> Artificial Sequence
<220>
<223> primer for point mutation
<400> 151
ggtgcaattt cggctttaac agagctatc    29
<210> 152
<211> 29
<212> DNA
<213> Artificial Sequence
<220>
<223> primer for point mutation
<400> 152
ggtgcaattt cggccagaac agagctatc    29
<210> 153
<211> 29
<212> DNA
<213> Artificial Sequence
<220>
<223> primer for point mutation
<400> 153
ggtgcaattt cggccataac agagctatc    29
<210> 154
<211> 29

<212> DNA
<213> Artificial Sequence
<220>
<223> primer for point mutation
<400> 154
ggtgcaattt cggcgttaac agagctatc 29
<210> 155
<211> 29
<212> DNA
<213> Artificial Sequence
<220>
<223> primer for point mutation
<400> 155
ggtgcaattt cggccggaac agagctatc 29
<210> 156
<211> 29
<212> DNA
<213> Artificial Sequence
<220>
<223> primer for point mutation
<400> 156
ggtgcaattt cggcctgaac agagctatc 29
<210> 157
<211> 29
<212> DNA
<213> Artificial Sequence
<220>
<223> primer for point mutation
<400> 157
ggtgcaattt cggcgaaaac agagctatc 29
<210> 158
<211> 29
<212> DNA
<213> Artificial Sequence
<220>
<223> primer for point mutation
<400> 158
ggtgcaattt cggcagaaac agagctatc 29
<210> 159
<211> 29
<212> DNA
<213> Artificial Sequence
<220>
<223> primer for point mutation
<400> 159
ggtgcaattt cggcggtaac agagctatc 29
<210> 160
<211> 29
<212> DNA
<213> Artificial Sequence
<220>
<223> primer for point mutation
<400> 160
ggtgcaattt cggcaacaac agagctatc 29
<210> 161
<211> 29
<212> DNA
<213> Artificial Sequence

<220>
<223> primer for point mutation
<400> 161
ggtgcaattt cggcgccaac agagctatc          29
<210> 162
<211> 29
<212> DNA
<213> Artificial sequence
<220>
<223> primer for point mutation
<400> 162
ggtgcaattt cggcgtaaac agagctatc          29
- 1 -

**Description**

Technical Field

The present invention relates to a cholesterol oxidase for use in a kit and a sensor for the measurement of cholesterol. More particularly, the present invention relates to a mutant cholesterol oxidase having reduced oxidase activity.

Background Art

The concentration of lipoproteins in blood is quite important in clinical tests. Lipoproteins in blood can be divided in High Density Lipoproteins and Low Density Lipoproteins, both having different biological functions. As a measure of the lipoprotein content in blood, cholesterol associated with the lipoproteins is measured. In biological samples like blood, cholesterol is present in the lipoproteins in the form of cholesterol esters. To measure the lipoprotein associated cholesterol levels the cholesterol esters are split by the use of enzymes like cholesterol esterase and the freed cholesterol is then determined. The cholesterol concentration in blood may be measured using an enzyme having specificity to cholesterol.

Cholesterol oxidases have been isolated from various kinds of strains and it has been suggested that cholesterol may be analyzed using such enzymes. For example, Vrielink et al., FEBS J (2009), 276 (23):6826-6848 provides an overview of structure and properties of wild type and mutant cholesterol oxidases from a variety of organisms. Particular mutant cholesterol oxidases from different organisms are disclosed to show altered substrate specificity (Pollegioni et al., FEBS J. (2009), 276(23):6857-70; Toyama et al., Protein Engineering (2002), 15(6):477-483), improved thermostability (Pollegioni, *supra,* Yan Sun et a., Biotechnology Letters (2011), 33(10):2049-2055) and reduced activity for either oxidation or isomerization (Vrielink, *supra,* Yue et al., Biochemistry (1999), 38(14):4277-4286, Toyama *supra*). However, none of those documents disclose a mutant cholesterol oxidase consisting of an amino acid sequence set forth in SEQ ID NO: 1 and having a reduced oxidase activity as compared to the wild-type cholesterol oxidase and having a dehydrogenase activity of 50% or more of the wild-type cholesterol oxidase, wherein the amino acid residue at position 228 is an amino acid residue selected from Ala, Thr, Lys, Cys, Ser, Gly, Glu, Tyr, Pro, Asn, Gln, Trp and His.

Cholesterol oxidase is a FAD-dependent enzyme which catalyzes a reaction where cholesterol is oxidized to generate cholest-4-en-3-one, with generating the reduced form of FAD (FADH2). FADH2 in turn transmits electron to an electron acceptor and is converted to its oxidized form. In the presence of oxygen, FADH2 preferentially transmits electrons to the oxygen molecule rather than to artificial electron acceptors (also referred to as mediators or electron mediators). Thus, when cholesterol is assayed by cholesterol oxidase with mediators, the assay results will be greatly affected by the dissolved oxygen level in the reaction system. Such a disadvantage will be particularly noted in clinical tests of blood samples by a point-of-care testing device utilizing an artificial electron acceptor. The enzyme used for the enzyme sensor strips employing artificial electron mediators desirably have low activity toward oxygen.

Accordingly, the object of the present invention is to provide an enzyme, in particular a cholesterol oxidase, whose activity is less affected by the dissolved oxygen level.

Disclosure of the Invention

The present application provides an enzyme, in particular a cholesterol oxidase, whose activity is less affected by the dissolved oxygen level. More specifically, this has been achieved by reducing the oxidase activity of an enzyme that in its wild-type form predominantly shows an oxidase activity and by preferably at the same time increasing the enzyme's dehydrogenase activity. As will be described in more detail below, this has been achieved by mutating the wild type enzyme. The present inventors have prepared various mutants of a cholesterol oxidase and surprisingly found that a certain type of mutants exhibit reduced oxidase activity while substantially retaining dehydrogenase activity, in particular dye-mediated dehydrogenase activity.

The present invention provides a mutant cholesterol oxidase as defined in independent claim 1. Preferred embodiments of the mutant cholesterol oxidase are the subject matter of the claims depending from claim 1.

In a preferred embodiment, the mutant cholesterol oxidase of the invention has a reduced oxidase activity as compared to the wild-type cholesterol oxidase, and preferably has an increased dehydrogenase activity compared to the wild-type cholesterol oxidase. Preferably, the mutant cholesterol oxidase of the invention has an oxidase activity of 30% or less of that of the wild-type cholesterol oxidase, and preferably also has a dehydrogenase activity of 50% or more of the wild-type cholesterol oxidase. Also preferably, the mutant cholesterol oxidase of the invention has an increased dehydrogenase activity compared to the wild-type cholesterol oxidase.

In another aspect, the present invention provides an isolated polynucleotide encoding the mutant cholesterol oxidase of the present invention.

In yet another aspect, the present invention provides a vector comprising the polynucleotide of the invention.

In still another aspect, the present invention provides a host cell transformed with a vector of the invention.

In another aspect, the present invention provides a method for assaying cholesterol in a sample, comprising contacting the sample with the cholesterol oxidase of the invention and measuring the amount of the cholesterol oxidized by cholesterol oxidase.

In another aspect, the present invention provides a device for assaying cholesterol in a sample comprising the cholesterol oxidase of the invention and preferably an electron mediator.

In yet another aspect, the present invention provides a kit for assaying cholesterol in a sample comprising the cholesterol oxidase of the invention and preferably an electron mediator.

In another aspect, the present invention provides an enzyme electrode having the cholesterol oxidase of the invention which is immobilized on the electrode.

In another aspect, the present invention provides an enzyme sensor for assaying cholesterol comprising the enzyme electrode of the invention as a working electrode.

Detailed Description of the Invention

The term "mutant" of a protein as used herein refers to a variant protein containing substitution in one or more of the amino acid residues on the protein at the indicated position(s). The term mutant is also used for a polynucleotide encoding such a mutant protein.

The phrase "a position corresponding to" as used herein means the position of an amino acid residue in a query amino acid sequence that is aligned with the amino acid residue in a reference amino acid sequence using a software AlignX of Vector NTI with default parameters (available from Invitrogen; see, Lu, G., and Moriyama, E. N. (2004) Vector NTI, a balanced all-in-one sequence analysis suite. Brief Bioinform 5, 378-88). Thus, "amino acid (AA) residue at a position corresponding to the position Y of the amino acid sequence set forth in SEQ ID NO: X" means the AA residue in a query amino acid sequence that is aligned with AA Y of SEQ ID NO: X when the query amino acid sequence is aligned with SEQ ID NO: X using AlignX of Vector NTI with default parameters. It should be noted that the AA Y of SEQ ID NO: X itself is also encompassed by this term.

The mutant cholesterol oxidase of the invention exhibit decreased oxidase (or Ox) activity while substantially retaining dehydrogenase (or Dh) activity.

As used herein "oxidase activity" is an enzymatic activity of the cholesterol oxidase to catalyze oxidation of cholesterol to generate cholest-4-en-3-one with utilizing oxygen as an electron acceptor. The oxidase activity may be assayed by measuring the amount of generated $H_2O_2$ by any methods known in the art, for example, by reagents for $H_2O_2$ detection such as 4AA/TODB/POD (4-aminoantipyrine/N,N-Bis(4-sulfobutyl)-3-methylaniline disodium salt/horseradish peroxidase)or by Pt electrode. As used herein in the context of the relative or quantitative activity, the oxidase activity is specifically defined to be the mole amount of the substrate (cholesterol) oxidized per unit time measured by the amount of generated $H_2O_2$ at 25 °C in 10 mM PPB, pH 7.0, 1.5 mM TODB, 2 U/ml horseradish peroxidase (POD), and 1.5 mM

4-aminoanti¬pyrine (4AA). The formation of quinoneimine dye may be measured spectrophotometrically at 546 nm.

As used herein, "dehydrogenase activity" is an enzymatic activity of the cholesterol oxidase to catalyze oxidation of cholesterol to generate cholest-4-en-3-one with utilizing an electron mediator other than oxygen as an electron acceptor. The dehydrogenase activity may be assayed by measuring the amount of electron transferred to the mediator using, for example, mPMS/DCIP (1-methoxy-5-methylphenazinium methylsulfate/ 2,6-dichloroindophenol), cPES (trifluoro-acetate-1-(3-carboxy-propoxy)-5-ethyl-phenanzinium, NA BM31_1144 (N,N-bis-(hydroxyethyl)¬3-methoxy-nitrosoaniline hydrochloride, NA BM31_1008 (N,N-bis-hydroxy¬ethyl-4-nitrosoaniline) and N-N-4-dimethyl-nitrosoaniline.

As used herein in the context of the relative or quantitative activity, the dehydrogenase activity is specifically defined to be the mole amount of the substrate (cholesterol) oxidized per unit time measured by the amount of electron transferred to the mediator at 25 °C in 10 mM PPB (pH 7.0), 0.6 mM DCIP, and 6 mM methoxy PMS (mPMS).

The mutant cholesterol oxidase of the invention has a reduced oxidase activity as compared to the wild-type cholesterol oxidase, while substantially retaining the dehydrogenase activity.

Preferably, the mutant cholesterol oxidase of the invention has an oxidase activity of 50% or less of that of the wild-type cholesterol oxidase. More preferably, the mutant cholesterol oxidase of the invention has an oxidase activity of 40% or less, more preferably 30% or less, even more preferably 20% or less, most preferably 15% or less of that of the wild-type cholesterol oxidase. Also preferably, the mutant cholesterol oxidase of the invention has a dehydrogenase activity of 50% or more of the wild-type cholesterol oxidase. More preferably, the mutant cholesterol oxidase of the invention has a dehydrogenase activity of 70% or more, more preferably 90% or more, even more preferably 100% or more, most preferably more than 100% of the wild-type cholesterol oxidase.

In the wild-type cholesterol oxidase, the oxidase activity is about 300 times higher than the dehydrogenase activity. When dissolved oxygen is present in the assay system, the electron generated by the oxidation of the substrate will be preferentially transferred to the oxygen. Thus the enzyme activity measured in the presence of electron mediator will be greatly affected by the dissolved oxygen concentration. In contrast, the mutant cholesterol oxidase of the invention has the ratio of dehydrogenase/oxidase activity of about 2.0 or more, preferably 4.0 or more, more preferably 10 or more. Since the dehydrogenase activity exceed the oxidase activity, the enzyme activity of the cholesterol oxidase of the present invention will be less affected by the dissolved oxygen concentration, which is advantageous in utilizing the cholesterol oxidase in clinical diagnosis with a blood sample.

It should be understood that the numbering of the amino acid sequence in this application begins at the initial Met and that the claimed mutant cholesterol oxidase may or may not have the signal peptide.

In another aspect, the present invention provides an isolated polynucleotide encoding the mutant cholesterol oxidase of the present invention. The nucleotide sequence of polynucleotides coding for cholesterol oxidase may be easily obtained from public databases. The polynucleotide encoding the wild type cholesterol oxidase may be cloned from the genome of respective organisms using PCR or other known techniques. Mutations may be introduced by site-directed mutagenesis, PCR mutagenesis or any other techniques well known in the art. The amino acid residue to be mutated may be identified using any of software for sequence alignment available in the art. Alternatively, polynucleotide coding for the mutant cholesterol oxidase may be prepared by PCR using a series of chemically synthesized oligonucleotides, or fully synthesized.

The mutated cholesterol oxidase may be prepared by inserting the mutant gene into an appropriate expression vector and introducing the vector into an appropriate host cell, such as E. coli cells. The transformant is cultured and the cholesterol oxidase expressed in the transformant may be collected from the cells or culture medium.

The recombinant cholesterol oxidase thus obtained may be purified by any of the purification techniques known in the art, including ion exchange column chromatography, affinity chromatography, liquid chromatography, filtration, ultrafiltration, salt precipitation, solvent precipitation, immunoprecipitation, gel electrophoresis, isoelectric electrophoresis and dialysis.

Thus, the present invention also encompasses a vector comprising the polynucleotide encoding the mutant cholesterol oxidase, a host cell transformed with such a vector, and a method for preparing the mutant cholesterol oxidase of the invention by culturing the transformant, collecting and purifying the mutant cholesterol oxidase from the culture.

The invention also encompasses a method for assaying cholesterol, HDL cholesterol or LDL cholesterol in a sample. The method comprises contacting the sample with the cholesterol oxidase of the invention and measuring the amount of the cholesterol oxidized by the cholesterol oxidase.

In another aspect, the present invention provides a device for assaying cholesterol, HDL cholesterol or LDL cholesterol in a sample comprising the cholesterol oxidase of the invention and an electron mediator.

The assay device may have a similar structure as any of conventional, commercially available amperometric biosensor test strips for monitoring the blood cholesterol level. One example of such a device has two electrodes (working electrode and reference or counter electrode) positioned on an insulating substrate, a reagent port and a sample receiver. The reagent port contains the mutated cholesterol oxidase of the invention and a mediator. When a sample such as blood sample is added to the sample receiver, cholesterol contained in the sample will react with cholesterol oxidase to generate current, which is indicative of the amount of cholesterol in the sample. Typical examples of electrochemical sensors suited for the determination of enzyme substrates are known, e.g. from WO 2004/113900 and US 5,997,817. As an alternative to electrochemical sensors, optical detection technologies might be used. Typically, such optical devices are based on color changes that occur in a reagent system comprising the enzyme, an electron mediator and an indicator. The color changes can be quantified using fluorescence, absorption or remission measurements. Typical examples of optical devices suited for the determination of enzyme substrates are known, e.g. from US 7,008,799, US 6,036,919, and US 5,334,508.

In yet another aspect, the present invention provides a kit for assaying cholesterol, HDL cholesterol or LDL cholesterol in a sample comprising the cholesterol oxidase of the invention and an electron mediator.

A kit for the measurement of cholesterol, HDL cholesterol or LDL cholesterol may be constructed using the enzyme of the present invention. In addition to the cholesterol oxidase of the invention, the kit contains buffer necessary for the measurement, appropriate mediator and, if necessary, further enzymes such as cholesterol esterase, a standard solution of cholesterol for the preparation of a calibration curve and an instruction for use. The cholesterol oxidase of the present invention may be provided in various forms, for example, as a freeze-dried reagent or as a solution in an appropriate storage solution.

In another aspect, the present invention provides an enzyme electrode having the cholesterol oxidase of the invention which is immobilized on the electrode.

In another aspect, the present invention provides an enzyme sensor for assaying cholesterol comprising the enzyme electrode of the invention as a working electrode.

The concentration of the cholesterol in a sample may be determined by measuring the amount of electron generated by the enzyme reaction. Various sensor systems have been known in the art, including carbon electrode, metal electrode, and platinum electrode. The mutated cholesterol oxidase of the present invention is immobilized on the electrodes. Examples of the means for immobilization include cross-linking, encapsulating into a macromolecular matrix, coating with a dialysis membrane, optical cross-linking polymer, electroconductive polymer, oxidation-reduction polymer, and any combination thereof.

When measurement is conducted in an amperometric system using carbon electrode, gold electrode or platinum electrode provided with an immobilized enzyme is used as a working electrode, together with a counter electrode (such as platinum electrode) and a reference electrode (such as Ag/AgCl electrode). The electrodes are inserted into a buffer containing a mediator and kept at predetermined temperature. Predetermined voltage is applied to the working electrode, then a sample is added and increased value in electric current is measured. Examples of the mediator used in the assay include potassium ferricyanide, ferrocene, osmium derivative, ruthenium derivative, phenazine methosulfate, etc. It is generally also possible to use so-called two-electrode systems with one working electrode and one counter or pseudo-reference electrode.

Further, cholesterol may be assayed using an immobilized electron mediator in an amperometric system using carbon electrode, gold electrode, or platinum electrode. The enzyme is immobilized on the electrode together with an electron mediator such as potassium ferricyanide, ferrocene, osmium derivative, phenazine methosulfate in a macromolecular matrix by means of adsorption or covalent bond to prepare a working electrode. It is inserted into buffer together with a counter electrode (such as platinum electrode) and a reference electrode (such as Ag/AgCl electrode), and kept at a predetermined temperature. Predetermined voltage is applied to the working electrode, then the sample is added and increased value in electric current is measured.

It is to be understood that whenever this application refers to cholesterol as an analyte also other analytes that can be converted to cholesterol, like e.g. HDL cholesterol or LDL cholesterol, shall be encompassed. A person of skill in the art knows that a cholesterol esterase enzyme may be needed to set free cholesterol from cholesterol esters which naturally occur in sample materials like blood or blood fractions.

Examples

The present invention will be illustrated in detail by way of the Examples below, although the present invention shall not be limited to those Examples.

**Example 1: Plasmid expressing ChOx of *Streptomyces* sp. strain SA-COO**

pET28 ChOx_Nhis was used as a plasmid expressing ChOx of *Streptomyces* sp. strain SA-COO. This plasmid has the DNA fragment of the ChOx structural gene derived from *Streptomyces* sp. strain SA-COO which is inserted in the *NheI/Hind*III cloning site of a vector pET28a. The ChOx gene in this plasmid is controlled by a T7 promoter. The pET28 ChOx_Nhis contains a kanamycin resistance gene.

**Example 2: Mutagenesis of ChOx structural gene derived from *Streptomyces* sp. strain SA-COO**

(1) Mutagenesis of residues 159, 228, and 396

The *Streptomyces* sp. strain SA-COO-derived ChOx structural gene contained in the pET28 ChOx_Nhis obtained in Example 1 was mutagenized such that methionine at residue 159, valine at residue 228, and phenylalanine at residue 396 in ChOx encoded by this gene were substituted by other amino acid residues.

Specifically, codon (ATG) for methionine at residue 159, codon (GTT) for valine at residue 228, and codon (TTT) for phenylalanine at residue 396 in the ChOx structural gene contained in the plasmid pET28 ChOx_Nhis described in Example 1 were substituted by other amino acid codons using a commercially available site-directed mutagenesis kit (Stratagene Corp., QuikChange II Site-Directed Mutagenesis Kit).

The sequences of forward and reverse primers used in the amino acid residue substitution are shown in the tables below.

In notation representing a mutation, the number represents a position in the amino acid sequence containing the signal sequence of ChOx; the alphabet described before the number represents an amino acid residue before amino acid substitution; and the alphabet described after the number represents an amino acid residue after amino acid substitution. For example, M159A represents the substitution of methionine at residue 159 to alanine.

In PCR reaction, a reaction solution of the composition shown below was subjected to reaction at 95°C for 30 seconds and then 15 repetitive cycles each involving 95°C for 30 seconds, 55°C for 1 minute, and 68°C for 8 minutes, followed by 68°C for 30 minutes and then kept at 4°C.

### Composition of reaction solution

| | |
|---|---|
| Template DNA (5 ng/μL) | 2 μL |
| 10×reaction buffer | 5 μL |
| Forward primer (100 ng/μL) | 1.25 μL |
| Reverse primer (100 ng/μL) | 1.25 μL |
| dNTP | 1 μL |
| Distilled water | 38.5 μL |
| DNA polymerase | 1 μL |
| Total | 50 μL |

After the PCR reaction, 0.5 μL of *Dpn*I was added to the reaction solution and incubated at 37°C for 1 hour to degrade the template plasmid.

*Escherichia coli* DH5α (supE44, ΔlacU169 (φ801acZΔM15), hsdR17, recA1, endA1, gyrA96, thi-1, relA1) competent cells were transformed with the obtained reaction solution. From colonies grown on an LB agar medium (1% Bacto tryptone, 0.5% yeast extracts, 1% sodium chloride, 1.5% agar) containing kanamycin (50 μg/mL), plasmid DNA was prepared and sequenced to confirm that the mutation of interest was introduced in the ChOx structural gene.

The plasmid confirmed to have the introduced mutation was digested with restriction enzymes *Nhe*I and *Hind*III to excise the mutagenized ChOx structural gene, which was in turn inserted to a pET28a vector. DH5α was transformed with this plasmid, and a plasmid was extracted from the obtained colonies to obtain a ChOx mutant expression plasmid.

**Table 1: Forward primer for M159**

| Amino acid substitution | Primer name | Sequence | SEQ ID NO. |
|---|---|---|---|
| M159A | M159AFw | 5'GGTTAACGGTGGCGCGGCGGTGGAACCG 3' | 49 |
| M159C | M159CFw | 5'GGTTAACGGTGGCTGCGCGGTGGAACCG 3' | 50 |
| M159D | M159DFw | 5'GGTTAACGGTGGCGACGCGGTGGAACCG 3' | 51 |
| M159E | M159EFw | 5'GGTTAACGGTGGCGAAGCGGTGGAACCG 3' | 52 |
| M159F | M159FFw | 5'GGTTAACGGTGGCTTCGCGGTGGAACCG 3' | 53 |
| M159G | M159GFw | 5'GGTTAACGGTGGCGGTGCGGTGGAACCG 3' | 54 |
| M159H | M159HFw | 5'GGTTAACGGTGGCCACGCGGTGGAACCG 3' | 55 |
| M159I | M159IFw | 5'GGTTAACGGTGGCATCGCGGTGGAACCG 3' | 56 |
| M159K | M159KFw | 5'GGTTAACGGTGGCAAAGCGGTGGAACCG 3' | 57 |
| M159L | M159LFw | 5'GGTTAACGGTGGCCTGGCGGTGGAACCG 3' | 58 |
| M159N | M159NFw | 5'GGTTAACGGTGGCAACGCGGTGGAACCG 3' | 59 |
| M159P | M159PFw | 5'GGTTAACGGTGGCCCGGCGGTGGAACCG 3' | 60 |
| M159Q | M159QFw | 5'GGTTAACGGTGGCCAGGCGGTGGAACCG 3' | 61 |
| M159R | M159RFw | 5'GGTTAACGGTGGCTTCGCGGTGGAACCG 3' | 62 |
| M159S | M159SFw | 5'GGTTAACGGTGGCTCTGCGGTGGAACCG 3' | 63 |
| M159T | M159TFw | 5'GGTTAACGGTGGCACCGCGGTGGAACCG 3' | 64 |
| M159V | M159VFw | 5'GGTTAACGGTGGCGTTGCGGTGGAACCG 3' | 65 |
| M159W | M159WFw | 5'GGTTAACGGTGGCGGCGCGGTGGAACCG 3' | 66 |
| M159Y | M159YFw | 5'GGTTAACGGTGGCTACGCGGTGGAACCG 3' | 67 |

**Table 2: Reverse primer for M159**

| Amino acid substitution | Primer name | Sequence | SEQ ID NO. |
|---|---|---|---|
| M159A | M159ARv | 5'CGGTTCCACCGCCGCGCCACCGTTAACC 3' | 68 |
| M159C | M159CRv | 5'CGGTTCCACCGCGCAGCCACCGTTAACC 3' | 69 |
| M159D | M159DRv | 5'CGGTTCCACCGCGTCGCCACCGTTAACC 3' | 70 |
| M159E | M159ERv | 5'CGGTTCCACCGCTTCGCCACCGTTAACC 3' | 71 |
| M159F | M159FRv | 5'CGGTTCCACCGCGAAGCCACCGTTAACC 3' | 72 |
| M159G | M159GRv | 5'CGGTTCCACCGCACCGCCACCGTTAACC 3' | 73 |
| M159H | M159HRv | 5'CGGTTCCACCGCGTGGCCACCGTTAACC 3' | 74 |

(continued)

| Amino acid substitution | Primer name | Sequence | SEQ ID NO. |
|---|---|---|---|
| M159I | M159IRv | 5'CGGTTCCACCGC<u>GAT</u>GCCACCGTTAACC 3' | 75 |
| M159K | M159KRv | 5'CGGTTCCACCGC<u>TTT</u>GCCACCGTTAACC 3' | 76 |
| M159L | M159LRv | 5'CGGTTCCACCGCC<u>AG</u>GCCACCGTTAACC 3' | 77 |
| M159N | M159NRv | 5'CGGTTCCACCGC<u>GTT</u>GCCACCGTTAACC 3' | 78 |
| M159P | M159PRv | 5'CGGTTCCACCGCC<u>GG</u>GCCACCGTTAACC 3' | 79 |
| M159Q | M159QRv | 5'CGGTTCCACCGCC<u>TG</u>GCCACCGTTAACC 3' | 80 |
| M159R | M159RRv | 5'CGGTTCCACCGC<u>GAA</u>GCCACCGTTAACC 3' | 81 |
| M159S | M159SRv | 5'CGGTTCCACCGC<u>AGA</u>GCCACCGTTAACC 3' | 82 |
| M159T | M159TRv | 5'CGGTTCCACCGC<u>GGT</u>GCCACCGTTAACC 3' | 83 |
| M159V | M159VRv | 5'CGGTTCCACCGC<u>AAC</u>GCCACCGTTAACC 3' | 84 |
| M159W | M159WRv | 5'CGGTTCCACCGC<u>GCC</u>GCCACCGTTAACC 3' | 85 |
| M159Y | M159YRv | 5'CGGTTCCACCGC<u>GTA</u>GCCACCGTTAACC 3' | 86 |

**Table 3:** Forward primer for V228

| Amino acid substitution | Primer name | Sequence | SEQ ID NO. |
|---|---|---|---|
| V228A | V228AFw | 5'GGGTACCGTGTTT<u>GCG</u>CCGAACGTGTATG 3' | 87 |
| V228C | V228CFw | 5'GGGTACCGTGTTT<u>TGC</u>CCGAACGTGTATG 3' | 88 |
| V228D | V228DFw | 5'GGGTACCGTGTTT<u>GAC</u>CCGAACGTGTATG 3' | 89 |
| V228E | V228EFw | 5'GGGTACCGTGTTT<u>GAA</u>CCGAACGTGTATG 3' | 90 |
| V228F | V228FFw | 5'GGGTACCGTGTTT<u>TTC</u>CCGAACGTGTATG 3' | 91 |
| V228G | V228GFw | 5'GGGTACCGTGTTT<u>GGT</u>CCGAACGTGTATG 3' | 92 |
| V228H | V228HFw | 5'GGGTACCGTGTTT<u>CAC</u>CCGAACGTGTATG 3' | 93 |
| V228I | V228IFw | 5'GGGTACCGTGTTT<u>ATC</u>CCGAACGTGTATG 3' | 94 |
| V228K | V228KFw | 5'GGGTACCGTGTTT<u>AAA</u>CCGAACGTGTATG 3' | 95 |
| V228L | V228LFw | 5'GGGTACCGTGTTT<u>CTG</u>CCGAACGTGTATG 3' | 96 |
| V228M | V228MFw | 5'GGGTACCGTGTTT<u>ATG</u>CCGAACGTGTATG 3' | 97 |
| V228N | V228NFw | 5'GGGTACCGTGTTT<u>AAC</u>CCGAACGTGTATG 3' | 98 |
| V228P | V228PFw | 5'GGGTACCGTGTTT<u>CCG</u>CCGAACGTGTATG 3' | 99 |
| V228Q | V228QFw | 5'GGGTACCGTGTTT<u>CAG</u>CCGAACGTGTATG 3' | 100 |
| V228R | V228RFw | 5'GGGTACCGTGTTT<u>TTC</u>CCGAACGTGTATG 3' | 101 |
| V228S | V228SFw | 5'GGGTACCGTGTTT<u>TCT</u>CCGAACGTGTATG 3' | 102 |
| V228T | V228TFw | 5'GGGTACCGTGTTT<u>ACC</u>CCGAACGTGTATG 3' | 103 |
| V228W | V228WFw | 5'GGGTACCGTGTTT<u>GGC</u>CCGAACGTGTATG 3' | 104 |
| V228Y | V228YFw | 5'GGGTACCGTGTTT<u>TAC</u>CCGAACGTGTATG 3' | 105 |

**Table 4:** Reverse primer for V228

| Amino acid substitution | Primer name | Sequence | SEQ ID NO. |
|---|---|---|---|
| V228A | V228ARv | 5'CATACACGTTCGG<u>CGC</u>AAACACGGTACCC 3' | 106 |
| V228C | V228CRv | 5'CATACACGTTCGG<u>GCA</u>AAACACGGTACCC 3' | 107 |
| V228D | V228DRv | 5'CATACACGTTCGG<u>GTC</u>AAACACGGTACCC 3' | 108 |
| V228E | V228ERv | 5'CATACACGTTCGG<u>TTC</u>AAACACGGTACCC 3' | 109 |
| V228F | V228FRv | 5'CATACACGTTCGG<u>ACC</u>AAACACGGTACCC 3' | 110 |
| V228G | V228GRv | 5'CATACACGTTCGG<u>ACC</u>AAACACGGTACCC 3' | 111 |
| V228H | V228HRv | 5'CATACACGTTCGG<u>GTG</u>AAACACGGTACCC 3' | 112 |
| V228I | V228IRv | 5'CATACACGTTCGG<u>GAT</u>AAACACGGTACCC 3' | 113 |
| V228K | V228KRv | 5'CATACACGTTCGG<u>TTT</u>AAACACGGTACCC 3' | 114 |
| V228L | V228LRv | 5'CATACACGTTCGG<u>CAG</u>AAACACGGTACCC 3' | 115 |
| V228M | V228MRv | 5'CATACACGTTCGG<u>CAT</u>AAACACGGTACCC 3' | 116 |

(continued)

| Amino acid substitution | Primer name | Sequence | SEQ ID NO. |
|---|---|---|---|
| V228N | V228NRv | 5'CATACACGTTCGGGTTAAACACGGTACCC 3' | 117 |
| V228P | V228PRv | 5'CATACACGTTCGGCGGAAACACGGTACCC 3' | 118 |
| V228Q | V228QRv | 5'CATACACGTTCGGCTGAAACACGGTACCC 3' | 119 |
| V228R | V228RRv | 5'CATACACGTTCGGGAAAAACACGGTACCC 3' | 120 |
| V228S | V228SRv | 5'CATACACGTTCGGAGAAAACACGGTACCC 3' | 121 |
| V228T | V228TRv | 5'CATACACGTTCGGGGTAAACACGGTACCC 3' | 122 |
| V228W | V228WRv | 5'CATACACGTTCGGGCCAAACACGGTACCC 3' | 123 |
| V228Y | V228YRv | 5'CATACACGTTCGGGTAAAACACGGTACCC 3' | 124 |

**Table 5: Forward primer for F396**

| Amino acid substitution | Primer name | Sequence | SEQ ID NO. |
|---|---|---|---|
| F396A | F396AFw | 5'GATAGCTCTGTTGCGGCCGAAATTGCACC 3' | 125 |
| F396C | F396CFw | 5'GATAGCTCTGTTTGCGCCGAAATTGCACC 3' | 126 |
| F396D | F396DFw | 5'GATAGCTCTGTTGACGCCGAAATTGCACC 3' | 127 |
| F396E | F396EFw | 5'GATAGCTCTGTTGAAGCCGAAATTGCACC 3' | 128 |
| F396G | F396GFw | 5'GATAGCTCTGTTGGTGCCGAAATTGCACC 3' | 129 |
| F396H | F396HFw | 5'GATAGCTCTGTTCACGCCGAAATTGCACC 3' | 130 |
| F396I | F396IFw | 5'GATAGCTCTGTTATCGCCGAAATTGCACC 3' | 131 |
| F396K | F396KFw | 5'GATAGCTCTGTTAAAGCCGAAATTGCACC 3' | 132 |
| F396L | F396LFw | 5'GATAGCTCTGTTCTGGCCGAAATTGCACC 3' | 133 |
| F396M | F396MFw | 5'GATAGCTCTGTTATGGCCGAAATTGCACC 3' | 134 |
| F396N | F396NFw | 5'GATAGCTCTGTTAACGCCGAAATTGCACC 3' | 135 |
| F396P | F396PFw | 5'GATAGCTCTGTTCCGGCCGAAATTGCACC 3' | 136 |
| F396Q | F396QFw | 5'GATAGCTCTGTTCAGGCCGAAATTGCACC 3' | 137 |
| F396R | F396RFw | 5'GATAGCTCTGTTTTCGCCGAAATTGCACC 3' | 138 |
| F396S | F396SFw | 5'GATAGCTCTGTTTCTGCCGAAATTGCACC 3' | 139 |
| F396T | F396TFw | 5'GATAGCTCTGTTACCGCCGAAATTGCACC 3' | 140 |
| F396V | F396VFw | 5'GATAGCTCTGTTGTTGCCGAAATTGCACC 3' | 141 |
| F396W | F396WFw | 5'GATAGCTCTGTTGGCGCCGAAATTGCACC 3' | 142 |
| F396Y | F396YFw | 5'GATAGCTCTGTTTACGCCGAAATTGCACC 3' | 143 |

**Table 6: Reverse primer for F396**

| Amino acid substitution | Primer name | Sequence | SEQ ID NO. |
|---|---|---|---|
| F396A | F396ARv | 5'GGTGCAATTTCGGCCGCAACAGAGCTATC 3' | 144 |
| F396C | F396CRv | 5'GGTGCAATTTCGGCGCAAACAGAGCTATC 3' | 145 |
| F396D | F396DRv | 5'GGTGCAATTTCGGCGTCAACAGAGCTATC 3' | 146 |
| F396E | F396ERv | 5'GGTGCAATTTCGGCTTCAACAGAGCTATC 3' | 147 |
| F396G | F396GRv | 5'GGTGCAATTTCGGCACCAACAGAGCTATC 3' | 148 |
| F396H | F396HRv | 5'GGTGCAATTTCGGCGTGAACAGAGCTATC 3' | 149 |
| F396I | F396IRv | 5'GGTGCAATTTCGGCGATAACAGAGCTATC 3' | 150 |
| F396K | F396KRv | 5'GGTGCAATTTCGGCTTTAACAGAGCTATC 3' | 151 |
| F396L | F396LRv | 5'GGTGCAATTTCGGCCAGAACAGAGCTATC 3' | 152 |
| F396M | F396MRv | 5'GGTGCAATTTCGGCCATAACAGAGCTATC 3' | 153 |
| F396N | F396NRv | 5'GGTGCAATTTCGGCGTTAACAGAGCTATC 3' | 154 |
| F396P | F396PRv | 5'GGTGCAATTTCGGCCGGAACAGAGCTATC 3' | 155 |
| F396Q | F396QRv | 5'GGTGCAATTTCGGCCTGAACAGAGCTATC 3' | 156 |
| F396R | F396RRv | 5'GGTGCAATTTCGGCGAAAACAGAGCTATC 3' | 157 |
| F396S | F396SRv | 5'GGTGCAATTTCGGCAGAAACAGAGCTATC 3' | 158 |

(continued)

| Amino acid substitution | Primer name | Sequence | SEQ ID NO. |
|---|---|---|---|
| F396T | F396TRv | 5'GGTGCAATTTCGGCGGTAACAGAGCTATC 3' | 159 |
| F396V | F396VRv | 5'GGTGCAATTTCGGCAACAACAGAGCTATC 3' | 160 |
| F396W | F396WRv | 5'GGTGCAATTTCGGCGCCAACAGAGCTATC 3' | 161 |
| F396Y | F396YRv | 5'GGTGCAATTTCGGCGTAAACAGAGCTATC 3' | 162 |

**Example 3: Analysis of enzymatic activity of mutant ChOx**

Mutant ChOx was produced using the mutant ChOx expression plasmid obtained in Example 2, and studied for its enzymatic activity.

(1) Culture

*Escherichia coli* strain BL21 (DE3) was transformed with the wild-type ChOx expression plasmid prepared in Example 1 or the mutant ChOx expression plasmid prepared in Example 2. These transformants were separately shake-cultured at 37°C for 12 hours in 3 mL of an LB medium (containing 50 μg/mL kanamycin) using an L-shaped tube. 1 mL each of these culture solutions was inoculated to a 500-mL Erlenmeyer flask with a baffle containing 100 mL of an LB medium (containing 50 μg/mL kanamycin) and gyratory-cultured at 37°C. At the point in time when OD600 reached around 0.6, IPTG (isopropyl-β-D-thiogalactopyranoside) was added thereto at a final concentration of 1 mM, followed by culture at 20°C for 24 hours.

(2) Preparation of water-soluble fraction

From the culture solution thus cultured, bacterial cells were collected and washed. Then, the obtained wet bacterial cells were suspended in a 10 mM potassium phosphate buffer (pH 7.0) and sonicated. Then, the homogenate was centrifuged at 17400xg at 4°C for 20 minutes, and the supernatant was collected. This supernatant was further ultracentrifuged at 100400xg at 4°C for 60 minutes, and the supernatant was collected. The obtained supernatant was dialyzed against a 10 mM potassium phosphate buffer (pH 7.0), and this was used as a water-soluble fraction. This water-soluble fraction was used as a ChOx sample to determine cholesterol oxidase (ChOx) and cholesterol dehydrogenase (ChDH) activities for each of wild-type ChOx and mutant ChOx.

(4) Preparation of substrate solution

Cholesterol powder was dissolved at a concentration of 100 mM in Triton X-100 and incubated at 80°C to completely dissolve cholesterol. The 100 mM cholesterol solution was diluted 10-fold with pure water, cooled in running water, and brought to room temperature. Then, sodium cholate was added thereto at a final concentration of 3 mM to prepare a 10 mM cholesterol solution. For activity determination, the cholesterol solution was appropriately diluted with pure water to prepare various concentrations of substrate solutions.

(5) Determination of ChOx activity

The ChOx activity was determined by determining change in absorbance at 546 nm over time derived from a dye generated using peroxidase, a Trinder reagent (TODB), and 4-aminoantipyrine from hydrogen peroxide generated through reaction with the substrate. The reaction was performed under conditions shown below. The reaction was initiated by adding the substrate to a reaction solution (10 mM potassium phosphate buffer pH 7.0 + 1.5 mM 4-aminoantipyrine + 1.5 mM TODB + 2 U/ml peroxidase; all the concentrations are final concentrations) containing the enzyme solution, and change in absorbance at 546 nm was determined. Various concentrations of cholesterol were used as the substrate. The amount of an enzyme that forms 1 μmol hydrogen peroxide for 1 minute is defined as 1 U. 38 mM-1 cm$^{-1}$ was used as the molar absorption coefficient of TODB at pH 7.0. The formula for calculating an activity value from change in absorbance is shown below.

$$U/ml = \Delta ABS_{546}/min \times 2/38 \times 10$$

$$U/mg = U/ml/protein\ mg/ml$$

(6) Determination of ChDH activity

The ChDH activity was determined by quantifying change in absorbance at 600 nm over time derived from the fading of DCIP reduced through reaction with the substrate. The reaction was performed under conditions shown below.
The reaction was initiated by adding the substrate to a reaction solution (10 mM potassium phosphate buffer pH 7.0 + 0.6 mM PMS + 0.06 mM DCIP; all the concentrations are final concentrations) containing the enzyme solution, and change in absorbance at 600 nm was determined. Those used in the ChOx activity determination were used as the substrate. The amount of an enzyme that reduces 1 $\mu$mol DCIP is defined as 1 U. The activity value was calculated according to the formula shown below. 16.3 mM-1 cm$^{-1}$ was used as the molar absorption coefficient of DCIP at pH 7.0.

$$U/ml = \Delta ABS_{600}/min \times 1/16.3 \times 5$$

$$U/mg = U/ml/protein\ mg/ml$$

The results of activity determination of the wild-type ChOx and the mutant ChOx are shown in Tables 7 to 9.
The oxidase activities of all M159 mutant enzymes were largely reduced. Among them, M159F, M159L, and M159V had dehydrogenase activity improved to 1.7 to 2.9 times the wild-type. Particularly, M159F had an oxidase activity value of $2.0 \times 10^{-2}$ U/mg and a dehydrogenase activity value of $2.2 \times 10^{-2}$ U/mg, which was 2.9 times the wild-type. The ratio of the dehydrogenase activity to the oxidase activity was 0.28% in the wild-type, whereas this ratio was 110% in M159F, which was improved to approximately 390 times the wild-type.
The oxidase activities of all the V228 mutant enzymes were lower than the wild-type (2.7 U/mg). The mutant that exhibited the lowest activity was V228D ($2.0 \times 10^{-4}$ U/mg). This value was approximately 1/10000 of the wild-type, showing significantly reduced reactivity to oxygen. In addition to V228D, V228N, V228Q, V228S, and V228K exhibited an oxidase activity value as very low as 1% or less of the wild-type (V228N: $7.0 \times 10^{-3}$ U/mg, V228E: $5.0 \times 10^{-3}$ U/mg, V228S: $9.0 \times 10^{-3}$ U/mg, V228K: $8.8 \times 10^{-3}$ U/mg). On the other hand, the Val228 mutants having substitution to isoleucine, leucine, or phenylalanine, including V228A, had a relatively high oxidase activity value (ratio to wild-type: 10%-) and thus retained reactivity to oxygen (V228I: 2.2 U/mg, V228L: $9.4 \times 10^{-1}$ U/mg, V228F: $2.2 \times 10^{-1}$ U/mg). 8 mutants having dehydrogenase activity improved compared with the wild-type were obtained. Among them, V228T exhibited activity ($5.6 \times 10^{-2}$ U/mg) approximately 5 times the wild-type. In addition, the mutants having substitution to lysine, serine, or cysteine exhibited high dehydrogenase activity (V228K: $3.4 \times 10^{-2}$ U/mg, V228C: $3.1 \times 10^{-2}$ U/mg, V228S: $2.0 \times 10^{-2}$ U/mg). On the other hand, the mutants having substitution to leucine or isoleucine had activity reduced to 1/10 of the wild-type. (V228L: $1.0 \times 10^{-3}$ U/mg, V228I: $1.0 \times 10^{-3}$ U/mg). V228D and V228R had no detectable dehydrogenase activity.
The oxidase activities of the F396 mutant enzymes were reduced. The F396W mutant enzyme had an oxidase activity value of $1.2 \times 10^{-1}$ U/mg and a dehydrogenase activity value of $2.0 \times 10^{-2}$ U/mg. The oxidase activity was reduced compared with that of the wild-type ChOx, and the dehydrogenase activity was improved to twice or more the wild-type. The ratio of the dehydrogenase activity to the oxidase activity was 16%, which was 57 times the wild-type (0.28%). F396N and F396D had not only oxidase activity reduced to 1.9% and 0.032%, respectively, of the wild-type but also dehydrogenase activity reduced to 23% and 14%, respectively, of the wild-type. Each of the mutant enzymes F396M, F396L, F396V, F396I, and F396Y maintained oxidase activity (30 to 80% of the wild-type) compared with the other mutant enzymes.

**Table 7:**

|  | Oxidase activity (U/mg) | Dehydrogenase activity (U/mg) | Dh/Ox (%) |
|---|---|---|---|
| WT | 2.8 (100%) | $7.7 \times 10^{-3}$ (100%) | 0.28 |
| M159A | $3.6 \times 10^{-2}$ (1.3%) | $6.4 \times 10^{-3}$ (83%) | 18 |
| M159C | $2.1 \times 10^{-2}$ (0.75%) | $7.7 \times 10^{-3}$ (100%) | 37 |
| M159D | $7.4 \times 10^{-4}$ (0.026%) | $1.6 \times 10^{-3}$ (21%) | 220 |
| M159E | $4.4 \times 10^{-4}$ (0.016%) | $1.0 \times 10^{-3}$ (13%) | 230 |

(continued)

|  | Oxidase activity (U/mg) | Dehydrogenase activity (U/mg) | Dh/Ox (%) |
|---|---|---|---|
| M159F | $2.0 \times 10^{-2}$ (0.71%) | $2.2 \times 10^{-2}$ (290%) | 110 |
| M159G | $1.4 \times 10^{-3}$ (0.05%) | $1.0 \times 10^{-3}$ (13%) | 71 |
| M159H | $6.2 \times 10^{-3}$ (0.22%) | $7.6 \times 10^{-4}$ (9.9%) | 12 |
| M159I | $3.1 \times 10^{-1}$ (11%) | $6.4 \times 10^{-3}$ (83%) | 2.1 |
| M159K | $6.0 \times 10^{-4}$ (0.021 %) | $3.0 \times 10^{-3}$ (39%) | 500 |
| M159L | $5.0 \times 10^{-2}$ (18%) | $1.6 \times 10^{-2}$ (210%) | 3.2 |
| M159N | $5.2 \times 10^{-2}$(1.9%) | $4.2 \times 10^{-4}$ (5.5%) | 0.81 |
| M159P | $1.9 \times 10^{-3}$ (0.068%) | $4.0 \times 10^{-4}$ (5.2%) | 21 |
| M159Q | $3.1 \times 10^{-2}$ (1.1%) | $5.0 \times 10^{-3}$ (65%) | 16 |
| M159R | $1.5 \times 10^{-4}$ (0.0054%) | $4.8 \times 10^{-4}$ (6.2%) | 320 |
| M159S | $1.8 \times 10^{-1}$ (6.4%) | $2.9 \times 10^{-3}$ (38%) | 1.6 |
| M159T | $2.9 \times 10^{-1}$ (10%) | $2.9 \times 10^{-3}$ (38%) | 1 |
| M159V | $4.7 \times 10^{-2}$ (1.7%) | $1.3 \times 10^{-2}$ (170%) | 28 |
| M159W | $2.3 \times 10^{-2}$ (0.82%) | $3.8 \times 10^{-4}$ (4.9%) | 1.7 |
| M159Y | $7.7 \times 10^{-3}$ (0.28%) | $5.0 \times 10^{-3}$ (65%) | 65 |

**Table 8:**

|  | Oxidase activity (U/mg) | Dehydrogenase activity (U/mg) | Dh/Ox (%) |
|---|---|---|---|
| WT | 2.5 - 2.9 | $7.0 \times 10^{-3}$ - $10 \times 10^{-3}$ | 0.25-0.35% |
| V228A | $3.0 \times 10^{-2}$ - $4.3 \times 10^{-2}$ | $5.7 \times 10^{-2}$ - $7.4 \times 10^{-2}$ | 150-180% |
| V228C | $7.0 \times 10^{-2}$ | $3.1 \times 10^{-2}$ | 44% |
| V228D | $2.0 \times 10^{-4}$ | not detected | - |
| V228E | $5.0 \times 10^{-3}$ | $1.4 \times 10^{-2}$ | 280% |
| V228F | $2.2 \times 10^{-1}$ | $5.0 \times 10^{-3}$ | 2.3% |
| V228G | $8.0 \times 10^{-3}$ | $1.8 \times 10^{-2}$ | 225% |
| V228H | $1.0 \times 10^{-2}$ | $6.0 \times 10^{-3}$ | 60% |
| V228I | 2.2 | $1.0 \times 10^{-3}$ | 0.06% |
| V228K | $8.8 \times 10^{-3}$ | $3.4 \times 10^{-2}$ | 374% |
| V228L | $9.4 \times 10^{-2}$ | $1.0 \times 10^{-3}$ | 0.1% |
| V228M | $2.2 \times 10^{-1}$ | $6.0 \times 10^{-4}$ | 0.27% |
| V228N | $7.0 \times 10^{-3}$ | $9.0 \times 10^{-3}$ | 130% |
| V228P | $4.8 \times 10^{-2}$ | $1.2 \times 10^{-2}$ | 25% |
| V228Q | $2.9 \times 10^{-2}$ | $6.7 \times 10^{-3}$ | 23% |
| V228R | $2.2 \times 10^{-3}$ | not detected | - |
| V228S | $9.0 \times 10^{-3}$ | $2.0 \times 10^{-2}$ | 222% |
| V228T | $1.7 \times 10^{-1}$ | $5.6 \times 10^{-2}$ | 33% |
| V228W | $4.9 \times 10^{-2}$ | $6.2 \times 10^{-3}$ | 13% |

(continued)

|  | Oxidase activity (U/mg) | Dehydrogenase activity (U/mg) | Dh/Ox (%) |
|---|---|---|---|
| V228Y | $1.2 \times 10^{-1}$ | $1.3 \times 10^{-2}$ | 110% |

**Table 9:**

|  | Oxidase activity (U/mg) | Dehydrogenase activity (U/mg) | Dh/Ox (%) |
|---|---|---|---|
| WT | 2.8 (100%) | $7.7 \times 10^{-3}$ (100%) | 0.28 |
| F396A | $5.9 \times 10^{-2}$ (2.1%) | $1.8 \times 10^{-3}$ (23%) | 3.1 |
| F396C | $2.2 \times 10^{-1}$ (7.9%) | $2.3 \times 10^{-3}$ (30%) | 1.0 |
| F396D | $8.9 \times 10^{-4}$ (0.032%) | $1.1 \times 10^{-3}$ (14%) | 120 |
| F396E | $2.5 \times 10^{-3}$ (0.089%) | not detected | - |
| F396G | $4.9 \times 10^{-1}$ (18%) | $8.3 \times 10^{-4}$ (11%) | 0.17 |
| F396H | $5.8 \times 10^{-2}$ (2.1%) | $1.5 \times 10^{-3}$ (19%) | 2.6 |
| F396I | $9.7 \times 10^{-1}$ (35%) | $2.3 \times 10^{-3}$ (30%) | 0.24 |
| F396K | $8.9 \times 10^{-2}$ (3.2%) | $3.0 \times 10^{-3}$ (39%) | 3.4 |
| F396L | 1.1 (39%) | $2.6 \times 10^{-3}$ (34%) | 0.24 |
| F396M | $8.4 \times 10^{-2}$ (30%) | $5.9 \times 10^{-3}$ (77%) | 0.70 |
| F396N | $5.3 \times 10^{-2}$ (1.9%) | $1.8 \times 10^{-3}$ (23%) | 3.4 |
| F396P | $2.1 \times 10^{-4}$ (0.0075%) | not detected | - |
| F396Q | $9.6 \times 10^{-4}$ (0.034%) | not detected | - |
| F396R | $3.3 \times 10^{-3}$ (0.12%) | not detected | - |
| F396S | $8.5 \times 10^{-2}$ (3.0%) | $4.1 \times 10^{-3}$ (53%) | 2.7 |
| F396T | $1.9 \times 10^{-1}$ (6.8%) | $3.1 \times 10^{-3}$ (40%) | 1.7 |
| F396V | $7.6 \times 10^{-1}$ (27%) | $2.4 \times 10^{-3}$ (31%) | 0.32 |
| F396W | $1.2 \times 10^{-1}$ (4.3%) | $2.0 \times 10^{-2}$ (260%) | 16 |
| F396Y | 1.7(61%) | $9.1 \times 10^{-3}$ (120%) | 0.54 |

Tables 10-12 show alignment of the amino acid sequences that are annotated to be cholesterol oxidases. The entire sequences of these mutated cholesterol oxidases are set forth in SEQ ID NOs: 1-48. Alignment was created using AlignX application of Vector NTI suite 6.0. Those skilled in the art will appreciate that another alignment software programs such as Blast will provide the same or substantially the same alignment.

It is evident from Table 10 that Met159 of SEQ ID NO:1 is conserved among the amino acid sequences listed in Table 10. Accordingly, a person skilled in the art can easily identify the Met or Ile residue corresponding to the Met159 of SEQ ID NO:1 within the conserved region using any of commercially available software programs for sequence alignment, and understand that a mutant cholesterol oxidase is easily prepared by introducing modification on that Met or Ile residue.

**Table 10:**

| Origin* | position of mutation |  |  |  | SEQ ID NO** |
|---|---|---|---|---|---|
| sp|P12676 | M159 | 149 | VGGGSLVNGG**M**AVEPKRSYFE | 169 | 1 |
| gb|ABS32193 | M155 | 145 | VGGGSLVNGG**M**AVAPKRSYFE | 165 | 2 |
| ref|NP_821583 | M160 | 150 | VGGGSLVNGG**M**AVTPRRSYFE | 170 | 3 |
| emb|CAC20926 | M162 | 152 | VGGGSLVNGG**M**AVVPKRSYFE | 172 | 4 |

(continued)

| Origin* | position of mutation | | | | SEQ ID NO** |
|---|---|---|---|---|---|
| gb\|ADX66466 | M165 | 155 | VGGGSLVNGG**M**AVVPKRSYFE | 175 | 5 |
| gb\|AAR16516 | M162 | 152 | VGGGSLVNGG**M**AVVPKRSYFE | 172 | 6 |
| gb\|AAZ66744 | M165 | 155 | VGGGSLVNGG**M**AVVPKRAYFE | 175 | 7 |
| gb\|AAA69655 | M160 | 150 | VGGGSLVNGG**M**AVTPRRSYFO | 170 | 8 |
| ref\|ZP_07282326 | M157 | 147 | VGGGSLVNGA**M**AVQPKRSYFE | 167 | 9 |
| ref\|ZP_07286354 | M165 | 155 | VGGGSLVNGG**M**APTPRRSYFA | 175 | 10 |
| ref\|ZP_04998002 | M166 | 156 | VGGGSLVNGG**M**SPTPRRSYFS | 176 | 11 |
| ref\|YP_003492288 | M154 | 144 | VGGGSLVNGS**M**AVTPLRSYFA | 164 | 12 |
| gb\|ADI09201 | M158 | 148 | VGGGSLVNGG**M**AVTPSRAYFQ | 168 | 13 |
| ref\|ZP_07603147 | 1164 | 154 | VGGGSLVNGG**I**AVTPSRSYFQ | 174 | 14 |
| ref\|YP_003512290 | M148 | 138 | VGGGSLVNGG**M**AVVPRRKYFQ | 158 | 15 |
| ref\|ZP_05008217 | M154 | 144 | VGGGSLVNGG**M**AVTPRPYFS | 164 | 16 |
| ref\|ZP_06770826 | M168 | 158 | VGGGSLVNGG**M**AVTPRPYFS | 178 | 17 |
| ref\|ZP_06566730 | M151 | 141 | VGGGSLVNGG**M**AVTPRRGYFE | 161 | 18 |
| ref\|NP_827244 | M159 | 149 | VGGGSLVNGG**M**AVTPLQSYFA | 169 | 19 |
| ref\|YP_001108512 | M139 | 129 | VGGGSLVNGG**M**AVTPRRGYFE | 149 | 20 |
| ref\|ZP_07292951 | I161 | 151 | VGGGSLVNGG**I**AVTPPRAYFQ | 171 | 21 |
| ref\|ZP_07303187 | M159 | 149 | VGGGSLVNGG**M**AVTPLRSYFA | 169 | 22 |
| ref\|ZP_01689718 | M146 | 136 | VGGGSLVNGG**M**AVTPPMNYFQ | 156 | 23 |
| ref\|YP_003384280 | M157 | 147 | VGGGSLVNGG**M**APTPRRSYFE | 167 | 24 |
| ref\|ZP_06916507 | M159 | 149 | VGGGSLVNGS**M**AVTPLQSYFA | 169 | 25 |
| ref\|YP_003134867 | M150 | 140 | VGGGSLVNGA**M**AVTPKRATFA | 160 | 26 |
| emb\|CAC44897 | M167 | 157 | VGGGSLVNGG**M**AVTPKRNYFE | 177 | 27 |
| pdb\|1C0Y_A | M122 | 112 | VGGGSLVNGG**M**AVTPKRNYFE | 132 | 28 |
| sp\|P22637 | M167 | 157 | VGGGSLVNGG**M**AVTPKRNYFE | 177 | 29 |
| ref\|ZP_06830857 | M167 | 157 | VGGGSLVNGG**M**AVTPKRNYFE | 177 | 30 |
| gb\|ABC75776 | M167 | 157 | VGGGSLVNGG**M**AVTPKRNYFE | 177 | 31 |
| gb\|ABG24169 | M167 | 157 | VGGGSLVNGG**M**AVTPKRNYFE | 177 | 32 |
| emb\|CAZ68116 | M158 | 148 | VGGGSLVNGG**M**AVTPKRNYFE | 168 | 33 |
| ref\|YP_002764459 | M167 | 157 | VGGGSLVNGG**M**AVTPKRNYFE | 177 | 34 |
| ref\|ZP_04388109 | M137 | 127 | VGGGSLVNGG**M**AVTPKRNYFE | 147 | 35 |
| ref\|YP_003769621 | M142 | 132 | VGGGSLVNGG**M**AVTPKRENFG | 152 | 36 |
| ref\|YP_003339891 | M166 | 156 | VGGGSLVNGG**M**AVTPKRENFG | 176 | 37 |
| ref\|ZP_07290670 | M168 | 158 | VGGGSLVNGG**M**AVTPKRQNFA | 178 | 38 |
| ref\|YP_003116660 | M165 | 155 | IGGGSLVNGG**M**AVTPKQENFG | 175 | 39 |
| ref\|ZP_06588880 | M169 | 159 | VGGGSLVNGG**M**AVTPRRENFG | 179 | 40 |
| ref\|ZP_04697978 | M121 | 111 | VGGGSLVNGG**M**AVTPRRENFG | 131 | 41 |
| ref\|ZP_06276136 | M166 | 156 | VGGGSLVNGG**M**AVTPRRENFG | 176 | 42 |

(continued)

| Origin* | position of mutation | | | | SEQ ID NO** |
|---|---|---|---|---|---|
| ref\|YP_001821989 | M166 | 156 | VGGGSLVNGG**M**AVTPRRENFG | 176 | 43 |
| ref\|ZP_05002034 | M165 | 155 | VGGGSLVNGG**M**AVTPKRERFG | 175 | 44 |
| ref\|ZP_06907496 | M166 | 156 | VGGGSLVNGG**M**AVTPRRENFG | 176 | 45 |
| ref\|YP_003100211 | M163 | 153 | VGGGSLVNGG**M**AVTPRRERFA | 173 | 46 |
| ref\|ZP_07085639 | M142 | 132 | VGGGSLVNGG**M**AVTPKESYFR | 152 | 47 |
| gb\|ADX68765 | M139 | 129 | VGGGSLVNGG**M**AVLPKKNYFK | 149 | 48 |
| * Databases: gb: GenBank; sp: Swissprot; ref: RefSeq; emb: EMBL; pdb: Protein Data Bank<br>** SEQ ID NOs represent the full length sequence | | | | | |

It is evident from Table 11 that Val228 of SEQ ID NO:1 is conserved among the amino acid sequences listed in Table 11. Accordingly, a person skilled in the art can easily identify the Val, Met or Ile residue corresponding to the Val228 of SEQ ID NO:1 within the conserved region using any of commercially available software programs for sequence alignment, and understand that a mutant cholesterol oxidase is easily prepared by introducing modification on that Val, Met or Ile residue.

**Table 11:**

| Origin* | position of mutation | | | | SEQ ID NO** |
|---|---|---|---|---|---|
| sp\|P12676 | V228 | 218 | AGKAGLGTVF<u>V</u>PNVYDFGYMQ | 238 | 1 |
| gb\|ABS32193 | V224 | 214 | AQKAGLGTVH<u>V</u>PNVYDFDHMR | 234 | 2 |
| ref\|NP_821583 | V229 | 219 | ASNAGLSTTF<u>V</u>PNVYDWDYMR | 239 | 3 |
| emb\|CAC20926 | V231 | 221 | AGKAGLGTTF<u>V</u>PNVYDFDYMR | 241 | 4 |
| gb\|ADX66466 | V234 | 224 | AGKAGLSTTF<u>V</u>PNVYDFDYMR | 244 | 5 |
| gb\|AAR16516 | V231 | 221 | AGKAGLSTTF<u>V</u>PNVYDFDHMR | 241 | 6 |
| gb\|AAZ66744 | V234 | 224 | ASKAGLGTTF<u>V</u>PNVYDFGHMR | 244 | 7 |
| gb\|AAA69655 | V229 | 219 | AENAGLKTTF<u>V</u>PNVYDWDYMR | 239 | 8 |
| ref\|ZP_07282326 | V226 | 216 | AQKTGLKTTF<u>V</u>PSVYDFEYMK | 236 | 9 |
| ref\|ZP_07286354 | V234 | 224 | AQNTGLKTTF<u>V</u>PNVYDFGYMK | 244 | 10 |
| ref\|ZP_04998002 | V235 | 225 | AQNTGLKTVF<u>V</u>PNVYDFEYMK | 245 | 11 |
| ref\|YP_003492288 | V223 | 213 | AAKAGLRTTF<u>V</u>PSVYDFDHMQ | 233 | 12 |
| gb\|ADI09201 | V227 | 217 | AKTAGLKTVF<u>V</u>PNVYDFDYMQ | 237 | 13 |
| ref\|ZP_07603147 | M233 | 223 | AARAGLKTVF<u>M</u>PNVYDFDYMR | 243 | 14 |
| ref\|YP_003512290 | V217 | 207 | AEETGLATTF<u>V</u>PNVYDFDHMA | 227 | 15 |
| ref\|ZP_05008217 | V223 | 213 | AARAGLGTVF<u>V</u>PNVYDFDYMR | 233 | 16 |
| ref\|ZP_06770826 | V237 | 227 | AARAGLGTVF<u>V</u>PNVYDFDYMR | 247 | 17 |
| ref\|ZP_06566730 | V220 | 210 | AQRAGLRTTF<u>V</u>PNVYDFGYMR | 230 | 18 |
| ref\|NP_827244 | V228 | 218 | ATNTGLKTTF<u>V</u>PNVYDFGYMQ | 238 | 19 |
| ref\|YP_001108512 | V208 | 198 | AQRAGLRTTF<u>V</u>PNVYDFGYMR | 218 | 20 |
| ref\|ZP_07292951 | V230 | 220 | ASAAGLKTVF<u>V</u>PSVYDFDYMR | 240 | 21 |
| ref\|ZP_07303187 | V228 | 218 | ADNAGLKTTF<u>V</u>PNVYDFGHME | 238 | 22 |
| ref\|ZP_01689718 | V215 | 205 | AEKAGFKTVT<u>V</u>PNIYDYNYMQ | 225 | 23 |

(continued)

| Origin* | position of mutation | | | | SEQ ID NO** |
|---|---|---|---|---|---|
| ref\|YP_003384280 | I226 | 216 | AHQAGFRTAV<u>I</u>PNVYDFGYLE | 236 | 24 |
| ref\|ZP_06916507 | V228 | 218 | AQNTGLKTTF<u>V</u>PSVYDFGYMQ | 238 | 25 |
| ref\|YP_003134867 | V219 | 209 | AHNAGLTTTF<u>V</u>PSVYDFARMR | 229 | 26 |
| emb\|CAC44897 | V236 | 226 | AQRSGFTTAF<u>V</u>PNVYDFEYMK | 246 | 27 |
| pdb\|1COY_A | V191 | 181 | AQRSGFTTAF<u>V</u>PNVYDFEYMK | 201 | 28 |
| sp\|P22637 | V236 | 226 | AQRSGFTTAF<u>V</u>PNVYDFEYMK | 246 | 29 |
| ref\|ZP_06830857 | V236 | 226 | AQRSGFTTAF<u>V</u>PNVYDFEYMK | 246 | 30 |
| gb\|ABC75776 | V236 | 226 | AQRSGFTTAF<u>V</u>PNVYDFEYMK | 246 | 31 |
| gb\|ABG24169 | V236 | 226 | AQRSGFTTAF<u>V</u>PNVYDFEYMK | 246 | 32 |
| emb\|CAZ68116 | V227 | 217 | AQRSGFTTAF<u>V</u>PNVYDFEYMK | 237 | 33 |
| ref\|YP_002764459 | V236 | 226 | AERSGYTTTF<u>V</u>PNVYDFNYMK | 246 | 34 |
| ref\|ZP_04388109 | V206 | 196 | AERSGYTTTF<u>V</u>PNVYDFNYMK | 216 | 35 |
| ref\|YP_003769621 | V211 | 201 | AQRSGFPFVF<u>V</u>PDVYDWDYME | 221 | 36 |
| ref\|YP_003339891 | V235 | 225 | AQRSGFPFVF<u>V</u>PDVYDWDYMK | 245 | 37 |
| ref\|ZP_07290670 | V237 | 227 | AQRSGFPFVF<u>V</u>PDVYDWDYMK | 247 | 38 |
| ref\|YP_003116660 | V234 | 224 | AGRSGFPFQF<u>V</u>PDVYDWNYMQ | 244 | 39 |
| ref\|ZP_06588880 | V238 | 228 | AQRSGFPFLF<u>V</u>PAVYDWDYMK | 248 | 40 |
| ref\|ZP_04697978 | V190 | 180 | AQRSGFPFLF<u>V</u>PAVYDWDYMK | 200 | 41 |
| ref\|ZP_06276136 | V235 | 225 | AQRSGFPFLF<u>V</u>PAVYDWDYMK | 245 | 42 |
| ref\|YP_001821989 | V235 | 225 | AQRSGFPFLF<u>V</u>PAVYDWDYMK | 245 | 43 |
| ref\|ZP_05002034 | V234 | 224 | AQRSGFPFVF<u>V</u>PNVYDWEYMK | 244 | 44 |
| ref\|ZP_06907496 | V235 | 225 | AERSGFPFVL<u>V</u>PGVYDWDYLE | 245 | 45 |
| ref\|YP_003100211 | V232 | 222 | AQRSGFPFEL<u>V</u>PGVYDWAHLE | 242 | 46 |
| ref\|ZP_07085639 | V211 | 201 | AHKAGFKTIR<u>V</u>PNVYDFKYME | 221 | 47 |
| gb\|ADX68765 | V208 | 198 | AQKAGYKTIR<u>V</u>PNVYNFKYME | 218 | 48 |
| * Databases: gb: GenBank; sp: Swissprot; ref: RefSeq; emb: EMBL; pdb: Protein Data Bank<br>** SEQ ID NOs represent the full length sequence | | | | | |

It is evident from Table 12 that Phe396 of SEQ ID NO:1 is conserved among the amino acid sequences listed in Table 12. Accordingly, a person skilled in the art can easily identify the Phe residue corresponding to the Phe396 of SEQ ID NO:1 within the conserved region using any of commercially available software programs for sequence alignment, and understand that a mutant cholesterol oxidase is easily prepared by introducing modification on that Phe residue.

**Table 12:**

| Origin* | position of mutation | | | | SEQ ID NO** |
|---|---|---|---|---|---|
| sp\|P12676 | F396 | 386 | DAWDN----SDSSV<u>F</u>AEIAPMPAGL | 406 | 1 |
| gb\|ABS32193 | F392 | 382 | DDWDN----PQNPV<u>F</u>AEIAPMPAGL | 402 | 2 |
| ref\|NP_821583 | F397 | 387 | DDWDN----PDTPV<u>F</u>AEIAPLPAGV | 407 | 3 |
| emb\|CAC20926 | F399 | 389 | DDWNN----PTAPV<u>F</u>AEIAPMPAGL | 409 | 4 |
| gb\|ADX66466 | F402 | 392 | DDWDN----PAAPV<u>F</u>AEIAPMPAGL | 412 | 5 |

(continued)

| Origin* | position of mutation | | | | SEQ ID NO** |
|---|---|---|---|---|---|
| gb\|AAR16516 | F399 | 389 | DDWDN----PAAPVFAEIAPMPAGL | 409 | 6 |
| gb\|AAZ66744 | F402 | 392 | DDWNN----AAAPVFAEIAPMPAGA | 412 | 7 |
| gb\|AAA69655 | F397 | 387 | DDWDN----PDNPVFAEIAPMPAGL | 407 | 8 |
| ref\|ZP_07282326 | F394 | 384 | DDWDN----PAHPVFAEIAPVPAGL | 404 | 9 |
| ref\|ZP_07286354 | F402 | 392 | DDWDN----AANPVFAEIAPLPMGI | 412 | 10 |
| ref\|ZP_04998002 | F403 | 393 | DDWDN----AANPVFAEIAPLPMGF | 413 | 11 |
| ref\|YP_003492288 | F391 | 381 | DDWAN----TANPVFAEIAPLPTGL | 401 | 12 |
| gb\|ADI09201 | F395 | 385 | DDWSN----ATNPVFAEIAPLPAGT | 405 | 13 |
| ref\|ZP_07603147 | F401 | 391 | DDWSN----TANPVFAEIAPLPAGL | 411 | 14 |
| ref\|YP_003512290 | F385 | 375 | DDWDN----EAARVFAEIAPVPAGF | 395 | 15 |
| ref\|ZP_05008217 | F397 | 387 | DDWNN----PTHPVFAEIAPLPMGL | 407 | 16 |
| ref\|ZP_06770826 | F411 | 401 | DDWNN----PTHPVFAEIAPLPMGL | 421 | 17 |
| ref\|ZP_06566730 | F388 | 378 | DNWDD----PVHPVFAEIAPLPAGL | 398 | 18 |
| ref\|NP_827244 | F396 | 386 | DDWAN----TSNPVFAEIAPLPMGL | 406 | 19 |
| ref\|YP_001108512 | F376 | 366 | DNWDD----PVHPVFAEIAPLPAGL | 386 | 20 |
| ref\|ZP_07292951 | F398 | 388 | DDWSN----AANPVFAEIAPLPAGT | 408 | 21 |
| ref\|ZP_07303187 | F396 | 386 | DDWAN----TANPVFAEIAPLPMGL | 406 | 22 |
| ref\|ZP_01689718 | F383 | 373 | NDWDN----ASNPVFAEIAPLPTGF | 393 | 23 |
| ref\|YP_003384280 | F393 | 383 | DNWDD----PVHPAFAEIAPLPTGL | 403 | 24 |
| ref\|ZP_06916507 | F396 | 386 | DDWAN----TDNPVFAEIAPLPTGL | 406 | 25 |
| ref\|YP_003134867 | F387 | 377 | DAWDD----PRHPVFAEVAPMPAGV | 397 | 26 |
| emb\|CAC44897 | F404 | 394 | DNWAD----PTAPIFAEIAPLPAGL | 414 | 27 |
| pdb\|1C0Y_A | F359 | 349 | DNWAD----PTAPIFAEIAPLPAGL | 369 | 28 |
| sp\|P22637 | F404 | 394 | DNWAD----PTAPIFAEIAPLPAGL | 414 | 29 |
| ref\|ZP_06830857 | F404 | 394 | DNWAD----PTAPIFAEIAPLPAGL | 414 | 30 |
| gb\|ABC75776 | F404 | 394 | DNWAD----PAAPIFAEIAPLPAGL | 414 | 31 |
| gb\|ABG24169 | F405 | 395 | DNWAD----PTAPIFAEIAPLPAGL | 415 | 32 |
| emb\|CAZ68116 | F395 | 385 | DNWAD----PTAPIFAEIAPLPAGL | 405 | 33 |
| ref\|YP_002764459 | F405 | 395 | DNWAD----TSAPVFAEIAPFPAGT | 415 | 34 |
| ref\|ZP_04388109 | F375 | 365 | DNWAD----TSAPVFAEIAPFPAGT | 385 | 35 |
| ref\|YP_003769621 | F377 | 369 | DNWAA----GGA--FAEVAPLPTGI | 387 | 36 |
| ref\|YP_003339891 | F401 | 393 | DNWAA----GGA--FAEVAPLPTGI | 411 | 37 |
| ref\|ZP_07290670 | F403 | 395 | DNWDA----GGA--FAEVAPLPTGI | 413 | 38 |
| ref\|YP_003116660 | F400 | 392 | DNWTK----GGA--FAEVAPLPIGI | 410 | 39 |
| ref\|ZP_06588880 | F404 | 396 | DNWDA----GGA--FAEIAPLPTGI | 414 | 40 |
| ref\|ZP_04697978 | F356 | 348 | DNWDA----GGA--FAEIAPLPTGI | 366 | 41 |
| ref\|ZP_06276136 | F401 | 393 | DNWDA----GGA--FAEVAPLPTGI | 411 | 42 |
| ref\|YP_001821989 | F401 | 393 | DNWDA----GGA--FAEVAPLPTGI | 411 | 43 |

(continued)

| Origin* | position of mutation | | | | SEQ ID NO** |
|---|---|---|---|---|---|
| ref\|ZP_05002034 | F400 | 392 | DNWDA----GGA--FAEVAPLPTGI | 410 | 44 |
| ref\|ZP_06907496 | F401 | 393 | DNWQA----GGA--FAEVAPLPTGI | 411 | 45 |
| ref\|YP_003100211 | F397 | 389 | DNWAA----GGA--FAEVAPLPTGV | 407 | 46 |
| ref\|ZP_07085639 | F380 | 370 | DNWDD----PEHQFFTEIAPLPMGM | 390 | 47 |
| gb\|ADX68765 | F377 | 367 | DNWDD----PKYPFFAEIAPLPMGM | 387 | 48 |

* Databases: gb: GenBank; sp: Swissprot; ref: RefSeq; emb: EMBL; pdb: Protein Data Bank
** SEQ ID NOs represent the full length sequence

Industrial Applicability

The mutated cholesterol oxidase of the invention may be used for the measurement of cholesterol or lipoproteins associated with cholesterol, such as high density lipoprotein or low density lipoprotein, which is clinically useful in diagnosis and control of certain health conditions.

**Claims**

1. A mutant cholesterol oxidase consisting of an amino acid sequence set forth in SEQ ID NO: 1 and having a reduced oxidase activity as compared to the wild-type cholesterol oxidase and having a dehydrogenase activity of 50% or more of the wild-type cholesterol oxidase, wherein the amino acid residue at position 228 is an amino acid residue selected from Ala, Thr, Lys, Cys, Ser, Gly, Glu, Tyr, Pro, Asn, Gln, Trp and His, the amino acid residue at position 159 is Met and the amino acid residue at position 396 is Phe.

2. The mutant cholesterol oxidase of claim 1 having an oxidase activity of less than its dehydrogenase activity, wherein the amino acid residue at position 228 is an amino acid residue selected from Ala, Lys, Ser, Gly, Glu, Tyr, and Asn.

3. An isolated polynucleotide encoding the mutant cholesterol oxidase of any one of claims 1-2.

4. A vector comprising the polynucleotide of claim 3.

5. A host cell transformed with a vector of claim 4.

6. A method for assaying cholesterol in a sample, comprising contacting the sample with the cholesterol oxidase of any one of claims 1-2, and measuring the amount of the cholesterol oxidized by the cholesterol oxidase.

7. A method for assaying HDL cholesterol comprising reacting HDL in a sample to generate cholesterol, contacting the cholesterol with the cholesterol oxidase of any one of claims 1-2, and measuring the amount of oxidized cholesterol.

8. A method for assaying LDL cholesterol comprising reacting LDL in a sample to generate cholesterol, contacting the cholesterol with the cholesterol oxidase of any one of claims 1-2, and measuring the amount of oxidized cholesterol.

9. A device for assaying cholesterol, HDL cholesterol, and/or LDL cholesterol in a sample comprising the cholesterol oxidase of any one of claims 1-2 and an electron mediator.

10. A kit for assaying cholesterol, HDL cholesterol, and/or LDL cholesterol in a sample comprising the cholesterol oxidase of any one of claims 1-2 and an electron mediator.

11. An enzyme electrode having the cholesterol oxidase of any one of claims 1-2 which is immobilized on the electrode.

12. An enzyme sensor for assaying cholesterol, HDL cholesterol, LDL cholesterol comprising the enzyme electrode of claim 11 as a working electrode.

**Patentansprüche**

1. Mutierte Cholesterinoxidase, bestehend aus einer Aminosäuresequenz gemäß SEQ ID NO: 1 und mit einer reduzierten Oxidase-Aktivität im Vergleich zur Wildtyp-Cholesterinoxidase und mit einer Dehydrogenase-Aktivität von 50% oder mehr der Wildtyp-Cholesterinoxidase, wobei es sich bei dem Aminosäurerest an Position 228 um einen aus Ala, Thr, Lys, Cys, Ser, Gly, Glu, Tyr, Pro, Asn, Gln, Trp und His ausgewählten Aminosäurerest, dem Aminosäurerest an Position 159 um Met und dem Aminosäurerest an Position 396 um Phe handelt.

2. Mutierte Cholesterinoxidase nach Anspruch 1 mit einer geringeren Oxidase-Aktivität als ihrer Dehydrogenase-Aktivität, wobei es sich bei dem Aminosäurerest an Position 228 um einen aus Ala, Lys, Ser, Gly, Glu, Tyr und Asn ausgewählten Aminosäurerest handelt.

3. Isoliertes Polynukleotid, codierend die mutierte Cholesterinoxidase nach einem der Ansprüche 1-2.

4. Vektor, umfassend das Polynukleotid nach Anspruch 3.

5. Wirtszelle, transformiert mit einem Vektor nach Anspruch 4.

6. Testverfahren für Cholesterin in einer Probe, umfassend Inkontaktbringen der Probe mit der Cholesterinoxidase nach einem der Ansprüche 1-2 und Messen der Menge des von der Cholesterinoxidase oxidierten Cholesterins.

7. Testverfahren für HDL-Cholesterin, umfassend Umsetzen von HDL in einer Probe unter Erzeugung von Cholesterin, Inkontaktbringen des Cholesterins mit der Cholesterinoxidase nach einem der Ansprüche 1-2 und Messen der Menge an oxidiertem Cholesterin.

8. Testverfahren für LDL-Cholesterin, umfassend Umsetzen von LDL in einer Probe unter Erzeugung von Cholesterin, Inkontaktbringen des Cholesterins mit der Cholesterinoxidase nach einem der Ansprüche 1-2 und Messen der Menge an oxidiertem Cholesterin.

9. Testvorrichtung für Cholesterin, HDL-Cholesterin und/oder LDL-Cholesterin in einer Probe, umfassend die Cholesterinoxidase nach einem der Ansprüche 1-2 und einen Elektronenvermittler.

10. Testkit für Cholesterin, HDL-Cholesterin und/oder LDL-Cholesterin in einer Probe, umfassend die Cholesterinoxidase nach einem der Ansprüche 1-2 und einen Elektronenvermittler.

11. Enzymelektrode mit der Cholesterinoxidase nach einem der Ansprüche 1-2, die auf der Elektrode immobilisiert ist.

12. Enzymsensor zum Testen auf Cholesterin, HDL-Cholesterin, LDL-Cholesterin, umfassend die Enzymelektrode nach Anspruch 11 als Arbeitselektrode.

**Revendications**

1. Cholestérol-oxydase mutante consistant en une séquence d'acides aminés présentée dans SEQ ID N° : 1 et ayant une activité oxydase réduite en comparaison de la cholestérol-oxydase de type sauvage et une activité déshydrogénase représentant 50 % ou plus de celle de la cholestérol-oxydase de type sauvage, dans laquelle le résidu d'acide aminé en position 228 est un résidu d'acide aminé choisi parmi Ala, Thr, Lys, Cys, Ser, Gly, Glu, Tyr, Pro, Asn, Gln, Trp et His, le résidu d'acide aminé en position 159 est Met et le résidu d'acide aminé en position 396 est Phe.

2. Cholestérol-oxydase mutante selon la revendication 1 ayant une activité oxydase inférieure à son activité déshydrogénase, dans laquelle le résidu d'acide aminé en position 228 est un résidu d'acide aminé choisi parmi Ala, Lys, Ser, Gly, Glu, Tyr, et Asn.

3. Polynucléotide isolé codant pour la cholestérol-oxydase mutante de l'une quelconque des revendications 1-2.

4. Vecteur comprenant le polynucléotide de la revendication 3.

5. Cellule hôte transformée avec un vecteur de la revendication 4.

6. Procédé de dosage du cholestérol dans un échantillon, comprenant les étapes qui consistent à mettre l'échantillon en contact avec la cholestérol-oxydase de l'une quelconque des revendications 1-2, et à mesurer la quantité du cholestérol oxydé par la cholestérol-oxydase.

7. Procédé de dosage du cholestérol HDL comprenant les étapes qui consistent à faire réagir des HDL dans un échantillon afin de produire du cholestérol, à mettre le cholestérol en contact avec la cholestérol-oxydase de l'une quelconque des revendications 1-2, et à mesurer la quantité de cholestérol oxydé.

8. Procédé de dosage du cholestérol LDL comprenant les étapes qui consistent à faire réagir des LDL dans un échantillon afin de produire du cholestérol, à mettre le cholestérol en contact avec la cholestérol-oxydase de l'une quelconque des revendications 1-2, et à mesurer la quantité de cholestérol oxydé.

9. Dispositif pour le dosage du cholestérol, du cholestérol HDL, et/ou du cholestérol LDL dans un échantillon comprenant la cholestérol-oxydase de l'une quelconque des revendications 1-2 et un médiateur d'électrons.

10. Trousse pour le dosage du cholestérol, du cholestérol HDL, et/ou du cholestérol LDL dans un échantillon comprenant la cholestérol-oxydase de l'une quelconque des revendications 1-2 et un médiateur d'électrons.

11. Électrode à enzymes ayant la cholestérol-oxydase de l'une quelconque des revendications 1-2 qui est immobilisée sur l'électrode.

12. Détecteur enzymatique pour le dosage du cholestérol, du cholestérol HDL, du cholestérol LDL comprenant l'électrode à enzymes de la revendication 11 en tant qu'électrode de travail.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004113900 A
- US 5997817 A
- US 7008799 B
- US 6036919 A
- US 5334508 A

### Non-patent literature cited in the description

- **VRIELINK et al.** *FEBS J,* 2009, vol. 276 (23), 6826-6848
- **POLLEGIONI et al.** *FEBS J.,* 2009, vol. 276 (23), 6857-70
- **TOYAMA et al.** *Protein Engineering,* 2002, vol. 15 (6), 477-483
- **YAN SUN.** *Biotechnology Letters,* 2011, vol. 33 (10), 2049-2055
- **YUE et al.** *Biochemistry,* 1999, vol. 38 (14), 4277-4286
- **LU, G. ; MORIYAMA, E. N.** Vector NTI, a balanced all-in-one sequence analysis suite. *Brief Bioinform,* 2004, vol. 5, 378-88